# EUROPEAN PATENT SPECIFICATION

(11) **EP 2 825 664 B1**
(45) Date of publication and mention of the grant of the patent: **15.05.2019**
(21) Application number: 13709109.6
(22) Date of filing: 13.03.2013
(51) Int. Cl.: C12Q 1/6886, C12Q 1/6888, C12N 5/0786, G01N 33/50

(54) **HIGH-RESOLUTION TRANSCRIPTOME OF HUMAN MACROPHAGES**
HOCHAUFLÖSENDES TRANSKRIPTOM HUMANER MAKROPHAGEN
TRANSCRIPTOME HAUTE RÉSOLUTION DE MACROPHAGES HUMAINS

(30) Priority: 14.03.2012 EP 12159479
(43) Date of publication of application: 21.01.2015
(73) Proprietor: Rheinische Friedrich-Wilhelms-Universität Bonn, 53115 Bonn (DE); Becton, Dickinson and Company, Franklin Lakes, NJ 07417-1880 (US)
(72) Inventor: SCHULTZE, Joachim, 53639 Königswinter (DE); MALLMANN, Michael, 50672 Köln (DE)
(74) Representative: dompatent von Kreisler Selting Werner - Partnerschaft von Patent- und Rechtsanwälten mbB
(86) International application number: PCT/EP2013/055159
(87) International publication number: WO 2013/135775

(56) References cited:
- WO-A1-2011/106521
- MURRAY PETER J ET AL: "Obstacles and opportunities for understanding macrophage polarization", JOURNAL OF LEUKOCYTE BIOLOGY, vol. 89, no. 4, April 2011 (2011-04), pages 557-563, XP009160896, ISSN: 0741-5400
- MARTINEZ FERNANDO O ET AL: "Transcriptional profiling of the human monocyte-to-macrophage differentiation and polarization: New molecules and patterns of gene expression", JOURNAL OF IMMUNOLOGY, AMERICAN ASSOCIATION OF IMMUNOLOGISTS, US, vol. 177, no. 10, 15 November 2006 (2006-11-15), pages 7303-7311, XP009160929, ISSN: 0022-1767
- WANG YAO-CHUN ET AL: "Notch Signaling Determines the M1 versus M2 Polarization of Macrophages in Antitumor Immune Responses", CANCER RESEARCH, vol. 70, no. 12, June 2010 (2010-06), pages 4840-4849, XP009160889, ISSN: 0008-5472
- SICA A ET AL: "Macrophage polarization in tumour progression", SEMINARS IN CANCER BIOLOGY, SAUNDERS SCIENTIFIC PUBLICATIONS, PHILADELPHIA, PA, US, vol. 18, no. 5, 1 October 2008 (2008-10-01), pages 349-355, XP023519773, ISSN: 1044-579X, DOI: 10.1016/J.SEMCANCER.2008.03.004 [retrieved on 2008-03-26]
- LAWRENCE TOBY ET AL: "Transcriptional regulation of macrophage polarization: enabling diversity with identity", NATURE REVIEWS IMMUNOLOGY, vol. 11, no. 11, November 2011 (2011-11), pages 750-761 URL, XP009160964,
- MANTOVANI A ET AL: "Macrophages, innate immunity and cancer: balance, tolerance, and diversity", CURRENT OPINION IN IMMUNOLOGY, ELSEVIER, OXFORD, GB, vol. 22, no. 2, 1 April 2010 (2010-04-01), pages 231-237, XP027020479, ISSN: 0952-7915 [retrieved on 2010-02-09]
- OZSOLAK FATIH ET AL: "Transcriptome profiling using single-molecule direct RNA sequencing.", METHODS IN MOLECULAR BIOLOGY (CLIFTON, N.J.) 2011 LNKD- PUBMED:21431762, vol. 733, 2011, pages 51-61, XP009160895, ISSN: 1940-6029

## Description

The invention is based on the finding of specific surface markers for M1-like and M2-like macrophages and provides for a method for identifying of, distinguishing between and isolating of M1-like (classically activated) and M2-like (alternatively activated) macrophages based on the abundance of said surface markers and for means for performing such method.

### Background of the Invention

Macrophages represent resident phagocytic cells in the tissue and are involved in tissue homeostasis and induction of inflammatory reaction towards pathogens by use of their broad range of pattern-recognition receptors (Geissmann F. et al., Science 327(5966):656-661 (2010)). In context of the respective immune response, macrophages are polarized to specific functional properties, often referred to as M1-like and M2-like phenotype. Classically polarized M1-like macrophages can be induced by IFN-γ alone or together with LPS or TNF-α using M-CSF or GM-CSF (Martinez F.O. et al., The Journal of Immunology 177(10):7303-7311 (2006)). M1-like macrophages are effector cells of classical inflammatory immune responses exerting an IL-12^{high}, IL-23^{high} and IL-10^{low} phenotype with secretion of inflammatory cytokines IL-1β, IL-6 and TNF-α. They display a phenotype characterized by the expression of CD86, CD64, and CD16 (Biswas S.K., Mantovani A., Nat Immunol. 11(10):889-896 (2010); Mantovani A., Sica A., Curr Opin Immunol. 22(2):231-237 (2010)). In contrast, macrophages that are activated by other mechanisms than IFN-γ/LPS/TNF-α are grouped in the alternatively activated M2-like macrophage subset. Non-classically activated macrophages can be induced by cytokines including IL-4 and IL-13, but other stimuli have been described as well (Biswas S.K., Mantovani A., Nat Immunol. 11(10):889-896 (2010); Mantovani A., Sica A., Curr Opin Immunol. 22(2):231-237 (2010)). These cells share an IL-12^{low} and IL-23^{low} phenotype and express CD23. Over the last decade, phenotypic adaptations of macrophages to environmental stimuli have been linked to radical changes in transcriptional regulation mainly by applying microarray-based gene expression profiling (Martinez F.O. et al., The Journal of Immunology 177(10):7303-7311 (2006); Gustafsson C, Mjosberg J, Matussek A. et al., PLoS One. 3(4):e2078 (2008); Lehtonen A. et al., J Leukoc Biol. 82(3):710-720 (2007); Nau G.J. et al., Proc Natl Acad Sci U S A 99(3):1503-1508 (2002)). In fact, a large amount of data covering transcriptional reprogramming of macrophages has been accumulated, albeit not always systematic (Martinez F.O. et al., The Journal of Immunology 177(10):7303-7311 (2006); Gustafsson C. et al., PLoS One. 3(4):e2078 (2008); Lehtonen A. et al., J Leukoc Biol. 82(3):710-720 (2007); Nau G.J. et al., Proc Natl Acad Sci U S A 99(3):1503-1508 (2002); Heng T.S. et al., Nat Immunol. 9(10):1091-1094 (2008)). However, molecular mechanisms controlling transcriptional reprogramming in macrophages are far from understood and it has been suggested that integrative analyses of epigenomic and transcriptomic data will be required to better understand how macrophages integrate the information they receive from their respective microenvironment (Lawrence T., Natoli G., Nat Rev Immunol. 11(11):750-761 (2011)), enabling the identification of specific transcription factor combinations being responsible for cellular macrophage programs.

The introduction of RNA sequencing (RNA-seq) to interrogate whole transcriptomes has challenged previously established gene expression profiling studies (Ozsolak F., Milos P.M., Nature reviews Genetics 12(2):87-98 (2011); Wang Z, Gerstein M, Snyder M., Nature reviews Genetics 10(1):57-63 (2009); Marioni J.C. et al., Genome Res. 18(9):1509-1517 (2008)). Advantages assigned to RNA-seq over microarray analysis include increases in transcript quantity and quality, improved detection of alternative splicing events and gene fusion transcripts, and a larger dynamic range of detection (Ozsolak F., Milos P.M., Nature reviews Genetics 12(2):87-98 (2011); Wang Z. et al., Nature reviews Genetics 10(1):57-63 (2009); Marioni J.C. et al., Genome Res. 18(9):1509-1517 (2008)).

### Short Description of the Invention

To better understand polarization and integration of environmental signals by macrophages and to identify more specific markers for different functional states, high-resolution transcriptome data have been asked for (Murray P.J., Wynn T.A., Nat Rev Immunol. 11(11):723-737 (2011)). Using M1 and M2 polarization as models we applied RNA-seq and compared the information content with data derived by microarray analysis. We provide new insights into human macrophage biology and determine several new markers associated with classical and alternative macrophage polarization in humans.

The invention thus provides
(1) an *in vitro* method for identifying of, distinguishing between and isolating of M1-like and M2-like macrophages which comprises characterizing the macrophages based on the expression level of one or more of the specific M1-associated cell surface markers CD120b having the nucleic acid sequence of SEQ ID NO:1, TLR2 having the nucleic acid sequence of SEQ ID NO:3 and SLAMF7 having the nucleic acid sequence of SEQ ID NO:5, or of one or more of the specific M2-associated cell surface markers CD1a having the nucleic acid sequence of SEQ ID NO:7, CD1b having the nucleic acid sequence of SEQ ID NO:9, CD93 having the nucleic acid sequence of SEQ ID NO:11 and CD226 having the nucleic acid sequence of SEQ ID NO:13, respectively, wherein the expression level of the M1-associated cell surface markers is at least 30% higher in M1-like macrophages than in M2-like macrophages and the expression level of the M2-associated cell surface markers is at least 30% higher in M2-like macrophages than in M1-like macrophages;
(2) a preferred embodiment of aspect (1) above, wherein the identifying of and distinguishing between the M1-like and M2-like macrophages is performed by an amplification or by a targeted resequencing of one or more of the specific M1-associated cell surface marker nucleic acids CD120b, TLR2 and SLAMF7 (SEQ ID NOs: 1, 3 and 5), or of one or more of the specific M2-associated cell surface marker nucleic acids CD1a, CD1b, CD93 and CD226 (SEQ ID NOs: 7, 9, 11 and 13), respectively, of the macrophage, notably by utilizing one or more primers derived from each of the marker nucleic acids, and a subsequent detection of the amplification/resequencing product;
(3) a preferred embodiment of aspect (1) above, wherein the identifying of and distinguishing between the M1-like and M2-like macrophages comprises hybridizing one or more probes selective for one of the specific M1-associated cell surface marker nucleic acids CD120b, TLR2 and SLAMF7 (SEQ ID NOs: 1, 3 and 5), or for one of the specific M2-associated cell surface marker nucleic acids CD1a, CD1b, CD93 and CD226 (SEQ ID NOs: 7, 9, 11 and 13), respectively, of the macrophage;
(4) a preferred embodiment of aspect (3) above, wherein the hybridization is performed on a hybridization array;
(5) a preferred embodiment of aspect (1) above, wherein the identifying of, distinguishing between and isolating of the M1-like and M2-like macrophages comprises contacting the macrophages with one or more binding molecules directed against the specific M1-associated cell surface marker proteins CD120b, TLR2 and SLAMF7 (SEQ ID NOs: 2, 4 and 6), or with one or more binding molecules directed against t0h0e0 specific M2-associated cell surface marker proteins CD1a, CD1b, CD93 and CD226 (SEQ ID NOs: 8, 10, 12 and 14), respectively;
(6) the use of a kit for performing the method according to (1) to (5) above, which kit comprises at least one reagent for identifying of, distinguishing between and isolating of M1-like macrophages and at least one reagent for identifying of, distinguishing between and isolating of M2-like macrophages, said reagents being selected from
   (i) one or more primers derived from the marker nucleic acids as defined in (2) above,
   (ii) one or more probes selective for the cell surface marker nucleic acids as defined in (3) above,
   (iii) a hybridization array as defined in (4) above, or
   (iv) one or more binding molecule as defined in (5) above;
(7) the use of antibodies selected from antibodies directed against CD120b having the protein sequence of SEQ ID NO:2, antibodies directed against TLR2 having the protein sequence of SEQ ID NO:4 and antibodies directed against SLAMF7 having the protein sequence of SEQ ID NO:6 for performing the method according to (1) to (5) above, wherein the antibodies are for identifying, characterizing and isolating M1-like macrophages;
(8) the use of antibodies selected from antibodies directed against CD1a having the protein sequence of SEQ ID NO:8, antibodies directed against CD1b having the protein sequence of SEQ ID NO:10, antibodies directed against CD93 having the protein sequence of SEQ ID NO:12 and antibodies directed against CD226 having the protein sequence of SEQ ID NO:14 for performing the method according to (1) to (5) above, wherein the antibodies are for identifying, characterizing and isolating M2-like macrophages.

### Short Description of the Figures

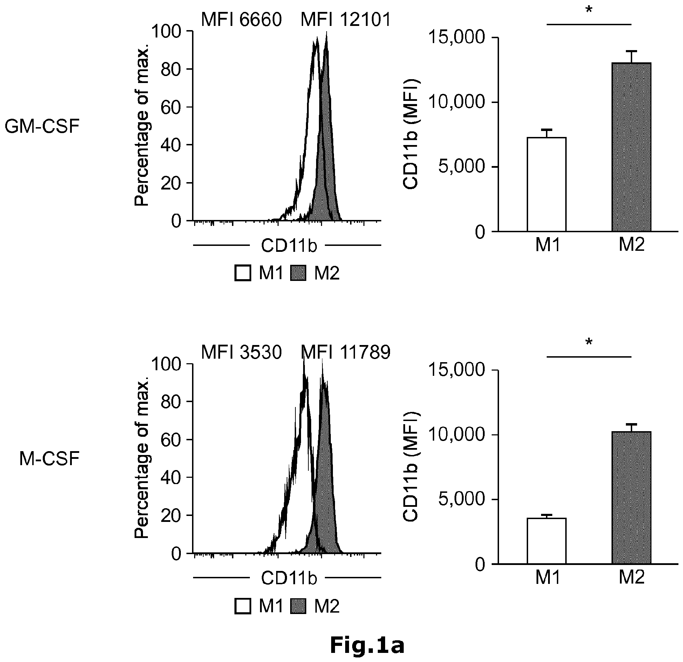

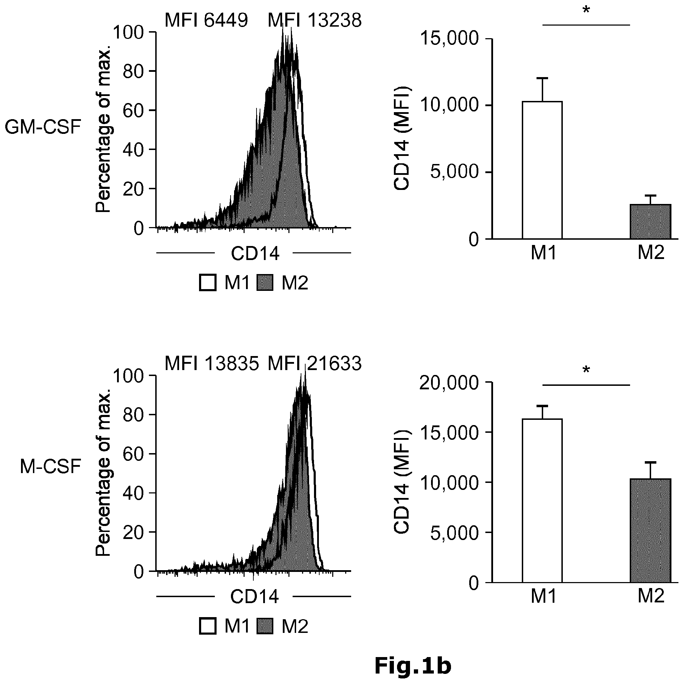

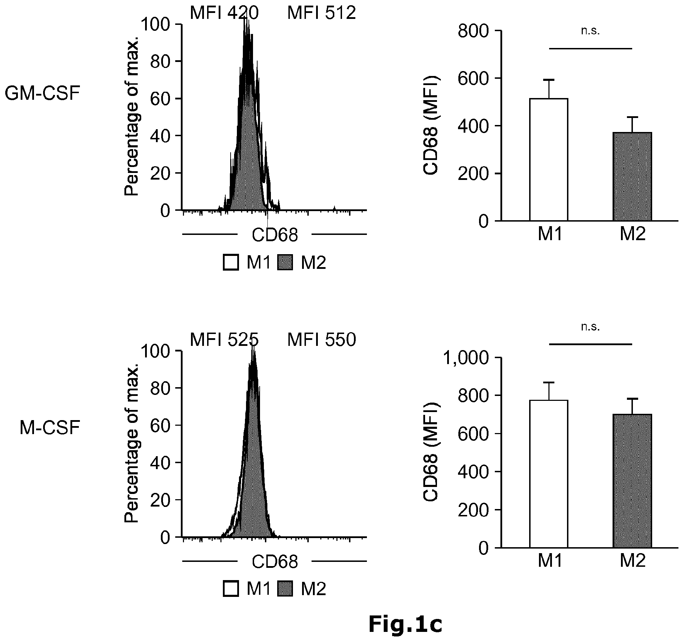

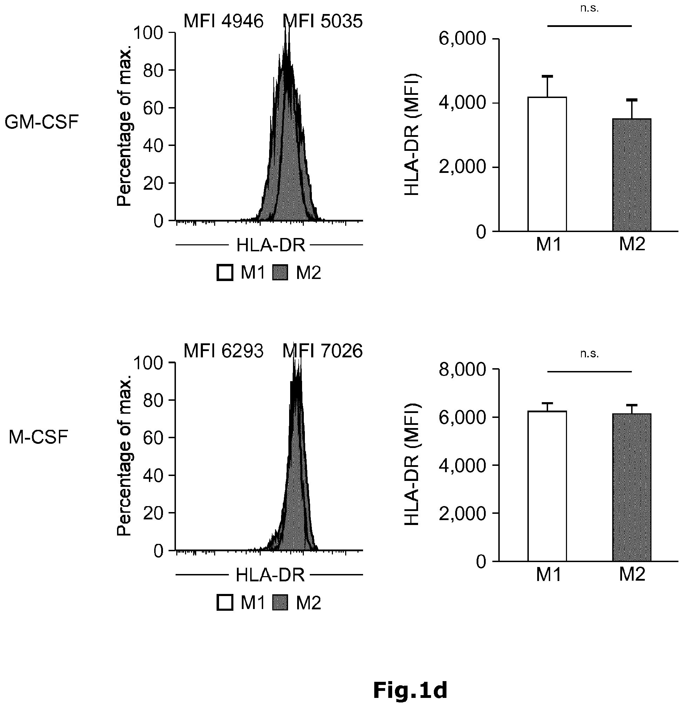

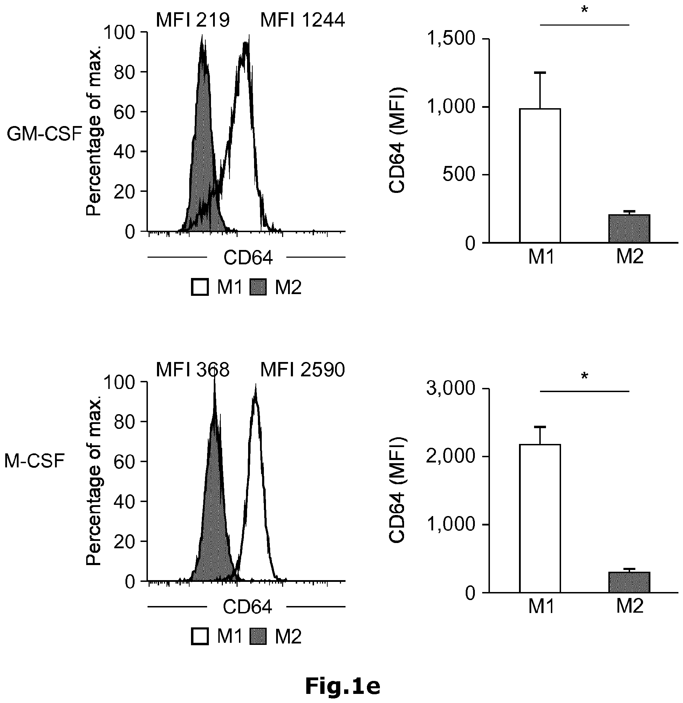

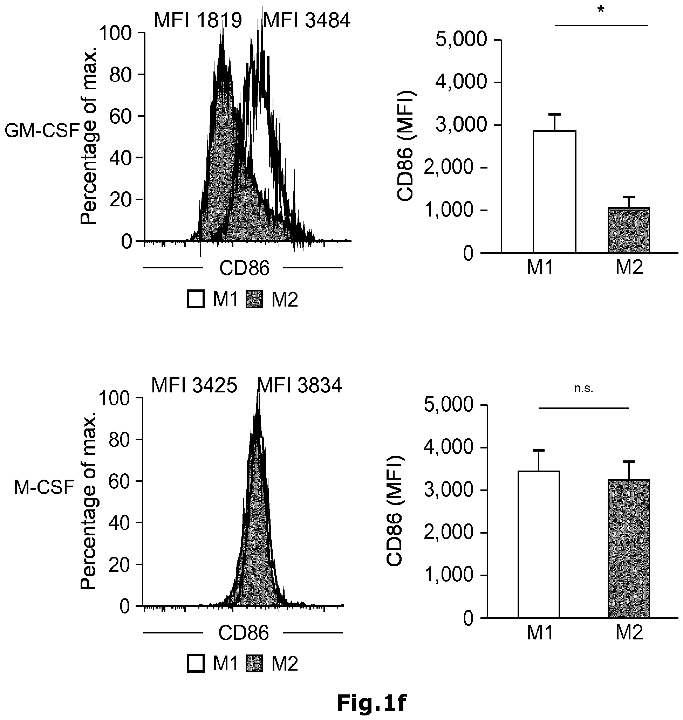

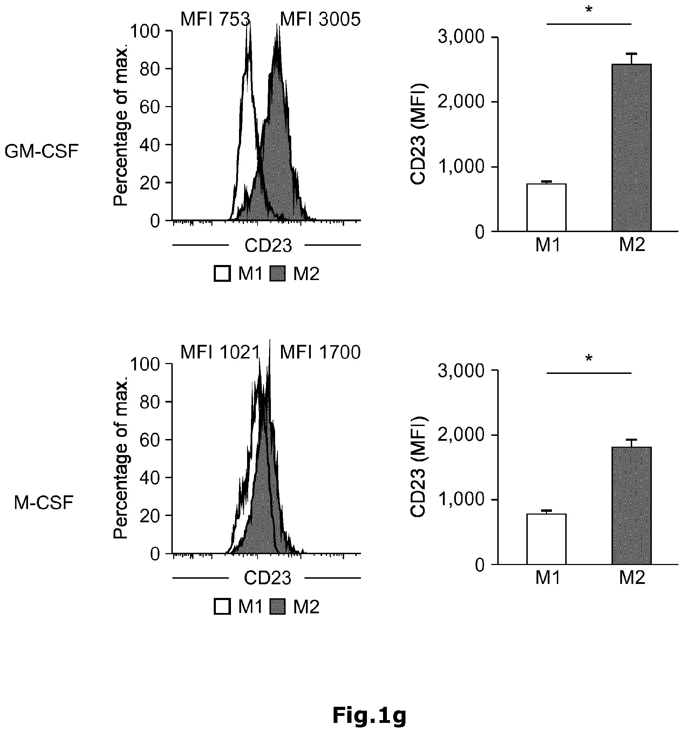

Figure 1: Phenotypic characterization of human M1- and M2-like macrophages derived from CD14⁺ peripheral blood monocytes. Expression of typical macrophage lineage markers was determined by flow cytometry (left) of M1- and M2-like macrophages generated in the presence of GM-CSF (upper panel) or M-CSF (lower panel) with quantification shown in the graph at the right. Expression of (a) CD11b, (b) CD14, (c) CD68, (d) HLA-DR, (e) CD64, (f) CD86, and (g) CD23, respectively. *P < 0.05 (Student's t-test). Numbers in plots indicate mean fluorescence intensity. Data are representative of nine independent experiments (a,b,d,e,f,g; mean and s.e.m.) or eight independent experiments (c; mean and s.e.m.), each with cells derived from a different donor.

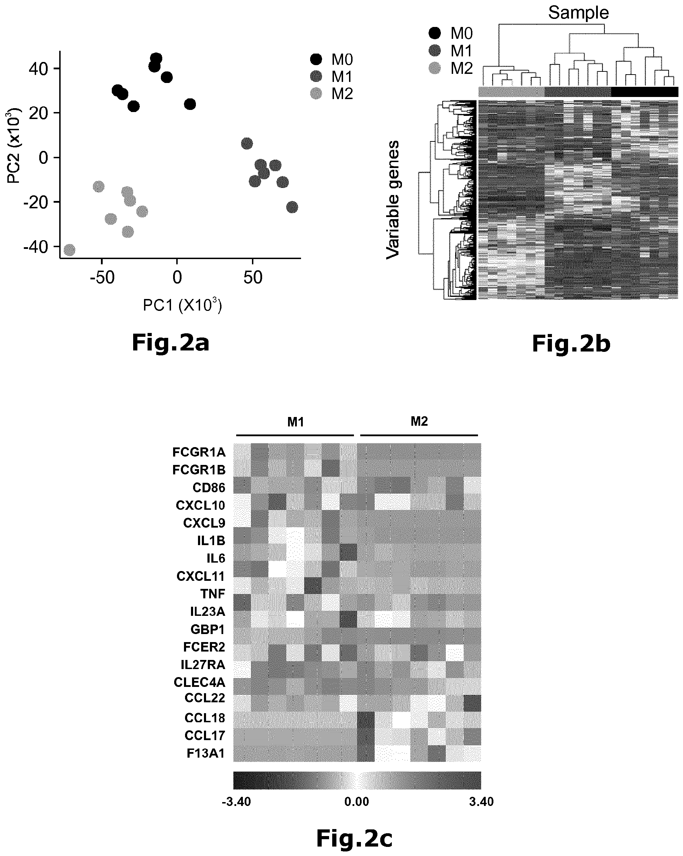

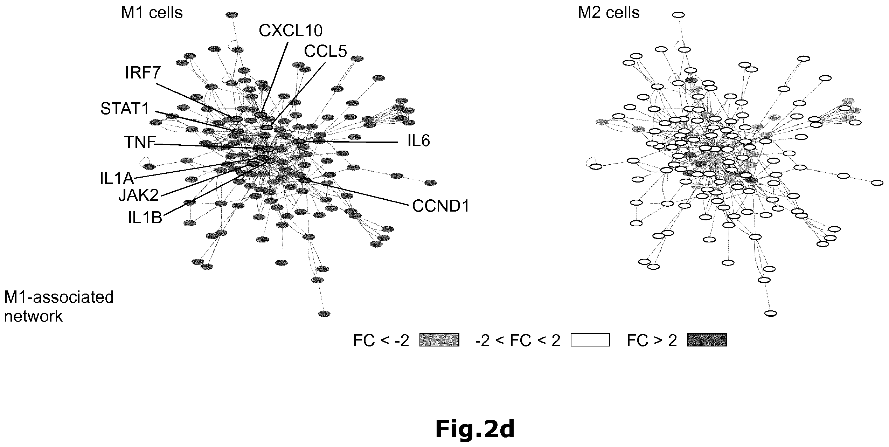

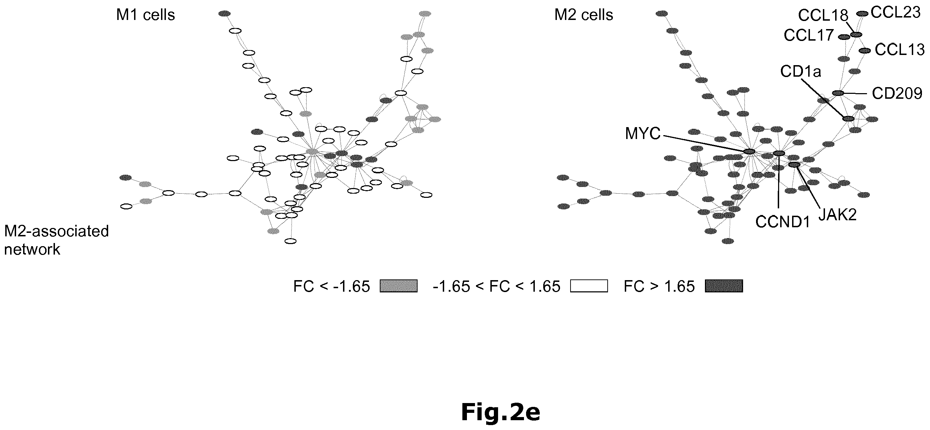

Figure 2: Microarray-based RNA fingerprinting of human M1- and M2-like macrophages. (a) Principle component analysis of human unpolarized (M0) and polarized (M1, M2) macrophages. (b) Unsupervised hierarchical clustering of human M0, M1-, and M2-like macrophages. (c) Visualization of known markers for human M1- and M2-like macrophages as a heatmap. Data were z-score normalized. (d) Left: network of genes highly expressed in M1-like macrophages (fold-change >2.0) in comparison to M0 macrophages identified by microarray analysis. Right: data for the comparison of M2-like versus M0 macrophages were loaded into the M1-network. (e) Right: network of genes highly expressed in M2-like macrophages (fold-change >1.65) in comparison to M0 macrophages identified by microarray analysis. Left: data for the comparison of M1-like versus M0 macrophages were loaded into the M2-network. All networks were generated using EGAN.

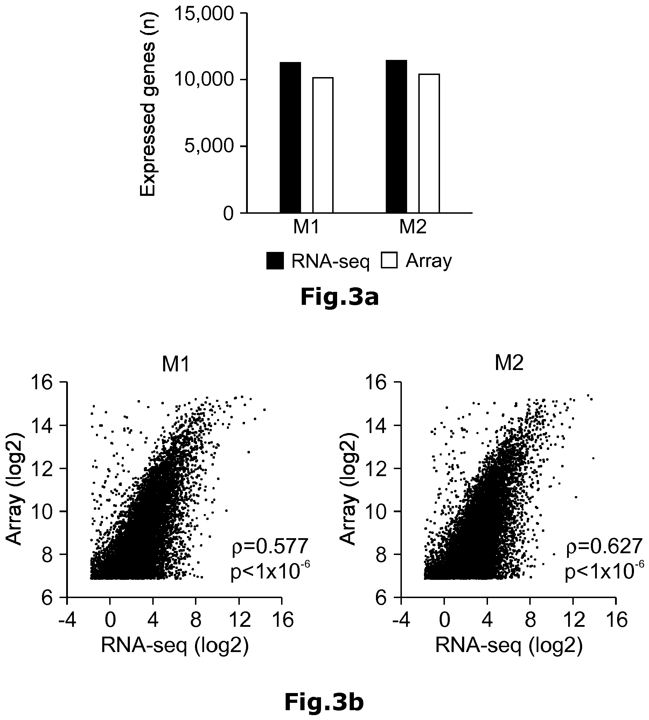

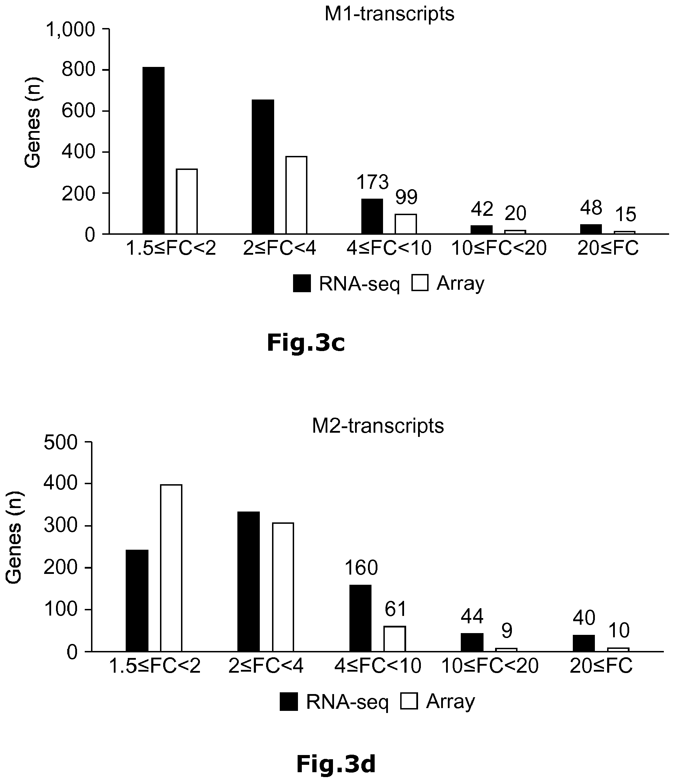

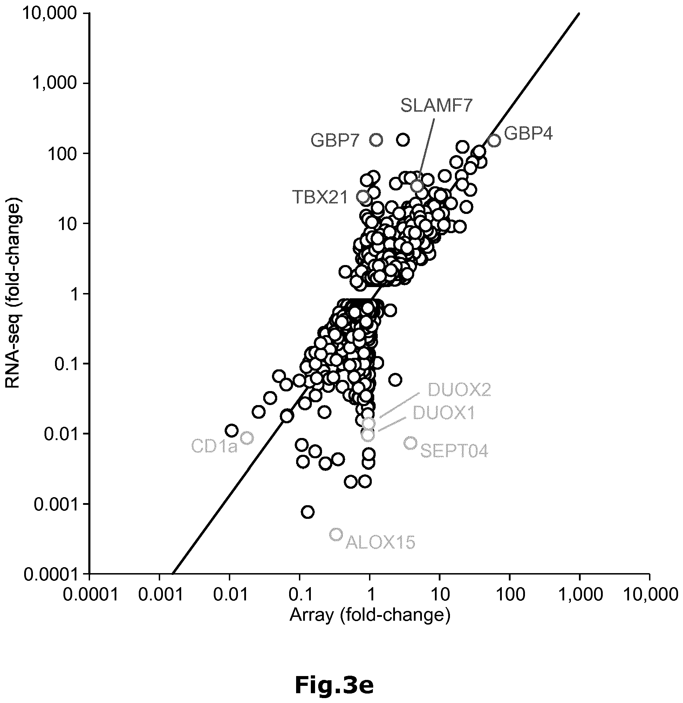

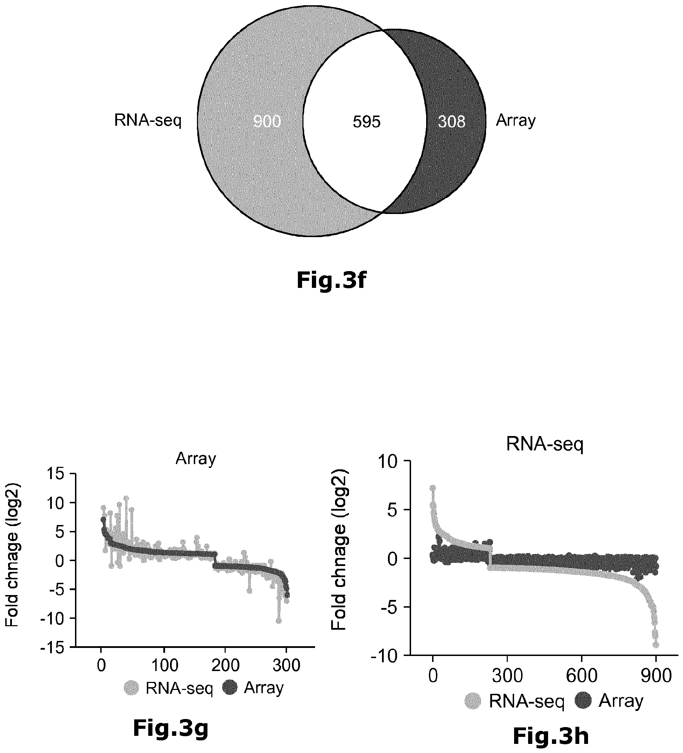

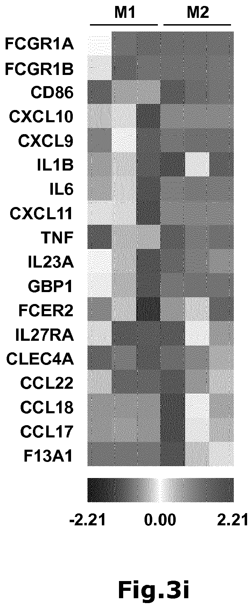

Figure 3: Comparison of RNA-seq and microarray analysis. (a) Number of genes expressed in human M1- (left) and M2-like macrophages (right) as detected using RNA-Seq (black) and microarray analysis (white). (b) Correlation (Spearman) of mean expression values of M1- (left) and M2-like macrophages (right) using RNA-Seq and microarray analysis. (c-d) Comparison of differentially expressed genes detected using RNA-seq or microarray analysis (p <0.05). Differentially expressed genes as assessed by RNA-seq (black) or microarray analysis (white) were divided into groups by their relative expression in (c) M1 versus M2 or (d) M2 versus M1. (e) Gene expression in M1- versus M2-like macrophages as fold change versus fold change plot comparing microarray analysis with RNA-seq using all Refseq genes differentially expressed in RNA-seq. (f) Venn-diagram of differentially expressed genes between M1- and M2-like macrophages in RNA-seq (light gray) and microarray analysis (dark gray), (FC > 2, p-value < 0.05, diff > 100 for microarray data). Fold-change-rank plots of genes detected as differentially expressed between M1- and M2-like macrophages (g) by microarray analysis (dark gray) with overlay of values obtained by RNA-seq (light gray) or (h) by RNA-seq (light gray) with overlay of values obtained by microarray analysis (dark gray). (i) Visualization of known markers for human M1- and M2-like macrophages from Figure 2c as a heatmap using RNA-seq. Data were z-score normalized.

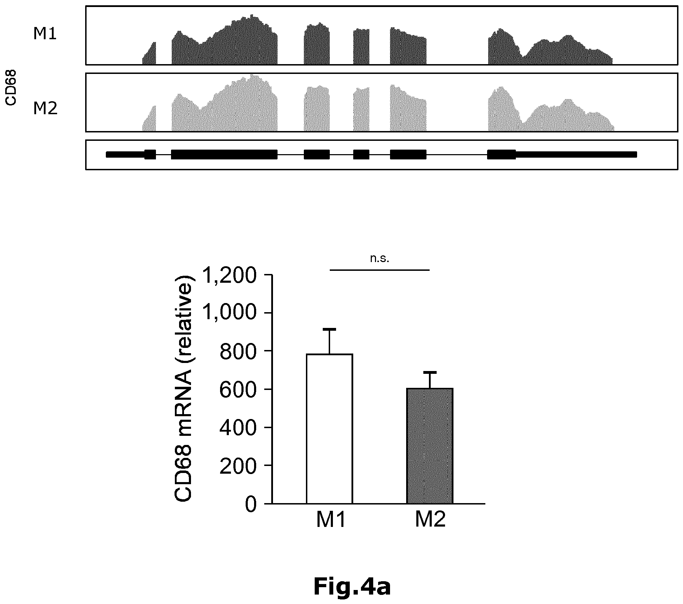

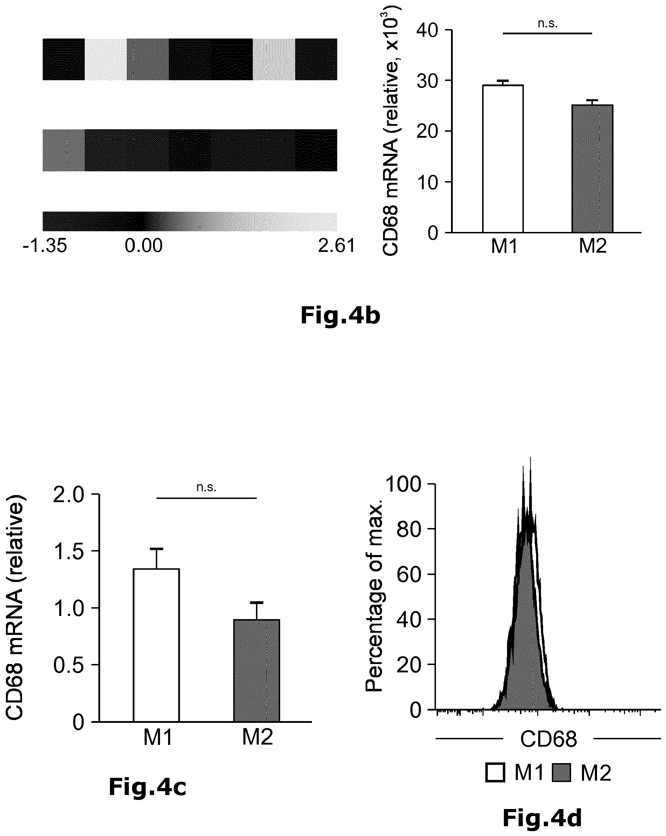

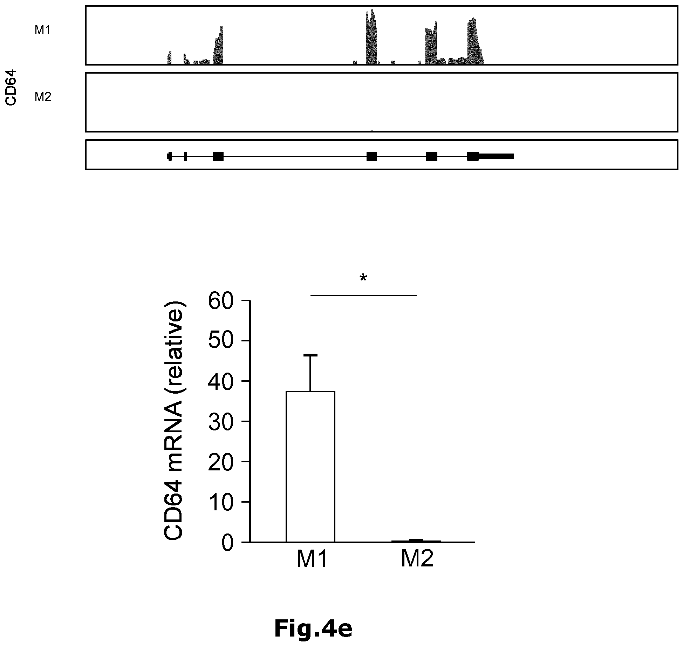

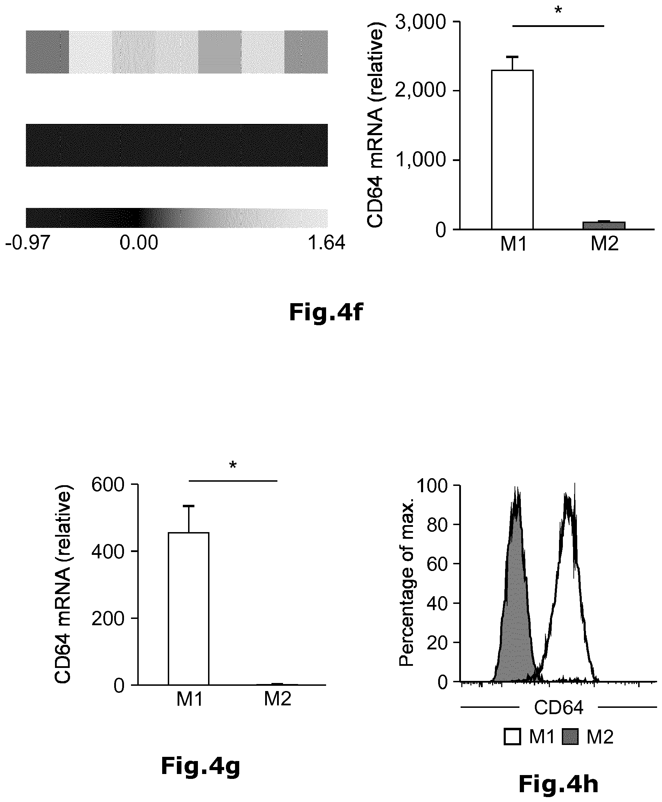

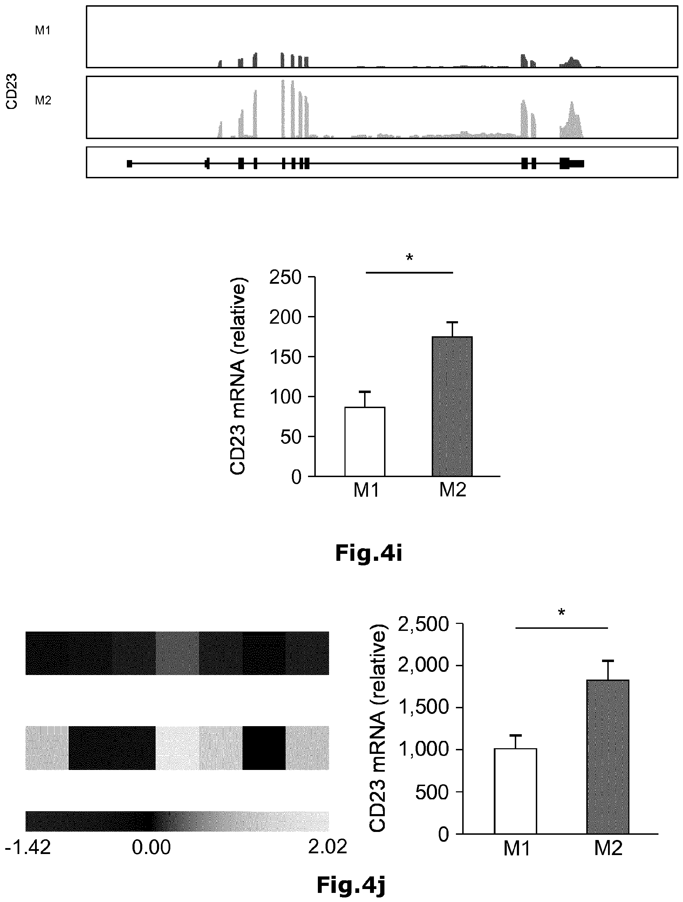

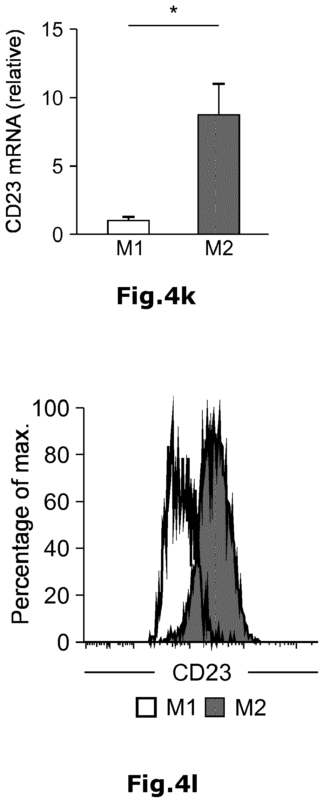

Figure 4: Correlation of RNA-seq, microarray, qPCR, and flow cytometric analysis. (a-d) CD68, (e-h) CD64, and (i-l) CD23 expression in human M1- and M2-like macrophages. (a, e, i) Upper panel, representative images of sequencing reads across the genomic loci of genes expressed in human macrophages. Pictures taken from the Integrative Genomics Viewer (IGV). The height of bars represents the relative accumulated number of 100-bp reads spanning a particular sequence. Gene maps (bottom portion of each panel, oriented 5'-3' direction) are represented by thick (exons) and thin (introns) lines. Lower panel, RPKM values by RNA-seq in M1- and M2-like macrophages. (b, f, j) Left, heatmaps presenting microarray results from M1- and M2-like macrophages from seven donors. Data were z-score normalized. Right, relative mRNA expression. (c, g, k) Relative mRNA expression by qPCR in M1- and M2-like macrophages. (d, h, l) Protein expression was determined by flow cytometry in human M1- and M2-like macrophages. Data are representative of three experiments (RNA-seq, mean and s.e.m.), seven experiments (microarray, mean and s.e.m.), at least seven experiments (qPCR; mean and s.e.m.), and nine experiments (flow cytometry), each with cells derived from a different donor. *P < 0.05 (Student's t-test)

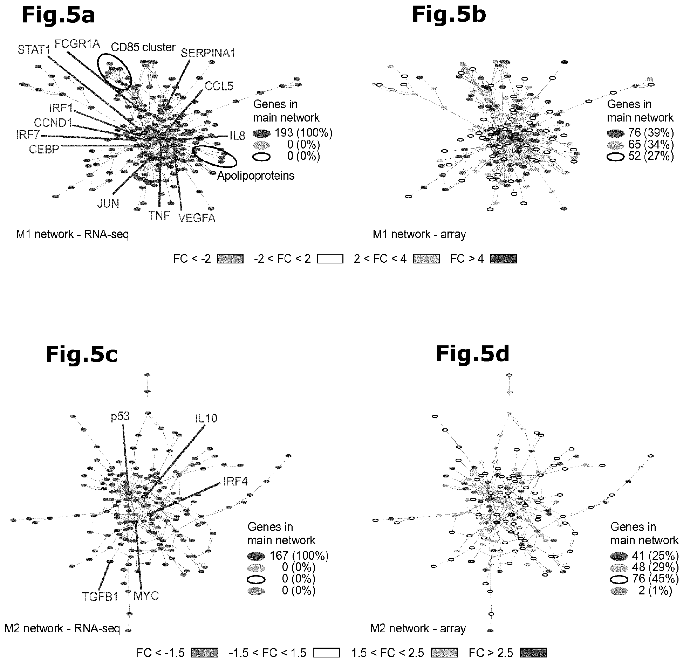

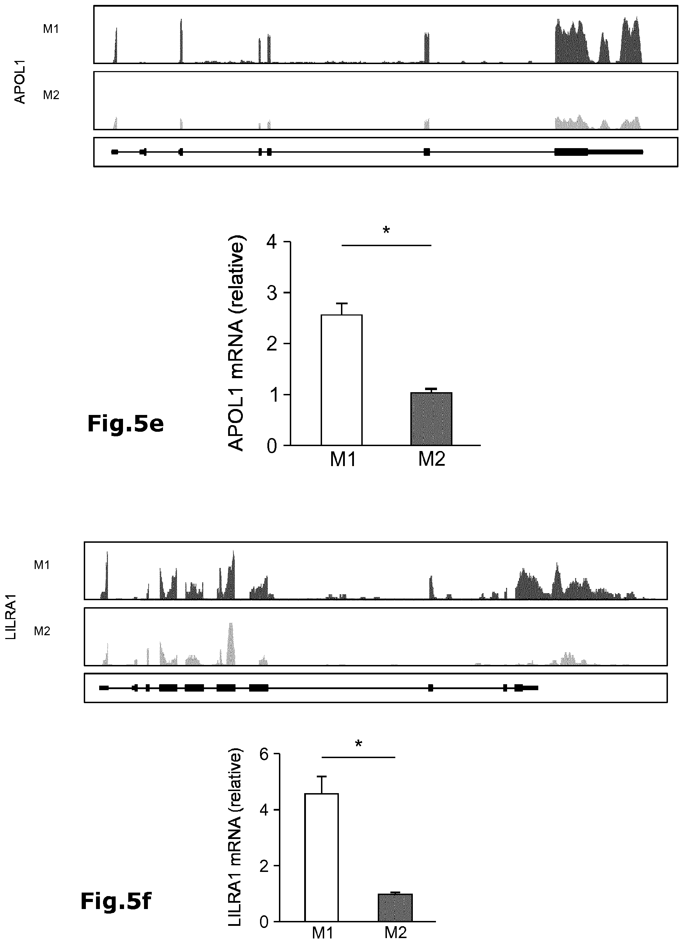

Figure 5: Network analysis of RNA-seq data. (a) Network of genes highly expressed in M1-like macrophages (fold-change >4.0) identified by RNA-seq. (b) Data generated by microarray analysis were loaded into the M1-network established using RNA-seq. (c) Network of genes highly expressed in M2-like macrophages (fold-change >2.5) identified by RNA-seq. (d) Data generated by microarray analysis were loaded into the M2-network established using RNA-seq. All networks were generated using EGAN. (e) APOL1 and (f) LILRA1 expression in human M1- and M2-like macrophages. Upper panel, representative images of sequencing reads across genes expressed in human macrophages as described in Figure 4. Lower panel, relative mRNA expression by qPCR in M1- and M2-like macrophages. Data are representative of three experiments (RNA-seq and qPCR; mean and s.e.m.) each with cells derived from a different donor. *P < 0.05 (Student's t-test)

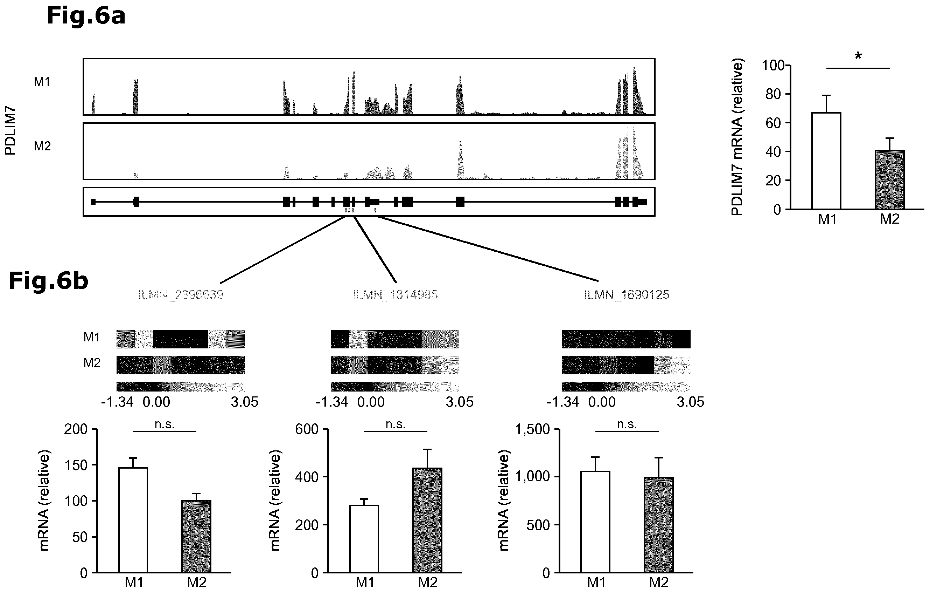

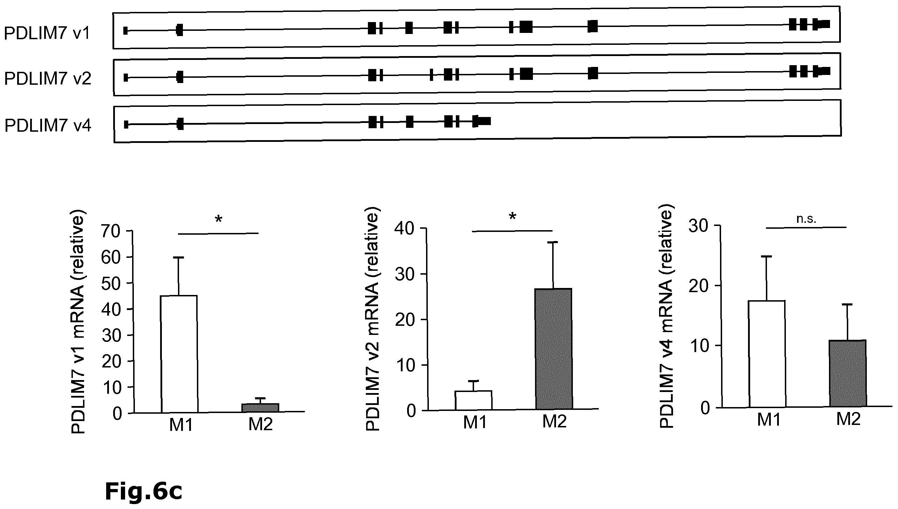

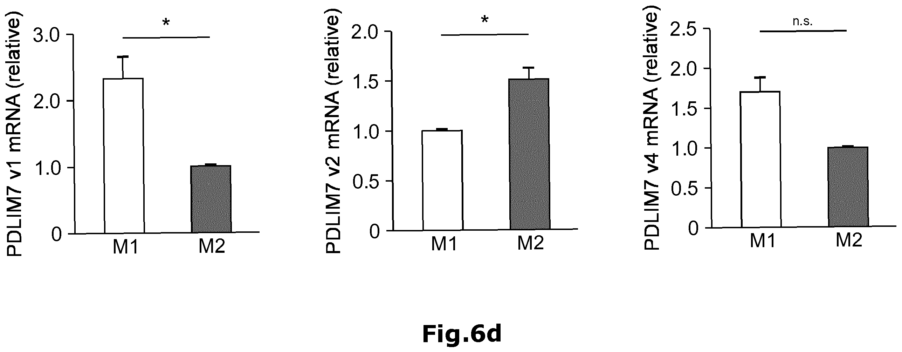

Figure 6: Detection of alternative splicing in human macrophages. (a) Summarized expression of all PDLIM7 transcripts in human M1- and M2-like macrophages. Left, representative images of sequencing reads across genes expressed in human macrophages as described in Figure 4. Right, RPKM values for PDLIM7 by RNA-seq in M1- and M2-like macrophages. (b) Expression of PDLIM7 as determined by microarray analysis using 3 different probes recognizing different parts of the PDLIM7 transcripts as depicted in (a). (c) Upper panel: representation of the 3 different mRNA transcripts from Refseq. Lower panel: abundance of the different transcripts as determined using Cuffdiff. (d) qPCR for the 3 different mRNA transcripts from Refseq in human M1- and M2-like macrophages. Data are representative of three experiments (RNA-seq), seven experiments (microarray analysis) or at least ten experiments (qPCR; mean and s.e.m.), each with cells derived from a different donor. *P < 0.05 (Student's t-test)

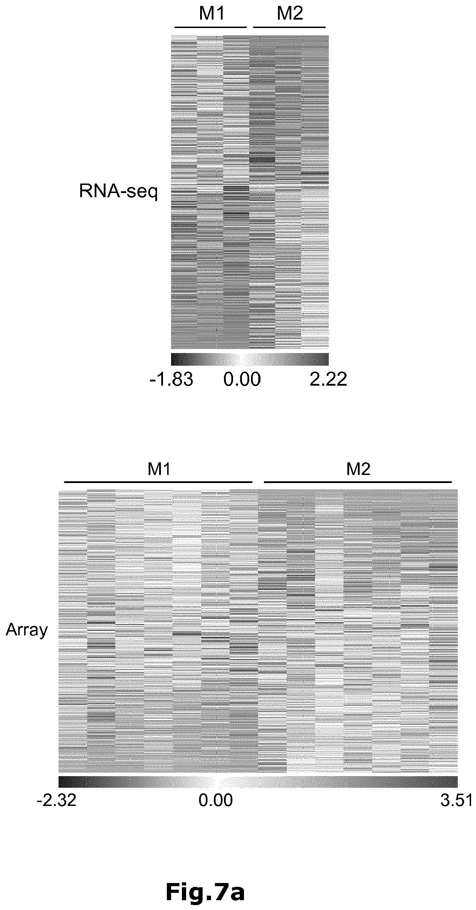

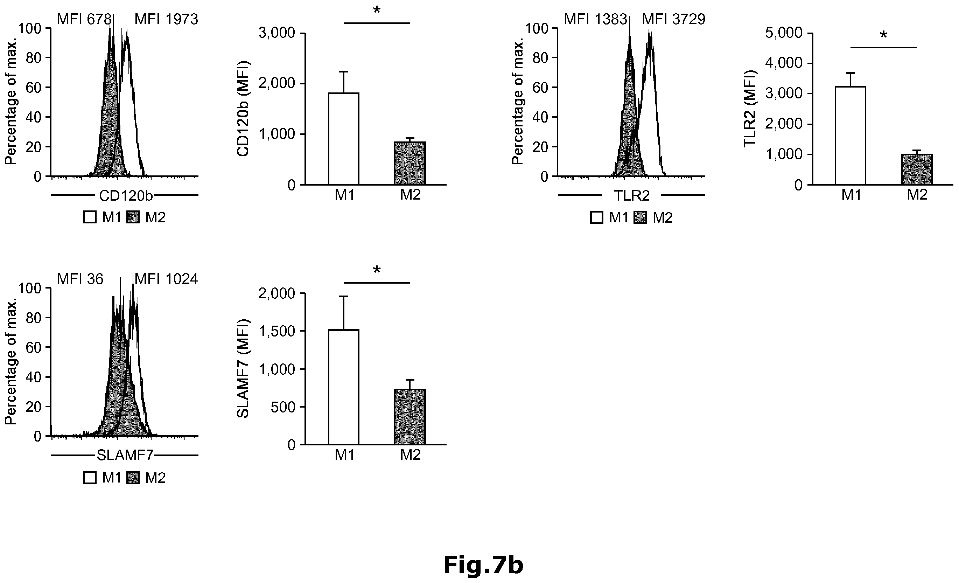

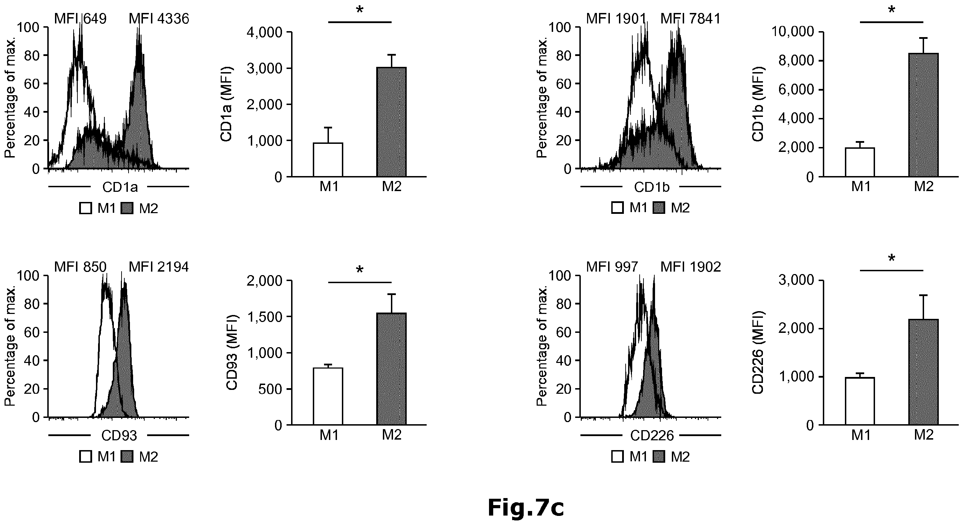

Figure 7: Identification of new macrophage polarization markers based on combined transcriptome analysis. Differentially expressed genes between M1- and M2-like macrophages of the human surfaceome were visualized as heatmaps for RNA-seq (upper panel) and microarray analysis (lower panel). Data were z-score normalized. (b-c) Expression of novel macrophage markers was determined by flow cytometry (left) of M1- and M2-like macrophages generated in the presence of GM-CSF with quantification shown in the graph at the right. Expression of (b) CD120b, TLR2, and SLAM7 as well as (c) CD1a, CD1b, CD93, and CD226. *P < 0.05 (Student's t-test). Numbers in plots indicate mean fluorescence intensity. Data are representative of nine independent experiments (b,c; mean and s.e.m.) each with cells derived from a different donor.

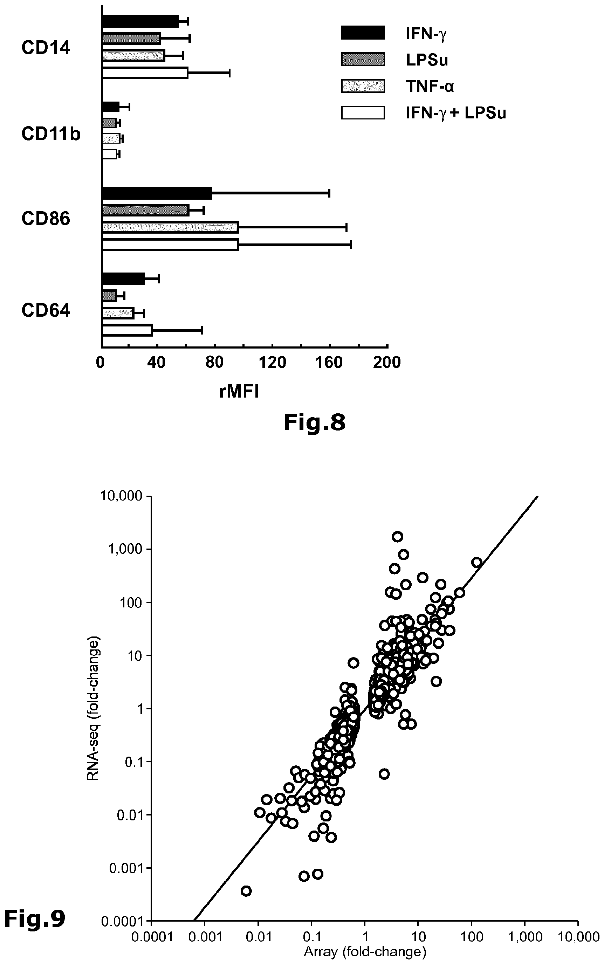

Figure 8: Phenotypic characterization of human M1-like macrophages derived from CD14⁺ peripheral blood monocytes. Expression of classical M1 markers after polarization of GM-CSF generated macrophages with IFN-γ, LPSu, TNF-α or IFN-γ and LPSu. Surface expression of lineage markers CD14 and CD11b as well as surface expression of the typical M1 markers CD86 and CD64 was assessed by flow cytometry.
Figure 9: Comparison of RNA-seq and microarray analysis. Gene expression in M1- versus M2-like macrophages as fold change versus fold change plot comparing microarray analysis with RNA-seq using only Refseq genes differentially expressed in microarrays.

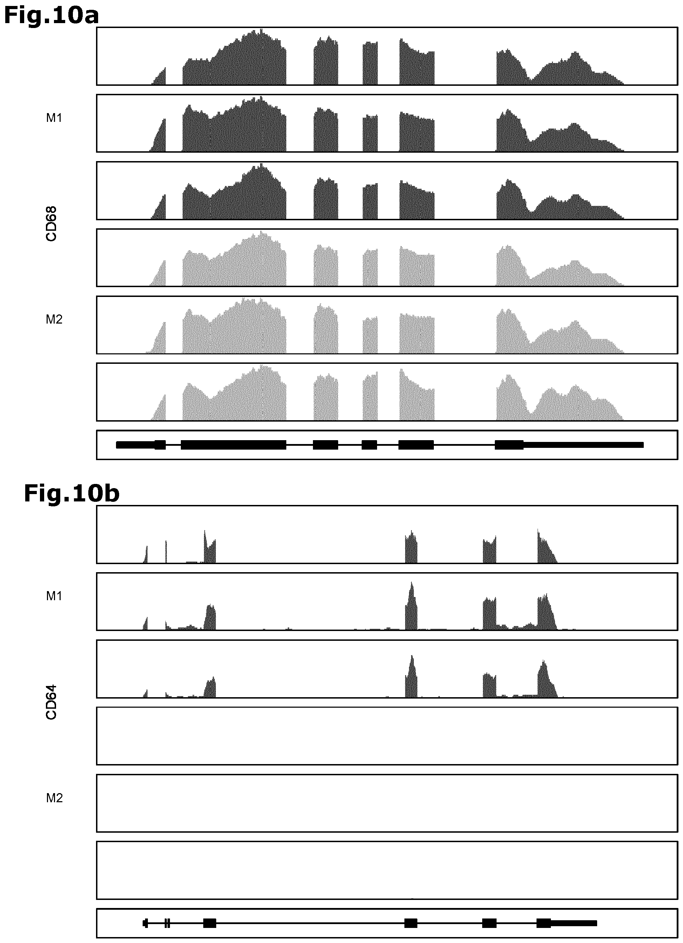

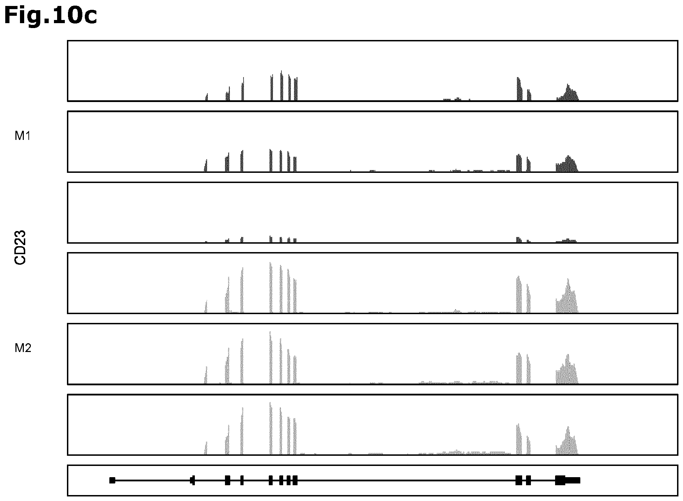

Figure 10: Analysis of classical macrophage markers. (a) CD68, (b), CD64, and (c) CD23 expression in human M1- and M2-like macrophages. Representative images of sequencing reads across genes expressed in human macrophages for all three donors analyzed. Pictures taken from the Integrative Genomics Viewer (IGV). The height of bars represents the relative accumulated number of 100-bp reads spanning a particular sequence. Gene maps (bottom portion of each panel, oriented 5'-3' direction) are represented by thick (exons) and thin (introns) lines.

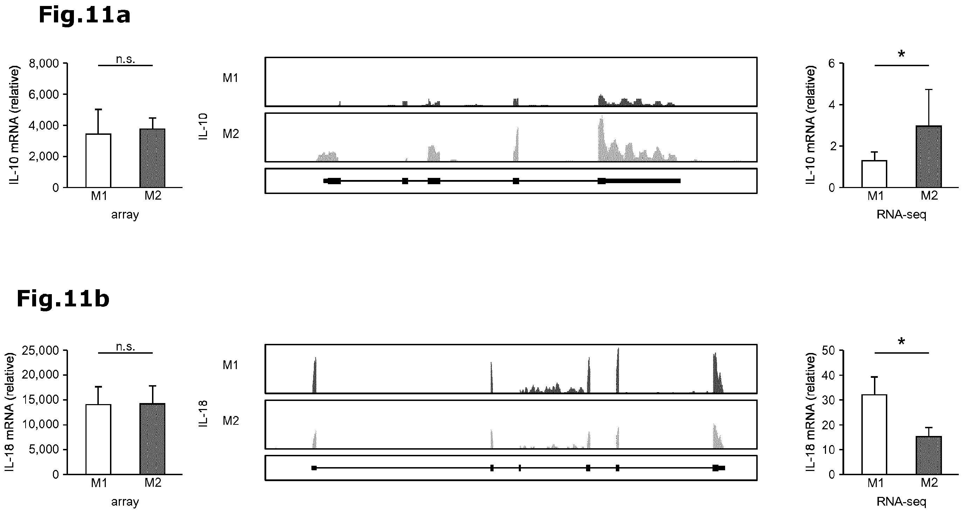

Figure 11: Detection of classical macrophage genes by RNA-seq. (a) IL-10 and (b) IL-18 expression in human M1- and M2-like macrophages. Left, expression as determined by microarray analysis; middle, representative images of sequencing reads across genes expressed in human macrophages. Pictures taken from the Integrative Genomics Viewer (IGV). The height of bars represents the relative accumulated number of 100-bp reads spanning a particular sequence. Gene maps (bottom portion of each panel, oriented 5'-3' direction) are represented by thick (exons) and thin (introns) lines. Right, relative mRNA expression by RNA-seq in M1- and M2-like macrophages. Data are representative of seven (microarrays, mean and s.d.) or three experiments (RNA-seq, mean and s.d.) each with cells derived from a different donor. *P < 0.05 (Student's t-test), n.s. = not significant.

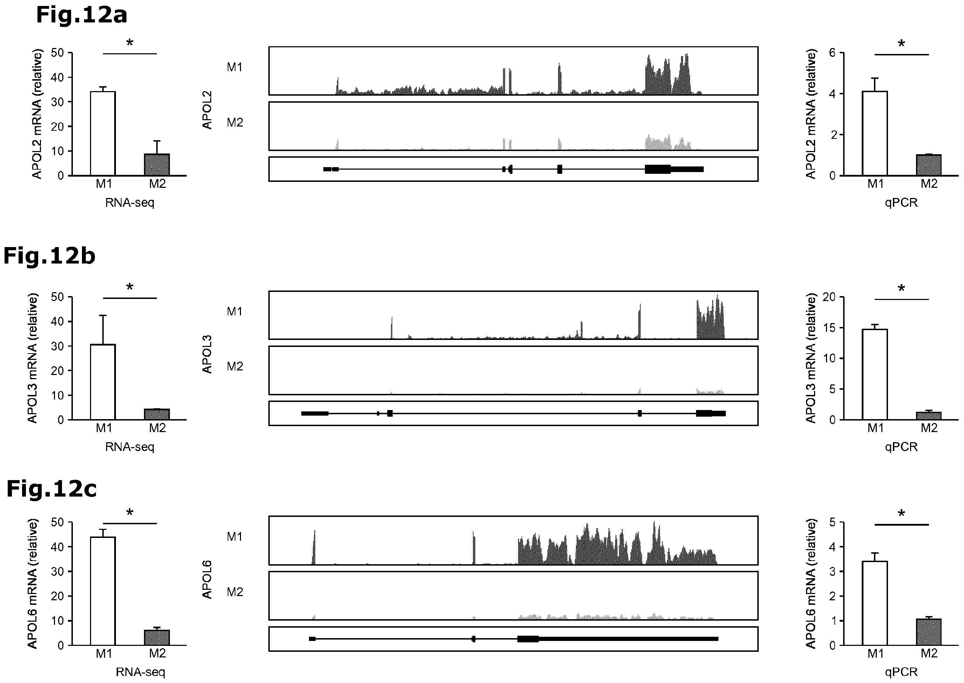

Figure 12: Analysis of the apolipoprotein L family genes in M1- and M2-like macrophages. (a) APOL2, (b) APOL3, and (c) APOL6 expression in human M1- and M2-like macrophages. Left, relative expression as determined by RNA-seq; middle, representative images of sequencing reads across genes expressed in human macrophages. Pictures taken from the Integrative Genomics Viewer (IGV). The height of bars represents the relative accumulated number of 100-bp reads spanning a particular sequence. Gene maps (bottom portion of each panel, oriented 5'-3' direction) are represented by thick (exons) and thin (introns) lines. Right, relative mRNA expression by qPCR in M1- and M2-like macrophages. Data are representative of three experiments (RNA-seq, mean and s.d. and qPCR, mean and s.e.m.) each with cells derived from a different donor. *P < 0.05 (Student's t-test).

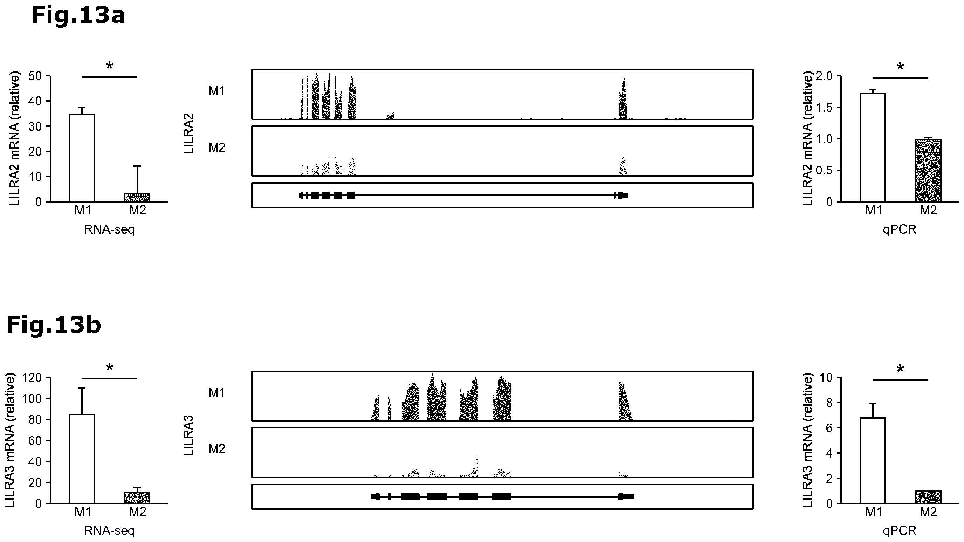

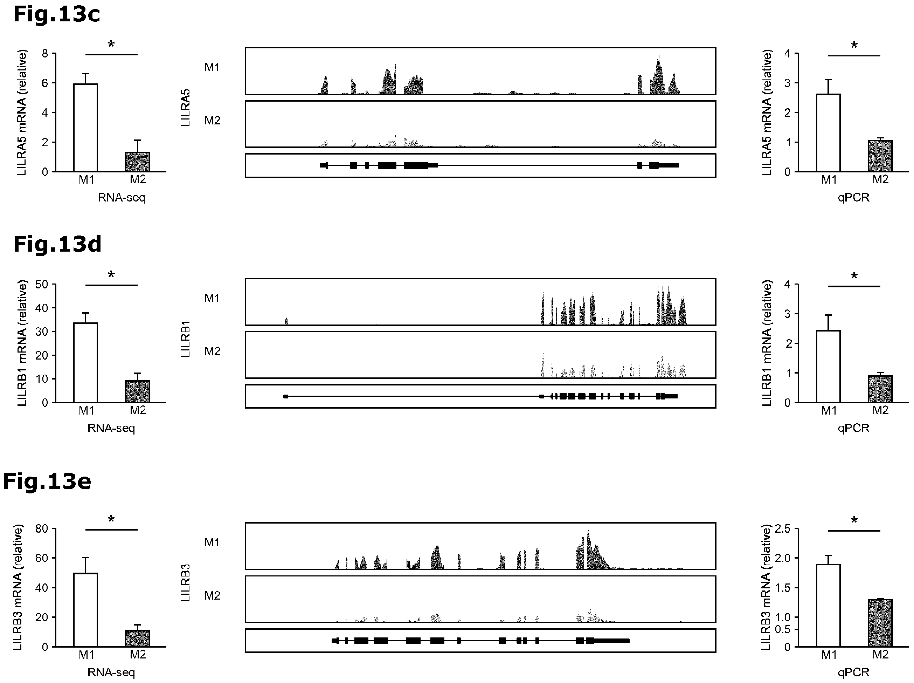

Figure 13: Analysis of the leukocyte immunoglobulin-like receptor family genes in M1- and M2-like macrophages. (a) LILRA2, (b) LILRA3, (c) LILRA5, (d) LILRB1, and (c) LILRB3 expression in human M1- and M2-like macrophages. Left, relative expression as determined by RNA-seq; middle, representative images of sequencing reads across genes expressed in human macrophages. Pictures taken from the Integrative Genomics Viewer (IGV). The height of bars represents the relative accumulated number of 100-bp reads spanning a particular sequence. Gene maps (bottom portion of each panel, oriented 5'-3' direction) are represented by thick (exons) and thin (introns) lines. Right, relative mRNA expression by qPCR in M1- and M2-like macrophages. Data are representative of three experiments (RNA-seq, mean and s.d. and qPCR, mean and s.e.m.) each with cells derived from a different donor. *P < 0.05 (Student's t-test).

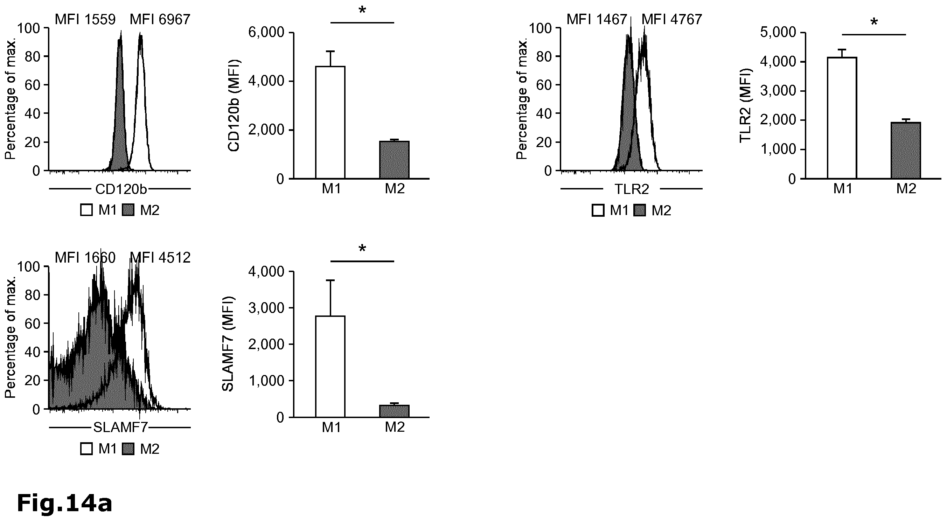

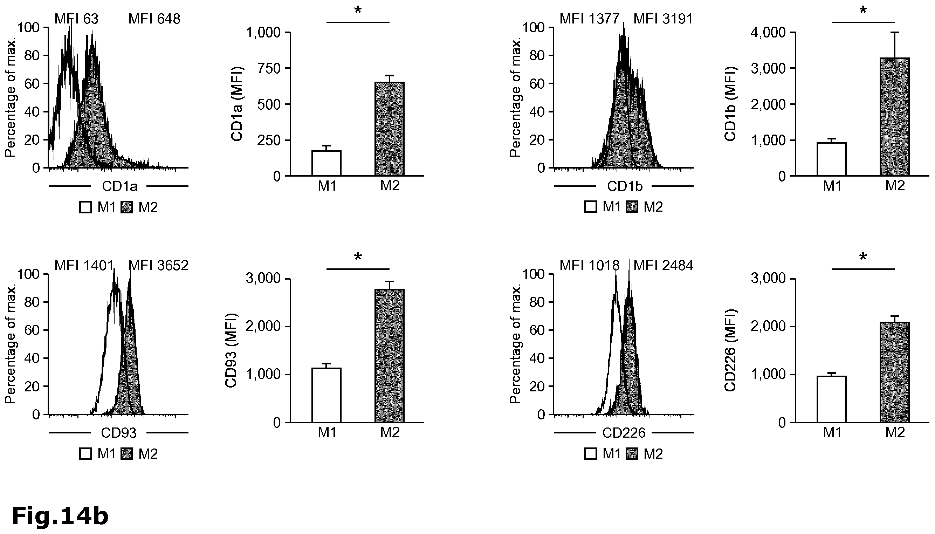

Figure 14: Identification of new macrophage polarization markers based on combined transcriptome analysis. (a-b) Expression of novel M1- and M2-like macrophage markers on CD11b+CD14+ macrophages was determined by flow cytometry (left) of M1- and M2-like macrophages generated in the presence of M-CSF with quantification shown in the graph at the right. Expression of (a) CD120b, TLR2, and SLAM7 as well as (b) CD1a, CD1b, CD93, and CD226. *P < 0.05 (Student's t-test). Numbers in plots indicate mean fluorescence intensity. Data are representative of nine independent experiments (b,c; mean and s.e.m.) each with cells derived from a different donor.

### Detailed Description of the Invention

Macrophages are dynamic cells integrating signals from their microenvironment to develop specific functional responses. Microarray-based transcriptional profiling has established transcriptional reprogramming as an important mechanism for signal integration and cell function of macrophages yet current knowledge on transcriptional regulation is far from complete. RNA sequencing (RNA-seq) is ideally suited to fill this need but also to discover novel marker genes, an area of great need particularly in human macrophage biology. Applying RNA-seq, a high-resolution transcriptome profile of human macrophages under classical (M1-like) and alternative (M2-like) polarization conditions is provided and shows a dynamic range exceeding observations obtained by previous technologies, resulting in a more comprehensive understanding of the transcriptome of human macrophages. In addition, differential promoter usage, alternative transcription start sites, and different coding sequences for 57 gene loci in human macrophages were detected. Moreover, this approach led to the identification of novel M1-associated (CD120b, TLR2, SLAMF7) as well as M2-associated (CD1a, CD1b, CD93, CD226) cell surface markers.

Because of the enormous plasticity of human macrophages, the classification of polarization states on the basis of few cell surface markers will remain a substantial challenge (Murray P.J., Wynn T.A., Nat Rev Immunol. 11(11):723-737 (2011)). Here, we addressed how RNA-seq based high-resolution transcriptome data can be utilized to better understand the biology of macrophage polarization. We observed a significant increase in dynamic range in RNA-seq data resulting in a significantly higher number of genes determined to be significantly differentially expressed. This was true despite the fact that we used seven biological replicates for array analysis but only three samples for RNA-seq. A priori information based network analysis further supported that the increased information content of RNA-seq data uncovered novel aspects of macrophage biology, which was illustrated by the recognition of differential expression of numerous family members of two gene families, namely the apolipoprotein L family and leukocyte immunoglobulin-like receptors. APOLs constitute a new class of apolipoproteins expressed by macrophages as they serve as lytic factors against invading pathogens, e.g. African trypanosomes inducing programmed cell death as well as inhibiting intracellular infection by Leishmania (Pays E., Vanhollebeke B., Curr Opin Immunol. 21(5):493-498 (2009); Samanovic M. et al., PLoS Pathog. 5(1):e1000276 (2009)). LILRs have been associated with balancing the effects of Toll-like receptor signaling, suggesting an important role of LILRs both in the initiation but also cessation of inflammatory responses mediated by macrophages (Brown D. et al., Tissue Antigens. 64(3):215-225 (2004)). Another aspect enhancing our knowledge about the polarization biology of macrophages was the identification of several genes with differential usage of alternative promoters and transcription start sites as well as differential splicing variants between M1- and M2-like macrophages. As visualized for PDLIM7, an intracellular scaffold protein that contains a PDZ domain and three LIM domains linked to mitogenic signaling through actin cytoskeleton organization (Nakagawa N. et al., Biochemical and biophysical research communications 272(2):505-512 (2000)), regulating Tbx5 transcriptional activity (Camarata T. et al., Developmental biology 337(2):233-245 (2010)), and suppressing p53 activity (Jung C.R. et al., The Journal of clinical investigation 120(12):4493-4506 (2010)), RNA-seq revealed significant differences in splice variant usage for M1- and M2-like macrophages potentially linking p53 regulation with macrophage polarization (Matas D. et al., Cell death and differentiation 11(4):458-467 (2004)). Usage of splice variant-specific qPCR reactions supported these findings while this differential regulation was not revealed by microarray analysis. Altogether we detected differential promoter usage, transcription start site usage and splice variant usage in over 50 gene loci, a number that was surprisingly low taking into account that such mechanisms of transcriptional regulation have been suggested for the majority of gene loci in mammalian genomes (Kapranov P. et al., Nature reviews Genetics 8(6):413-423 (2007)).

While studies in other cell systems suggested that RNA-seq data will further improve cell characterization (Ozsolak F., Milos P.M., Nature reviews Genetics 12(2):87-98 (2011); Wang Z. et al., Nature reviews Genetics 10(1):57-63 (2009); Marioni J.C. et al., Genome Res. 18(9):1509-1517 (2008)), the direct assessment of the new technology in macrophage polarization was necessary to estimate its potential information gain. Both, increased dynamic range and the identification of transcripts that were missed by microarray analysis were major reasons for the discovery of novel genes associated with either M1- or M2-polarization. Nevertheless, despite a lower number of informative transcripts in the microarray data, 73 % of the major M1-network was still revealed - at least when using transcripts defined to be enriched in M1-like macrophages. However, this rate dropped to only 54 % in the M2-network and major hubs like MYC and TP53 where only revealed by RNA-seq data in M2-like macrophages. Overall these findings point towards an advantage of RNA-seq data, when the endpoint of the analysis is the identification of novel biological mechanisms.

An important aspect of genomic characterization is the identification of novel marker genes in macrophage polarization (Murray P.J., Wynn T.A., Nat Rev Immunol. 11(11):723-737 (2011)). When focusing on genes being part of the human surfaceome in most cases RNA-seq data revealed larger differences between M1-like and M2-like cells when compared to microarray data. Nevertheless, some genes only reached significant differential expression in the array data clearly pointing toward the necessity to include a large enough number of biological replicates also when applying RNA-seq. On the other hand, a subset of genes showed the well-known background noise effect in the microarray data resulting in non-significant differences between the two cell types. Irrespective of these different shortcomings of the two technologies, the overall differences between the two techniques in this defined gene space were less obvious suggesting that both technologies are well suited for cell surface marker identification. Taken together, we introduced several new marker genes for which we established FACS assays that can be used to distinguish between M1 and M2 polarization of macrophages and that can be combined with the analysis of common macrophage markers.

In the method of aspect (1) of the invention, the relative abundance of the M1-associated cell surface markers is at least 30 % higher in M1-like macrophages than in M2-like macrophages and the relative abundance of the M2-associated cell surface markers is at least 30 % higher in M2-like macrophages than in M1-like macrophages. It is preferred that the abundance of the cell surface marker in the respective M1-like or M2-like macrophage is at least 50 % and preferably at least 70 % higher than in the other macrophage type.

The one or more primers employed in the amplification/resequencing employed in the method of aspect (2) of the invention are derived from the respective marker nucleic acid. Preferably said primers have at least 12, more preferably at least 15, most preferably at least 19 contiguous nucleotides of the respective marker nucleic acid sequence.

Similarly, the one or more probes employed in the method of aspect (3) of the invention are derived from the respective marker nucleic acid. Preferably said probes have a length that allows for a selective hybridization to the marker nucleic acid. The probe may also be labeled with a suitable marker molecule (e.g. with a fluorescence marker) to allow the detection of the resulting probe-surface marker nucleic acid complex.

Such probes may also be utilized in a hybridization array of aspect (4) of the invention.

The binding molecules utilized in aspect (5) of the invention include antibodies, preferably monoclonal antibodies. Moreover said binding molecules may be labeled with marker molecules, preferably fluorescence markers. Particularly preferred binding molecules include the FITC-labeled CD1b, CD93, CD226 and anti-TLR2, PE-labeled CD120b and anti-SLAMF7, and PE-Cy5-labeled CD1a monoclonal antibodies. Further it is preferred that the method of aspect (5), notably if it is utilized to isolate the M1-like macrophages or M2-like macrophages, is performed on a FACS sorter.

The identification of novel marker genes distinguishing human M1-like and M2-like macrophages opens new avenues towards understanding the biology of differentially polarized macrophages. One of the M1-marker identified in this study, namely CD120b (TNFR2) has been linked to cell survival, activation and even proliferation in other cell types such as T cells (Faustman D., Davis M., Nat Rev Drug Discov. 9(6):482-493 (2010)). In contrast to TNFR1, TNFR2 preferentially leads to NFκB activation. Whether this is true in myeloid cells as well requires further investigation. However, earlier studies already suggested that production of TNF-α in macrophages might be interpreted as a phenotype-stabilizing feed-forward loop (Popov A. et al., The Journal of clinical investigation. 116(12):3160-3170 (2006)) and TNFR2 might actually play an important role in such a process.

SLAMF7 was originally identified as a NK cell-associated surface molecule (Boles K.S. et al., Immunol Rev. 181:234-249 (2001)). Subsequently, it was shown to be expressed on lymphocytes and monocytes (Murphy J.J. et al., Biochem J. 361(Pt 3):431-436 (2002)). More recently, a reduced expression on monocytes and NK cells with a simultaneous increase of SLAMF7 on B cells was observed in patients with lupus erythematosus (Kim J.R. et al., Clin Exp Immunol. 160(3):348-358 (2010)). The strongest link to SLAMF7 as an M1 marker gene comes from observations in intestine allograft rejection, demonstrating that tissue macrophages derived from patients rejecting the graft showed elevated levels of SLAMF7 (Ashokkumar C. et al., Am J Pathol. 179(4):1929-1938 (2011)). It would be interesting to see if macrophages in other settings of transplant rejection are also enriched for this novel M1 marker gene. Considering the identification of single specific marker genes for macrophage polarization our findings clearly point to the necessity for multi-parameter analysis instead. This can be exemplified by the differential expression of CD1a and CD1b, two cell surface molecules that are mainly studied in context of antigen presentation by dendritic cells (Porcelli S.A., Modlin R.L., Annu Rev Immunol. 17:297-329 (1999)). Previous reports suggested upregulation of CD1 proteins on human monocytes by GM-CSF (Kasinrerk W. et al., J Immunol. 150(2):579-584 (1993)). However, we clearly present evidence that expression is induced in both M-CSF and GM-CSF driven macrophages and polarization towards M2-like macrophages is significantly increasing expression of CD1a and CD1b suggesting that they might be up-regulated on tissue macrophages in an M2-driving environment. This is similarly true for CD93, which was originally identified to be expressed on early hematopoietic stem cells and B cells (Greenlee-Wacker M.C. et al., Curr Drug Targets. (2011)). CD93 is involved in biological processes such as adhesion, migration, and phagocytosis (McGreal E.P. et al., J Immunol. 168(10):5222-5232 (2002); Nepomuceno R.R. et al., J Immunol. 162(6):3583-3589 (1999)). CD93 expressed on myeloid cells can be shed from the cell surface and the soluble form seems to be involved in differentiation of monocytes towards a macrophage phenotype (Jeon J.W. et al., J Immunol. 185(8):4921-4927 (2010)). Since soluble CD93 has been implicated in inflammatory responses, it will be important to further elucidate how polarization-induced differential expression of CD93 contributes to specific inflammatory responses. Another surprising finding is the differential expression of CD226 between human M1- and M2-like macrophages, a molecule initially shown to be involved in cytolytic function of T cells (Shibuya A. et al., Immunity. 4(6):573-581 (1996)). Subsequently, it could be shown that CD226 has additional functions including the regulation of monocyte migration through endothelial junctions (Reymond N. et al., J Exp Med. 199(10):1331-1341 (2004)). Similar to the other M2-associated markers, so far little is known about CD226 on polarized macrophages. Since CD226 expression levels on lymphocytes have been implicated in autoimmune diseases (Sinha S. et al., PLoS One. 6(7):e21868 (2011)) further research is necessary to understand its role in the myeloid compartment during such processes.

Overall, by using RNA-seq we introduce a high-resolution transcriptome analysis of human macrophages unraveling novel insights into macrophage polarization. While previously established transcriptome datasets addressing macrophage biology are still very suitable to assess important biological and medical questions, a deeper understanding of transcriptional regulation during macrophage polarization will require higher resolution that is provided by current and future RNA-seq technologies. Moreover, the novel cell surface markers will help to better understand macrophage programs and functions in human disease.

The Invention is further described in the following Examples.

### Examples

### Materials and Methods

Abbrevations: LPSu, ultrapure LPS; GEP, gene expression profiling; PCA, principle component analysis; RNA-seq, RNA sequencing technologies; MFI, mean fluorescence intensity; EGAN, exploratory gene association network; RPKM, Reads Per Kilobase of exon model per Million mapped reads; FC, fold change; TSS, transcription start sites; CDS, coding sequences.

Cell isolation from healthy blood donors: Peripheral blood mononuclear cells (PBMC) were obtained by Pancoll (PAN-Biotech, Aidenbach, Germany) density centrifugation from buffy coats from healthy donors obtained following protocols accepted by the institutional review board at the University of Bonn (local ethics vote no. 045/09). Informed consent was provided for each specimen according to the Declaration of Helsinki. CD14⁺ monocytes were isolated from PBMC using CD14-specific MACS beads (Miltenyi Biotec) according to the manufacturer's protocol (routinely >95 % purity).

Generation of macrophages: CD14⁺ monocytes were cultured in 6-well plates in RPMI1640 medium containing 10 % FCS and differentiated into immature macrophages using GM-CSF (500 U/ml) or M-CSF (100 U/ml) for 3 days. Growth-factor containing medium was exchanged on day 3 and cells were polarized for 3 days with the following stimuli: IFN-γ (200 U/ml), TNF-α (800 U/ml), ultrapure LPS (LPSu, 10 µg/ml), IL-4 (1,000 U/ml), IL-13 (100 U/ml), or combinations thereof (all from Immunotools, Friesoythe, Germany).

Monoclonal antibodies and flow cytometry: Cells were stained after FcR blockade incubating cells in PBS with 20 % FCS for 10 min at 4 °C using the following monoclonal antibodies (all from Becton Dickinson (BD), BioLegend, or eBioscience): FITC-labeled CD1b, CD23, CD93, CD226, anti-HLA-DR, anti-TLR2; PE-labeled CD64, CD68, CD120b, anti-SLAMF7; PE-Cy5-labeled CD1a; PerCP-Cy5.5-labeled CD209; APC-labeled CD86; Pacific Blue-labeled CD11b; and APC-Cy7-labeled CD14 with matched isotype antibodies as controls. Intracellular staining of CD68 was performed using the BD Cytofix/Cytoperm kit (BD). Data were acquired on a LSR II (BD) and analyzed using FlowJo software (Tree Star). RNA isolation: 5x10⁶-2x10⁷ macrophages were harvested, subsequently lysed in TRIZOL (Invitrogen) and total RNA was extracted according to the manufacturer's protocol. The precipitated RNA was solved in RNAse free water. The quality of the RNA was assessed by measuring the ratio of absorbance at 260 nm and 280 nm using a Nanodrop 2000 Spectrometer (Thermo Scientific) as well as by visualizing the integrity of the 28S and 18S band on an agarose gel. Quantitative PCR conditions and primer sequences: 500 ng RNA was reverse transcribed using the Transcriptor First Strand cDNA Synthesis Kit (Roche Diagnostics). qPCR was performed using the LightCyclerTaqman master kit with GAPDH as reference on a LightCycler 480 II (Roche). qPCR primer sequences are summarized in Table 2.

Isoform specific PCR to identify alternative splicing events were performed using the Maxima SYBR Green/Fluorescein qPCR Master Mix (Fermentas). The relative enrichment of each isoform relative to GAPDH was calculated using the 2^{-ΔΔCT} method. qPCR primer sequences are listed in Table 3.

Microarray-based transcriptional profiling and bioinformatic analysis of microarray data: Isolated RNA was further purified using the MinElute Reaction Cleanup Kit (Qiagen). Biotin labeled cRNA was generated using the TargetAmp Nano-g Biotin-aRNA Labeling Kit (Epicentre). Biotin labeled cRNA was hybridized to Human HT-12V3 Beadchips (Illumina) and scanned on an Illumina HiScanSQ system. Raw intensity data were exported with BeadStudio 3.1.1.0 (Illumina) and subsequently analysed using R (R Development Core Team. R: A Language and Environment for Statistical Computing (2011)). After quantile normalization non-informative genes (coefficient of variation < 0.5) were excluded. From the resulting data sets we extracted a list of genes with a significantly different expression in macrophage subtypes. Variable genes were plotted as heatmaps with hierarchical clustering using the correlation coefficient as a distance measure for the samples and the average of each cluster for cluster formation of the genes. Expression values are visualized with shading ranging from light gray (high expression) over white (indermediate expression) to dark gray (low expression). Principal component analysis (PCA) was performed using the "pcurve" package in R. Microarray data can be accessed under GSE35449.

RNA-seq and data analysis: Sequencing and analysis were performed individually on M1-like and M2-like macrophages from 3 independent donors. Total RNA was converted into libraries of double stranded cDNA molecules as a template for high throughput sequencing using the Illumina CBot station and HiScanSQ following the manufacturer's recommendations using the Illumina TruSeq RNA Sample Preparation Kit. Shortly, mRNA was purified from 5-10 µg of total RNA using poly-T oligo-attached magnetic beads. Fragmentation was carried out using divalent cations under elevated temperature in Illumina proprietary fragmentation buffer. First strand cDNA was synthesized using random oligonucleotides and SuperScript II. Second strand cDNA synthesis was subsequently performed using DNA Polymerase I and RNase H. Remaining overhangs were converted into blunt ends via exonuclease/polymerase activities and enzymes were removed. After adenylation of 3' ends of DNA fragments, Illumina PE adapter oligonucleotides were ligated to prepare for hybridization. In order to select cDNA fragments of preferentially 200 bp in length the library fragments were separated on a 2 % (w/v) agarose gel. The corresponding gel-fraction for each library was excised and purified using the QIAquick gel extraction kit (Qiagen). DNA fragments with ligated adapter molecules were selectively enriched using Illumina PCR primer PE1.0 and PE2.0 in a 15 cycle PCR reaction. Products were purified (QIAquick PCR purification kit) and quantified using the Agilent high sensitivity DNA assay on a Bioanalyzer 2100 system (Agilent). After cluster generation, 100 bp paired-end reads were generated and analyzed using CASAVA 1.8. Alignment to the human reference genome hg19 from UCSC was performed stepwise. First, all reads passing the chastity filter were aligned to the reference genome. Next, reads were aligned to the RNA reference transcriptome. Based on these alignments the numbers of reads aligning to intragenic regions, or intergenic regions, respectively, were calculated. In addition the numbers of reads mapping to exonic and intronic regions as well as to splice sites were calculated based on the UCSC annotation file. Reads per kilobase of exon model per million mapped reads (RPKM) values for Refseq genes were established using CASAVA 1.8. In order to identify reads spanning altered splicing events or gene fusion breakpoints we also analyzed reads using TopHat and Bowtie. Results were further processed using Cufflinks and Cuffdiff (Trapnell C. et al., Nature biotechnology 28(5):511-515 (2010)).

A priori information-based network analysis using EGAN software: To visualize connectivity between genes in high-throughput datasets contextual network graphs were generated based on a priori knowledge stored in literature, pathway, interaction, or annotation term databases by EGAN (exploratory gene association network) Paquette J., Tokuyasu T., Bioinformatics 26(2):285-286 (2010). To visualize the transcriptional regulation of genes enriched in M1 respectively M2, array data were used and fold change differences calculated using unpolarized macrophages as comparison. Genes with a FC > 2 for M1 and FC > 1.65 for M2 were visualized; represented is the major network. Using the network topology established for M1-like macrophages the expression values for M2-like macrophages were plotted and vice versa. For comparison of network components and density between RNA-seq and array data, the network was first visualized for the RNA-seq data (FC > 4 for M1 and FC > 2.5 for M2). Keeping the network topology, genes were marked according to their fold change when visualizing the array-based network. Graphs for genes enriched in M1 respectively in M2 were generated independently.

Statistical analysis: Student's *t*-tests were performed with SPSS 19.0 software.
Example 1: Generation of human M1- and M2-like macrophages as a model system. To establish a high-resolution transcriptome of human macrophages as a result of specific polarization signals, we used classical (M1-like) and alternative (M2-like) polarization of human macrophages as a model system. Since both M-CSF and GM-CSF have been described to differentiate macrophages from blood-derived CD14⁺ monocytes, we first compared the two different stimuli in respect to macrophage polarization and used expression of well-known macrophage markers as the initial readout (Martinez F.O. et al., The Journal of Immunology 177(10):7303-7311 (2006); Hamilton J.A., Nat Rev Immunol. 8(7):533-544 (2008)). For classical polarization we primarily used IFN-γ as the model stimulus and IL-4 for alternative polarization. When assessing the macrophage surface marker CD11b, the total percentage of CD11b⁺ cells under M1 and M2 polarization conditions was similar while the MFI was slightly higher in M2-like macrophages independent of the usage of GM-CSF or M-CSF (Figure 1a). Further, we observed high expression of CD14 on all cells under M1 and M2 polarizing conditions irrespective of GM-CSF or M-CSF differentiation with a higher CD14 expression in M1-like macrophages (Figure 1b). For both classical macrophage markers CD68 and MHC class II molecules (Figure 1c and 1d) we observed no differences in all four conditions tested. Of note, when the IL-4 signal was provided to monocytes from the beginning of the differentiation period, immature dendritic cells were generated with a typical loss of macrophage markers such as CD14 or CD68 (data not shown).
   When assessing markers previously associated with M1 or M2 polarization (Mantovani A. et al., Trends Immunol. 23(11):549-555 (2002)), a selective induction of the M1 marker CD64 in M1-like macrophages was observed in cultures differentiated with both GM-CSF and M-CSF (Figure 1e) while CD86 only showed an M1-specific expression in GM-CSF differentiated cells (Figure If). Assessment of these markers following other M1-associated polarization signals, e.g. LPS, TNF-α or combinations thereof resulted in comparable results (Figure 8). Inversely, strong induction of the M2-marker CD23 was observed in IL-4 polarized macrophages with significantly higher induction in GM-CSF polarized M2-like macrophages (Figure 1g). For further experiments we therefore differentiated monocytes with GM-CSF for 3 days prior to polarization with either IFN-γ or IL-4 as the model signals.
Example 2: Characterization of M1- and M2-like macrophages by microarray-based gene expression profiling. Most recently it has been suggested that assessment of macrophage polarization in humans cannot solely rely on few cell surface markers but should be accommodated by gene expression profiling (Murray P.J., Wynn T.A., Nature reviews Immunology 11(11):723-737 (2011)). Using one of the most recent array generations, gene expression profiling was performed on unpolarized and polarized macrophages derived from seven healthy donors. To determine sample relationships, PCA (Figure 2a) and hierarchical clustering (Figure 2b) based on variable genes were performed and showed segregation of the samples by polarization state. Comparing our data with publically available datasets from M1- and M2-like macrophages generated with earlier array versions we observed concordant gene expression patterns (data not shown) (Martinez F.O. et al., The Journal of Immunology 177(10):7303-7311 (2006)). Heatmap visualization of known M1- and M2-like macrophage markers (Figure 2c) further demonstrated that the genes coding for the surface molecules FCGR1A and FCGR1B (both representing CD64) and CD86, the cytokine/chemokine genes CXCL10, CXCL9, IL-1B, IL-6, CXCL11, TNF, IL-23A, and the genes coding for the intracellular protein GBP1 were increased in M1-like macrophages, results similar to what has been previously reported for M1 polarization (Martinez F.O. et al., The Journal of Immunology 177(10):7303-7311 (2006); Mantovani A. et al., Immunity 23(4):344-346 (2005); Mosser D.M., Edwards J.P., Nat Rev Immunol. 8(12):958-969 (2008)). Inversely, M2-associated genes coding for the surface molecules FCER2 (CD23), IL27RA, and CLEC4A, the chemokine genes CCL22, CCL18, and CCL17, and the intracellular protein F13A1 were increased in the M2-like macrophages (Mantovani A. et al., Immunity 23(4):344-346 (2005); Mosser D.M., Edwards J.P., Nat Rev Immunol. 8(12):958-969 (2008); Wirnsberger G. et al., Eur J Immunol. 36(7):1882-1891 (2006)).
   To further illustrate differential macrophage polarization, we generated a priori knowledge based M1-associated (Figure 2d) and M2-associated (Figure 2e) networks applying EGAN (Paquette J., Tokuyasu T., Bioinformatics 26(2):285-286 (2010)) using genes significantly upregulated in M1- (FC > 2) respectively M2-polarized cells (FC > 1.65). When applying expression values from the comparison of M2-like with M0 macrophages on the M1-associated network, the vast majority of genes showed either no change or even a reduction in expression, likely to represent an active repression of M1-associated genes in M2-like macrophages (Figure 2d). Only few genes showed a simultaneous increase in expression, and these genes represented common cell cycle associated genes. Similarly, members of the M2-associated network were mostly not changed or even reduced in M1-like macrophages (Figure 2e).
Example 3: Increase in overall transcriptome information by RNA-seq. Gene expression profiling using microarrays has recently been suggested to be inferior to newer sequencing based technologies in providing genome-wide transcriptome information (Wang Z. et al., Nature reviews Genetics 10(1):57-63 (2009)). To address the potential information increase for macrophages, RNA-seq was performed on *in vitro* generated M1- and M2-like macrophages. After quality filtering, we obtained 15.0 ± 2.8 million and 20.4 ± 9.2 million reads for the M1- and M2-like macrophage cDNA libraries (Table 1). Consistent with RNA-seq data obtained from other eukaryotic cells (Ramskold D. et al., PLoS computational biology 5(12):e1000598(2009)) the majority of sequencing reads for M1- and M2-like macrophages mapped to annotated exons (Refseq transcripts). The remaining reads mapped to exon-intron boundaries, introns, or other uncharacterized intergenic regions (Table 1). RPKMs are measures of individual transcript abundance in RNA-seq datasets and have been shown to be highly accurate across multiple cell types (Mortazavi A. et al., Nature methods 5(7):621-628 (2008)). We used CASAVA to assign RPKMs. To compare RNA-seq and microarray data we cross-annotated RNA-seq and microarray data using HGNC symbols. In human M1- and M2-like macrophages, 11317 and 11466 Refseq genes were expressed applying a previously defined optimal threshold (0.3 RPKM) for gene expression (Figure 3a) (Ramskold D. et al., PLoS computational biology 5(12):e1000598 (2009)). The present call rate for Refseq genes for M1-(n=10155) and M2-like macrophages (n=10418) was only slightly lower when using microarrays (Figure 3a). Furthermore, when assessing the levels of mRNA expression on a global scale a high correlation between RNA-seq and microarray data - similar to other cells (Mortazavi A. et al., Nature methods. 5(7):621-628 (2008)) - was observed for M1- and M2-like macrophages (Figure 3b).
Example 4: RNA-seq reveals differential expression at significantly higher resolution. RNA-seq showed a significantly increased dynamic range over background mainly due to significantly lower background levels. This suggested that the assessment of differential expression using RNA-seq might lead to an improved resolution in comparison to array-based data. Applying standard filter criteria (FC ≥ 1.5, p < 0.05, RPKM ≥ 0.3) revealed a total of 1736 genes elevated in M1- versus M2-like macrophages by RNA-seq, while 834 genes were recognized by array analysis (Figure 3c). Similarly, 822 genes were identified as being elevated in M2- versus M1-like macrophages by RNA-seq, while 786 genes were detected by array analysis (Figure 3d). More importantly, when categorizing differentially expressed genes according to their level of differential expression, RNA-seq data clearly revealed up to 4-fold more genes with FC > 4 for M1- and M2-associated genes (Figure 3c and d), which was similarly true for M1-associated genes at lower levels (1.5 < FC < 4). To reveal potential reasons for this difference on the single-gene level we utilized FC-FC plotting, correlating RNA-seq and array-based data for individual genes (Figure 3e). The majority of genes showed similar behavior in both RNA-seq and microarray experiments, albeit the relative differences were higher in RNA-seq data (Figure 3e). Altogether, we observed a considerable increase in the dynamic range of fold-differences in RNA-seq data with differences spanning six orders of magnitude in contrast to only four orders of magnitude in the microarray data (Figures 3e and 9). In addition, there was a significant number of genes showing differential expression in RNA-seq data (e.g. DUOX1, DUOX2, TBX21, GBP7) but not in the array data suggesting that the array data are not informative for this class of genes. As anticipated, when using Venn diagrams with a defined cutoff (-2 ≥ FC ≥ 2, p < 0.05, RPKM ≥ 0.3) for data presentation (Figure 3f), both RNA-seq and array data revealed 595 genes to be differentially expressed, but RNA-seq revealed 900 additional genes. Surprisingly, 308 genes were classified as being differentially expressed by array analysis alone (Figure 3f). When further assessing these genes, it became apparent that these genes show a similar trend in the RNA-seq data but these differences did not yet reach statistical significance (Figure 3g). In contrast, genes only identified by RNA-seq, clearly showed no differences when assessed by array analysis (Figure 3h). Visualization of typical marker genes as depicted for array data in Figure 2c demonstrated a comparable differentiation of M1- and M2-like macrophages when assessed by RNA-seq (Figure 3i).
Example 5: Exon resolution transcriptome analysis of known macrophage markers. Another advantage of RNA-seq is to resolve gene expression on the exon level (Figure 4). For the macrophage related genes CD68 (Figure 4a-d), CD64 (Figure 4e-h) and CD23 (Figure 4i-l), RNA-seq data were visualized for the genomic loci of the respective genes and compared with array, qPCR, and FACS data. For CD68, RNA-seq data revealed similarly high expression for M1 and M2 macrophages for all exons with little variance in expression levels between donors (Figures 4a and 11). Slightly higher variance was observed for both microarray (Figure 4b) and qPCR data (Figure 4c) while protein levels showed equal expression in all samples analyzed (Figure 4d). For CD64, RNA-seq revealed complete absence of expression for all exons in M2-like macrophages with high expression in M1-like macrophages (Figure 4e), which was similarly observed by other technologies (Figure 4f-h). For CD23, protein data suggest significantly elevated expression on M2-like macrophages with low level expression on M1-like macrophages (Figure 4l), a result which was also observed for RNA-seq data (Figure 4i) as well as array (Figure 4j) and qPCR (Figure 4k). Similar results were obtained for other marker genes (data not shown). Additionally, we were able to detect classical M1/M2-markers not accessible using microarrays (Figure 11), suggesting that high-resolution RNA-seq data are predestined for exploration of genes not detectable using microarrays, novel marker genes, as well as biological principles of macrophage polarization. Example 6: RNA-seq ameliorates network-based analysis in M1- and M2-like macrophages. To understand if RNA-seq would also enhance the understanding of biological principles of macrophage polarization we applied network analysis based on a priori information assessing the information content of RNA-seq data in comparison to array data. Genes expressed at elevated levels in M1 RNA-seq data (FC > 4) were used for network generation (Figure 5a). This primary RNA-seq based M1 network was subsequently used to visualize array-based gene expression (Figure 5b). When genes at a lower level of differential expression (FC > 2) were included 73 % of the network was revealed in the array data and central hubs of the network were also categorized as being highly (FC > 4) differentially expressed. However, only RNA-seq data revealed two gene clusters of immunomodulating proteins highly enriched in the M1 network, namely apolipoproteins L (APOL) (Figures 5a and 12) and the leukocyte immunoglobulin-like receptor (LILR) family (Figures 5a and 13) (Pays E., Vanhollebeke B., Curr Opin Immunol. 21(5):493-498 (2009); Samanovic M. et al., PLoS Pathog. 5(1):e1000276 (2009); Brown D. et al., Tissue Antigens. 64(3):215-225 (2004)). As exemplified for LILRA1 and APOL1 both genes were clearly identified by RNA-seq and qRT-PCR (Figure 5e and f) but not by microarray analysis (data not shown). Applying the RNA-seq data-based M2 network (Figure 5c) to the array data (Figure 5d) revealed only 54 % elevated genes and major network hubs were not revealed at all. Taken together, RNA-seq data were clearly enriched for biological a priori information in both M1 and M2 polarization.
Example 7: Identification of splice variants and RNA chimaera in differentially stimulated human macrophages. It has recently been suggested that cell differentiation can result in usage of alternative gene transcripts or isoform switching (Trapnell C. et al., Nature biotechnology 28(5):511-515 (2010)). We applied Cufflinks and Cuffdiff to illuminate switches in dominant promoter usage, transcription start sites (TSS), and coding sequences (CDS) (Trapnell C. et al., Nature biotechnology 28(5):511-515 (2010)). This analysis revealed 9 genes with alternative promoters (Table 4), 28 genes using alternative TSS (Table 5), and 20 genes with different CDS in M1- and M2-like macrophages (Table 6). We analyzed one of these genes in greater detail. For the gene encoding PDZ and LIM domain 7 (enigma) (PDLIM7) we observed a slight but significant increase in M1-like macrophages for the complete locus in RNA-seq data (Figure 6a) while the probes on the microarray revealed no significant changes (Figure 6b). Previous screening projects suggested three different CDS for PDLIM7. Applying Cufflinks and Cuffdiff to M1 and M2 RNA-seq data clearly revealed differential expression of individual CDS (Figure 6c). While PDLIM7 v1 was mainly expressed by M1-like macrophages, M2-like macrophages mainly expressed PDLIM7 v2, while no difference was observed for PDLIM7 v4. We validated the usage of these variants by version-specific qPCR (Figure 6d). Taken together, these new findings might open new avenues towards the role of alternative splicing in macrophages potentially linking alternative transcript usage with macrophage polarization.
Example 8: New markers for M1- and M2-like macrophages identified by combined transcriptome analysis. In light of the still limited number of cell surface markers clearly distinguishing human M1- from M2-like macrophages, we interrogated the genes of the human surfaceome (da Cunha J.P. et al., Proceedings of the National Academy of Sciences of the United States of America 106(39):16752-16757 (2009)) for differential expression between M1- and M2-like macrophages. By this approach 475 surface molecules were found to be differentially expressed (Figure 7a). As visualized in Figure 7b, the cell surface molecules CD120b, TLR2, and SLAMF7 showed preferential expression in M1-like macrophages, which was true irrespective of differentiation of macrophages by GM-CSF or M-CSF (Figure 14a). Several surface molecules, including CD1a, CD1b, CD93 and CD226 were significantly increased in expression in M2-like macrophages (Figures 7c and 14b). Taken together, screening higher-resolution transcriptome data established by RNA-seq allows for the identification of novel genes related to specific polarization programs in macrophages.

### Tables

| **Table 1 RNA-Seq** | | | | |
|---|---|---|---|---|
| | **M1** | | **M2** | |
| | reads (x10⁶) | percentage (%) | reads (x10⁶) | percentage (%) |
| Total | 15.0 ± 2.8 | | 20.4 ± 9.2 | |
| Exons | 11.8 ± 2.2 | 78.4 ± 1.1 | 16.1 ± 7.4 | 79.4 ± 2.0 |
| Exon-Intron | 0.4 ± 0.1 | 2.5 ± 0.1 | 0.5 ± 0.2 | 2.4 ± 0.2 |

| boundaries | | | | |
|---|---|---|---|---|
| Introns | 2.1 ± 0.5 | 14.1 ± 1.0 | 2.7 ± 1.3 | 13.2 ± 1.6 |
| Intergenic regions | 0.8 ± 0.1 | 5.0 ± 0.2 | 1.0 ± 0.5 | 5.0 ± 0.3 |

| **Table 2 qPCR oligonucleotides** | | |
|---|---|---|
| CD68 Forward | 5'-GTCCACCTCGACCTGCTCT-3' | (SEQ ID NO:15) |
| CD68 Reverse | 5'-CACTGGGGCAGGAGAAACT-3' | (SEQ ID NO:16) |
| CD64 Forward | 5'-TGGGAAAGCATCGCTACAC-3' | (SEQ ID NO:17) |
| CD64 Reverse | 5'-GCACTGGAGCTGGAAATAGC-3' | (SEQ ID NO:18) |
| CD23 Forward | 5'-GGGAGAATCCAAGCAGGAC-3' | (SEQ ID NO:19) |
| CD23 Reverse | 5'-GGAAGCTCCTCGATCTCTGA-3' | (SEQ ID NO:20) |
| LILRA1 Forward | 5'-TCGCTGTTTCTACGGTAGCC-3' | (SEQ ID NO:21) |
| LILRA1 Reverse | 5'-GGGTGGGTTTGATGTAGGC-3' | (SEQ ID NO:22) |
| LILRA2 Forward | 5'-CAGCCACAATCACTCATCAGA-3' | (SEQ ID NO:23) |
| LILRA2 Reverse | 5'-AGGGTGGGTTTGCTGTAGG-3' | (SEQ ID NO:24) |
| LILRA3 Forward | 5'-GGAGCTCGTGGTCTCAGG-3' | (SEQ ID NO:25) |
| LILRA3 Reverse | 5'-CTTGGAGTCGGACTTGTTTTG-3' | (SEQ ID NO:26) |
| LILRA5 Forward | 5'-GCACCATGGTCTCATCCAT-3' | (SEQ ID NO:27) |
| LILRA5 Reverse | 5'-GTGGCTTTGGAGAGGTTCC-3' | (SEQ ID NO:28) |
| LILRB1 Forward | 5'-GGAGCTCGTGGTCTCAGG-3' | (SEQ ID NO:29) |
| LILRB1 Reverse | 5'-GGGTTTGATGTAGGCTCCTG-3' | (SEQ ID NO:30) |
| LILRB3 Forward | 5'-GGAGATACCGCTGCCACTAT-3' | (SEQ ID NO:31) |
| LILRB3 Reverse | 5'-GGTGGGTTTGCTGTAGGC-3' | (SEQ ID NO:32) |
| APOL1 Forward | 5'-TTCGAATTCCTCGGTATATCTTG-3' | (SEQ ID NO:33) |
| APOL1 Reverse | 5'-CACCTCCAGTTATGCGTCTG-3' | (SEQ ID NO:34) |
| APOL2 Forward | 5'-ATGATGAAGCCTGGAATGGA-3' | (SEQ ID NO:35) |
| APOL2 Reverse | 5'-TCAGAGCTTTACGGAGCTCAT-3' | (SEQ ID NO:36) |
| APOL3 Forward | 5'-GCCTGGAAGAGATTCGTGAC-3' | (SEQ ID NO:37) |
| APOL3 Reverse | 5'-CTTCAGAGCTTCGTAGAGAGCA-3' | (SEQ ID NO:38) |
| APOL6 Forward | 5'-AAGTGAGGCTGGTGTTGGTT-3' | (SEQ ID NO:39) |
| APOL6 Reverse | 5'-CGTCTTGTAGCTCCACGTCTT-3' | (SEQ ID NO:40) |
| GAPDH Forward | 5'-AGCCACATCGCTCAGACAC-3' | (SEQ ID NO:41) |
| GAPDH Reverse | 5'-GCCCAATACGACCAAATCC-3' | (SEQ ID NO:42) |

| **Table 3 Isoform specific qPCR oligonucleotides** | | |
|---|---|---|
| PDLIM7 v1 Forward | 5'-CCGCAGCAGAATGGACAG-3' | (SEQ ID NO:43) |
| PDLIM7 v1 Reverse | 5'-GCTCCTGGGGTGTAGATG-3' | (SEQ ID NO:44) |
| PDLIM7 v2 Forward | 5'-ACCGCAGAAGGTGCAGAC-3' | (SEQ ID NO:45) |
| PDLIM7 v2 Reverse | 5'-CTGGCTTGATTTCTTCAGGTG-3' | (SEQ ID NO:46) |
| PDLIM7 v4 Forward | 5'-CCGCAGCAGAATGGACAG-3' | (SEQ ID NO:47) |
| PDLIM7 v4 Reverse | 5'-GCAGGAGGAACAGAAAGAG-3' | (SEQ ID NO:48) |
| GAPDH Forward | 5'-AGCCACATCGCTCAGACAC-3' | (SEQ ID NO:49) |
| GAPDH Reverse | 5'-GCCCAATACGACCAAATCC-3' | (SEQ ID NO:50) |

| **Table 4 Alternative promoter usage** | |
|---|---|
| C8orf83 | chr8:93895757-93978372 |
| EIF4ENIF1 | chr22:31835344-31885874 |
| HRH1 | chr3:11178778-11304939 |
| JDP2 | chr14:75894508-75939404 |
| NCS1 | chr9:132934856-132999583 |
| PDE2A | chr11:72287184-72385494 |
| PUS7L | chr12:44122409-44152596 |
| SMARCD3 | chr7: 150936058-150974231 |
| TSC22D1 | chr13:45007654-45154568 |

| **Table 5 Alternative TSS usage** | |
|---|---|
| WFS1 | chr4:6271576-6304992 |
| ARNT | chr1:150768686-150849186 |
| ASRGL1 | chr11:62104773-62160887 |
| OSBPL9 | chr1:52082546-52344609 |
| GBA | chr1:155204238-155214653 |
| HES6 | chr2:239146907-239148681 |
| BAT5 | chr6:31654725-31671137 |
| DCTN1 | chr2:74588280-74621008 |
| RASGRP4 | chr19:38893774-38916945 |
| SNX16 | chr8:82711817-82754521 |
| NDUFC1 | chr4:140211070-140311935 |
| CCDC17 | chr1:46085715-46089731 |
| CD200R1 | chr3:112641531-112693937 |
| FECH | chr18:55212072-55253969 |
| NRGN | chr11:124609828-124617102 |
| RB1CC1 | chr8:53535017-53627026 |
| UBQLN1 | chr9:86274877-86323168 |
| MTERFD3 | chr12:107371068-107380929 |
| MBOAT7 | chr19:54677105-54693733 |
| RANBP3 | chr19:5914192-5978320 |
| RAP1GDS1 | chr4:99182526-99365012 |
| TNNT1 | chr19:55644160-55660606 |
| ABCC1 | chr16:16043433-16236931 |
| CDCA7L | chr7:21582832-21985542 |
| HYI | chr1:43888796-43919660 |
| C8orf83 | chr8:93895757-93978372 |
| CD36 | chr7:80231503-80308593 |
| NT5C3 | chr7:33053741-33102409 |

| **Table 6 Alternative CDS usage** | |
|---|---|
| ABCC1 | chr16:16043433-16236931 |
| CCDC17 | chr1:46085715-46089731 |
| CD200R1 | chr3:112641531-112693937 |
| CDCA7L | chr7:21582832-21985542 |
| FECH | chr18:55212072-55253969 |
| HES6 | chr2:239146907-239148681 |
| HYI | chr1:43888796-43919660 |
| JDP2 | chr14:75894508-75939404 |
| MYO1B | chr2:192110106-192290115 |
| NCS1 | chr9:132934856-132999583 |
| PDLIM7 | chr5:176910394-176924602 |
| RANBP3 | chr19:5914192-5978320 |
| RAP1GDS1 | chr4:99182526-99365012 |
| RASGRP4 | chr19:38893774-38916945 |
| RB1CC1 | chr8:53535017-53627026 |
| RP6-213H19.1 | chrX:131157244-131209971 |
| SLC25A45 | chr11:65142662-65150142 |
| SNX16 | chr8:82711817-82754521 |
| TNNT1 | chr19:55644160-55660606 |
| UBQLN1 | chr9:86274877-86323168 |

**Sequence Listing, Free Text**

| SEQ ID NO: | Description |
|---|---|
| 1/2 | tumor necrosis factor receptor superfamily, member 1B (CD120b) |
| 3/4 | toll-like receptor 2 (TLR2) |
| 5/6 | SLAM family member 7 (SLAMF7) |
| 7/8 | CD1a |
| 9/10 | CD1b |
| 11/12 | CD93 |
| 14/15 | CD226 |
| 16-50 | primer |

## Claims

1. An in vitro method for identifying of, distinguishing between and isolating of M1-like and M2-like macrophages which comprises characterizing the macrophages based on the expression level of one or more of the specific M1-associated cell surface markers CD120b having the nucleic acid sequence of SEQ ID NO:1, TLR2 having the nucleic acid sequence of SEQ ID NO:3 and SLAMF7 having the nucleic acid sequence of SEQ ID NO:5, or of one or more of the specific M2-associated cell surface markers CD1a having the nucleic acid sequence of SEQ ID NO:7, CD1b having the nucleic acid sequence of SEQ ID NO:9, CD93 having the nucleic acid sequence of SEQ ID NO:11 and CD226 having the nucleic acid sequence of SEQ ID NO:13, respectively, wherein the expression level of the M1-associated cell surface markers is at least 30 % higher in M1-like macrophages than in M2-like macrophages and the expression level of the M2-associated cell surface markers is at least 30 % higher in M2-like macrophages than in M1-like macrophages.

2. The method of claim 1, wherein the expression level of the cell surface marker in the respective M1-like or M2-like macrophage is at least 50 % and preferably at least 70 % higher than in the other macrophage type.

3. The method of claim 1 or 2, wherein the identifying of and distinguishing between the M1-like and M2-like macrophages is performed by an amplification or by a targeted resequencing of one or more of the specific M1-associated cell surface marker nucleic acids CD120b, TLR2 and SLAMF7 (SEQ ID NOs: 1, 3 and 5), or of one or more of the specific M2-associated cell surface marker nucleic acids CD1a, CD1b, CD93 and CD226 (SEQ ID NOs: 7, 9, 11 and 13), respectively, of the macrophage, and a subsequent detection of the amplification/resequencing product.

4. The method of claim 3, wherein the amplification/resequencing employs one or more primers derived from each of the marker nucleic acids, preferably said primers have at least 12, preferably at least 15, most preferably at least 19 contiguous nucleotides of the respective marker nucleic acid sequence.

5. The method of claim 1 or 2, wherein the identifying of and distinguishing between the M1-like and M2-like macrophages comprises hybridizing one or more probes selective for one of the specific M1-associated cell surface marker nucleic acids CD120b, TLR2 and SLAMF7 (SEQ ID NOs: 1, 3 and 5), or for one of the specific M2-associated cell surface marker nucleic acids CD1a, CD1b, CD93 and CD226 (SEQ ID NOs: 7, 9, 11 and 13), respectively, of the macrophage.

6. The method of claim 5, which is performed on a hybridization array.

7. The method of claim 1 or 2, wherein the identifying of, distinguishing between and isolating of the M1-like and M2-like macrophages comprises contacting the macrophages with one or more binding molecules directed against the specific M1-associated cell surface marker proteins CD120b, TLR2 and SLAMF7 (SEQ ID NOs: 2, 4 and 6), or with one or more binding molecules directed against the specific M2-associated cell surface marker proteins CD1a, CD1b, CD93 and CD226 (SEQ ID NOs: 8, 10, 12 and 14), respectively.

8. The method of claim 7, wherein the binding molecules
(i) are antibodies, preferably monoclonal antibodies; and/or
(ii) are labeled with marker molecules, preferably fluorescence markers.

9. The method of claim 8, wherein the binding molecules are selected from FITC-labeled CD1b, CD93, CD226 and anti-TLR2; PE-labeled CD120b and anti-SLAMF7; PE-Cy5-labeled CD1a monoclonal antibodies.

10. The method according to one or more of claims 7 to 9, which is performed on a FACS sorter.

11. Use of a kit for performing the method according to any one of claims 1 to 10, which kit comprises at least one reagent for identifying of, distinguishing between and isolating of M1-like macrophages and at least one reagent for identifying of, distinguishing between and isolating of M2-like macrophages, said reagents being selected from
(i) one or more primers derived from the marker nucleic acids as defined in claim 4,
(ii) one or more probes selective for the cell surface marker nucleic acids as defined in claim 5,
(iii) a hybridization array as defined in claim 6, or
(iv) one or more binding molecule as defined in claims 7 to 9.

12. Use of antibodies selected from antibodies directed against CD120b having the protein sequence of SEQ ID NO:2, antibodies directed against TLR2 having the protein sequence of SEQ ID NO:4 and antibodies directed against SLAMF7 having the protein sequence of SEQ ID NO:6 for performing the method according to any one of claims 1 to 10, wherein the antibodies are for identifying, characterizing and isolating M1-like macrophages.

13. Use of antibodies selected from antibodies directed against CD1a having the protein sequence of SEQ ID NO:8, antibodies directed against CD1b having the protein sequence of SEQ ID NO:10, antibodies directed against CD93 having the protein sequence of SEQ ID NO: 12 and antibodies directed against CD226 having the protein sequence of SEQ ID NO:14 for performing the method according to any one of claims 1 to 10, wherein the antibodies are for identifying, characterizing and isolating M2-like macrophages.

## Patentansprüche

1. In-vitro-Verfahren zum Identifizieren von, Unterscheiden zwischen und Isolieren von M1-artigen und M2-artigen Makrophagen, umfassend das Charakterisieren der Makrophagen anhand des Expressionsniveaus von einem oder mehreren der spezifischen M1-assoziierten Zelloberflächenmarker CD120b mit der Nucleinsäuresequenz von SEQ ID Nr. 1, TLR2 mit der Nucleinsäuresequenz von SEQ ID Nr. 3 und SLAMF7 mit der Nucleinsäuresequenz von SEQ ID Nr. 5 oder von einem oder mehreren der spezifischen M2-assoziierten Zelloberflächenmarker CD1a mit der Nucleinsäuresequenz von SEQ ID Nr. 7, CD1b mit der Nucleinsäuresequenz von SEQ ID Nr. 9, CD93 mit der Nucleinsäuresequenz von SEQ ID Nr. 11 und CD226 mit der Nucleinsäuresequenz von SEQ ID Nr. 13, wobei das Expressionsniveau der M1-assoziierten Zelloberflächenmarker in M1-artigen Makrophagen um wenigstens 30% höher ist als in M2-artigen Makrophagen und das Expressionsniveau der M2-assoziierten Zelloberflächenmarker in M2-artigen Makrophagen um wenigstens 30% höher ist als in M1-artigen Makrophagen.

2. Verfahren gemäß Anspruch 1, wobei das Expressionsniveau der Zelloberflächenmarker in dem jeweiligen M1-artigen oder M2-artigen Makrophagen um wenigstens 50% und vorzugsweise um wenigstens 70% höher ist als in dem jeweils anderen Makrophagentyp.

3. Verfahren gemäß Anspruch 1 oder 2, wobei das Identifizieren von und Unterscheiden zwischen den M1-artigen und M2-artigen Makrophagen durch eine Amplifikation oder durch eine gezielte Resequenzierung von einem oder mehreren der spezifischen M1-assoziierten Zelloberflächenmarker-Nucleinsäuren CD120b, TLR2 und SLAMF7 (SEQ ID Nr. 1, 3 bzw. 5), von einem oder mehreren der spezifischen M2-assoziierten Zelloberflächenmarker-Nucleinsäuren CD1a, CD1b, CD93 und CD226 (SEQ ID Nr. 7, 9, 11 bzw. 13) des Makrophagen und einen anschließenden Nachweis des Amplifikations-/Resequenzierungsprodukts durchgeführt wird.

4. Verfahren gemäß Anspruch 3, wobei bei der Amplifikation/Resequenzierung ein oder mehrere Primer eingesetzt werden, die jeweils von den Markernucleinsäuren abgeleitet sind, wobei vorzugsweise die Primer wenigstens 12, vorzugsweise wenigstens 15, am meisten bevorzugt wenigstens 19 aufeinanderfolgende Nucleotide der jeweiligen Markernucleinsäuresequenz aufweisen.

5. Verfahren gemäß Anspruch 1 oder 2, wobei das Identifizieren von und Unterscheiden zwischen den M1-artigen und M2-artigen Makrophagen das Hybridisieren von einer oder mehreren Sonden, die selektiv bezüglich einer der spezifischen M1-assoziierten Zelloberflächenmarker-Nucleinsäuren CD120b, TLR2 und SLAMF7 (SEQ ID Nr. 1, 3 bzw. 5) oder bezüglich einer der spezifischen M2-assoziierten Zelloberflächenmarker-Nucleinsäuren CD1a, CD1b, CD93 und CD226 (SEQ ID Nr. 7, 9, 11 bzw. 13) des Makrophagen sind, umfasst.

6. Verfahren gemäß Anspruch 5, das auf einem Hybridisierungsarray durchgeführt wird.

7. Verfahren gemäß Anspruch 1 oder 2, wobei das Identifizieren von, Unterscheiden zwischen und Isolieren der M1-artigen und M2-artigen Makrophagen das In-Kontakt-Bringen der Makrophagen mit einem oder mehreren Bindungsmolekülen, die gegen die spezifischen M1-assoziierten Zelloberflächenmarkerproteine CD120b, TLR2 und SLAMF7 (SEQ ID Nr. 2, 4 bzw. 6) gerichtet sind, oder mit einem oder mehreren Bindungsmolekülen, die gegen die spezifischen M2-assoziierten Zelloberflächenmarkerproteine CD1a, CD1b, CD93 und CD226 (SEQ ID Nr. 8, 10, 12 bzw. 14) gerichtet sind, umfasst.

8. Verfahren gemäß Anspruch 7, wobei die Bindungsmoleküle
(i) Antikörper, vorzugsweise monoklonale Antikörper, sind; und/oder
(ii) mit Markermolekülen, vorzugsweise Fluoreszenzmarkern, markiert sind.

9. Verfahren gemäß Anspruch 8, wobei die Bindungsmoleküle aus monoklonalen FITC-markierten CD1b-, CD93-, CD226- und Anti-TLR2-, PEmarkierten CD120b- und Anti-SLAMF7-, PE-Cy5-markierten CD1a-Antikörpern ausgewählt sind.

10. Verfahren gemäß einem oder mehreren der Ansprüche 7 bis 9, das auf einem FACS-Sortierer durchgeführt wird.

11. Verwendung eines Kits zur Durchführung des Verfahrens gemäß einem der Ansprüche 1 bis 10, wobei der Kit wenigstens ein Reagens zum Identifizieren von, Unterscheiden zwischen und Isolieren von M1-artigen Makrophagen und wenigstens ein Reagens zum Identifizieren von, Unterscheiden zwischen und Isolieren von M2-artigen Makrophagen umfasst, wobei die Reagentien ausgewählt sind aus
(i) einem oder mehreren Primern, die von den in Anspruch 4 definierten Markernucleinsäuren abgeleitet sind,
(ii) einer oder mehreren Sonden, die selektiv bezüglich der Zelloberflächenmarker-Nucleinsäuren sind, wie sie in Anspruch 5 definiert sind;
(iii) einem Hybridisierungsarray, wie er in Anspruch 6 definiert ist; oder
(iv) einem oder mehreren Bindungsmolekülen, wie sie in den Ansprüchen 7 bis 9 definiert sind.

12. Verwendung von Antikörpern, die aus gegen CD120b mit der Proteinsequenz von SEQ ID Nr. 2 gerichteten Antikörpern, gegen TLR2 mit der Proteinsequenz von SEQ ID Nr. 4 gerichteten Antikörpern und gegen SLAMF7 mit der Proteinsequenz von SEQ ID Nr. 6 gerichteten Antikörpern ausgewählt sind, zur Durchführung des Verfahrens gemäß einem der Ansprüche 1 bis 10, wobei die Antikörper zum Identifizieren, Charakterisieren und Isolieren von M1-artigen Makrophagen dienen.

13. Verwendung von Antikörpern, die aus gegen CD1a mit der Proteinsequenz von SEQ ID Nr. 8 gerichteten Antikörpern, gegen CD1b mit der Proteinsequenz von SEQ ID Nr. 10 gerichteten Antikörpern, gegen CD93 mit der Proteinsequenz von SEQ ID Nr. 12 gerichteten Antikörpern und gegen CD226 mit der Proteinsequenz von SEQ ID Nr. 14 gerichteten Antikörpern ausgewählt sind, zur Durchführung des Verfahrens gemäß einem der Ansprüche 1 bis 10, wobei die Antikörper zum Identifizieren, Charakterisieren und Isolieren von M2-artigen Makrophagen dienen.

## Revendications

1. Procédé in vitro pour identifier, faire la distinction entre, et isoler les macrophages de type M1 et de type M2, qui comprend la caractérisation des macrophages sur la base du niveau d'expression d'un ou de plusieurs marqueurs de surface cellulaire associés à M1 spécifiques CD120b ayant la séquence d'acide nucléique de SEQ ID NO:1, TLR2 ayant la séquence d'acide nucléique de SEQ ID NO:3 et SLAMF7 ayant la séquence d'acide nucléique de SEQ ID NO:5, ou d'un ou de plusieurs marqueurs de surface cellulaire associés à M2 spécifiques CD1a ayant la séquence d'acide nucléique de SEQ ID NO:7, CD1b ayant la séquence d'acide nucléique de SEQ ID NO:9, CD93 ayant la séquence d'acide nucléique de SEQ ID NO:11 et CD226 ayant la séquence d'acide nucléique de SEQ ID NO:13, respectivement, dans lequel le niveau d'expression des marqueurs de surface cellulaire associés à M1 est d'au moins 30% plus élevé dans les macrophages de type M1 que dans les macrophages de type M2 et le niveau d'expression des marqueurs de surface cellulaire associés à M2 est d'au moins 30% plus élevé dans les macrophages de type M2 que dans les macrophages de type M1.

2. Procédé selon la revendication 1, dans lequel le niveau d'expression du marqueur de surface cellulaire dans les macrophages de type M1 ou de type M2 respectifs est d'au moins 50% et, de préférence, d'au moins 70% plus élevé que dans l'autre type de macrophage.

3. Procédé selon la revendication 1 ou 2, dans lequel l'identification des macrophages et la distinction entre les macrophages de type M1 et ceux de type M2 sont réalisées à l'aide d'une amplification ou d'un reséquençage ciblé d'un ou de plusieurs des acides nucléiques marqueurs de surface cellulaire associés à M1 spécifiques CD120B, TLR2 et SLAMF7 (SEQ ID NO: 1, 3 et 5), ou d'un ou de plusieurs des acides nucléiques marqueurs de surface cellulaire associés à M2 spécifiques CD1a, CD1b, CD93 et CD226 (SEQ ID NO: 7, 9, 11 et 13), respectivement, du macrophage, et d'une détection subséquente du produit d'amplification/de reséquençage.

4. Procédé selon la revendication 3, dans lequel l'amplification/le reséquençage met en oeuvre une ou plusieurs amorces dérivées de chacun des acides nucléiques marqueurs, de préférence, lesdites amorces ont au moins 12, de préférence, au moins 15, le plus préférablement, au moins 19 nucléotides contigus de la séquence d'acide nucléique marqueur respective.

5. Procédé selon la revendication 1 ou 2, dans lequel l'identification des macrophages et la distinction entre les macrophages de type M1 et ceux de type M2 comprennent l'hybridation d'une ou de plusieurs sondes sélectives pour l'un des acides nucléiques marqueurs de surface cellulaire associés à M1 spécifiques CD120B, TLR2 et SLAMF7 (SEQ ID NO: 1, 3 et 5), ou pour l'un des acides nucléiques marqueurs de surface cellulaire associés à M2 spécifiques CD la, CD1b, CD93 et CD226 (SEQ ID NO: 7, 9, 11 et 13), respectivement, du macrophage.

6. Procédé selon la revendication 5, qui est réalisé sur une puce d'hybridation.

7. Procédé selon la revendication 1 ou 2, dans lequel l'identification des macrophages, la distinction entre les macrophages de type M1 et ceux de type M2 et leur isolement comprennent la mise en contact des macrophages avec une ou plusieurs molécules de liaison dirigées contre les protéines marqueurs de surface cellulaire associées à M1 spécifiques CD120B, TLR2 et SLAMF7 (SEQ ID NO: 2, 4 et 6), ou avec une ou plusieurs molécules de liaison dirigées contre les protéines marqueurs de surface cellulaire associées à M2 spécifiques CD1a, CD1b, CD93 et CD226 (SEQ ID NO: 8, 10, 12 et 14), respectivement.

8. Procédé selon la revendication 7, dans lequel les molécules de liaison
(i) sont des anticorps, de préférence des anticorps monoclonaux ; et/ou
(ii) sont marquées à l'aide de molécules marqueurs, de préférence, des marqueurs de fluorescence.

9. Procédé selon la revendication 8, dans lequel les molécules de liaison sont choisies parmi les CD1b, CD93, CD226 et anti-TLR2 marqués au FITC ; les CD120b et anti-SLAMF7 marqués au PE ; les anticorps monoclonaux CD1a marqués au PE-Cy5.

10. Procédé selon une ou plusieurs des revendications 7 à 9, qui est réalisé sur un trieur FACS.

11. Utilisation d'une trousse permettant de réaliser le procédé selon l'une quelconque des revendications 1 à 10, laquelle trousse comprend au moins un réactif pour l'identification, la distinction entre les macrophages et l'isolement des macrophages de type M1 et au moins un réactif pour l'identification, la distinction entre les macrophages et l'isolement des macrophages de type M2, lesdits réactifs étant choisis parmi
(i) une ou plusieurs amorces dérivées des acides nucléiques marqueurs tels que définis dans la revendication 4,
(ii) une ou plusieurs sondes sélectives pour les acides nucléiques marqueurs de surface cellulaire tels que définis dans la revendication 5,
(iii) une puce d'hybridation telle que définie dans la revendication 6, ou
(iv) une ou plusieurs molécules de liaison telles que définies dans les revendications 7 à 9.

12. Utilisation d'anticorps choisis parmi les anticorps dirigés contre CD120b ayant la séquence protéique de SEQ ID NO:2, les anticorps dirigés contre TLR2 ayant la séquence protéique de SEQ ID NO:4 et les anticorps dirigés contre SLAMF7 ayant la séquence protéique de SEQ ID NO:6 pour réaliser le procédé selon l'une quelconque des revendications 1 à 10, où les anticorps sont destinés à l'identification, la caractérisation et l'isolement des macrophages de type M1.

13. Utilisation d'anticorps choisis parmi les anticorps dirigés contre CD1a ayant la séquence protéique de SEQ ID NO:8, les anticorps dirigés contre CD1b ayant la séquence protéique de SEQ ID NO:10, les anticorps dirigés contre CD93 ayant la séquence protéique de SEQ ID NO:12 et les anticorps dirigés contre CD226 ayant la séquence protéique de SEQ ID NO:14 pour réaliser le procédé selon l'une quelconque des revendications 1 à 10, où les anticorps sont destinés à l'identification, la caractérisation et l'isolement des macrophages de type M2.


### SEQUENCE LISTING

<110> Rheinische Friedrich-Wilhelms Universitaet Bonn Becton Dickinson and Company
<120> High-resolution Transcriptome of Human Macrophages
<130> 130369wo
<150> EP 12159479.0
   <151> 2012-03-14
<160> 50
<170> PatentIn version 3.3
<210> 1
   <211> 3682
   <212> DNA
   <213> Homo sapiens
<400> 1
   gcgagcgcag cggagcctgg agagaaggcg ctgggctgcg agggcgcgag ggcgcgaggg      60
   cagggggcaa ccggaccccg cccgcaccca tggcgcccgt cgccgtctgg gccgcgctgg     120
   ccgtcggact ggagctctgg gctgcggcgc acgccttgcc cgcccaggtg gcatttacac     180
   cctacgcccc ggagcccggg agcacatgcc ggctcagaga atactatgac cagacagctc     240
   agatgtgctg cagcaaatgc tcgccgggcc aacatgcaaa agtcttctgt accaagacct     300
   cggacaccgt gtgtgactcc tgtgaggaca gcacatacac ccagctctgg aactgggttc     360
   ccgagtgctt gagctgtggc tcccgctgta gctctgacca ggtggaaact caagcctgca     420
   ctcgggaaca gaaccgcatc tgcacctgca ggcccggctg gtactgcgcg ctgagcaagc     480
   aggaggggtg ccggctgtgc gcgccgctgc gcaagtgccg cccgggcttc ggcgtggcca     540
   gaccaggaac tgaaacatca gacgtggtgt gcaagccctg tgccccgggg acgttctcca     600
   acacgacttc atccacggat atttgcaggc cccaccagat ctgtaacgtg gtggccatcc     660
   ctgggaatgc aagcatggat gcagtctgca cgtccacgtc ccccacccgg agtatggccc     720
   caggggcagt acacttaccc cagccagtgt ccacacgatc ccaacacacg cagccaactc     780
   cagaacccag cactgctcca agcacctcct tcctgctccc aatgggcccc agccccccag     840
   ctgaagggag cactggcgac ttcgctcttc cagttggact gattgtgggt gtgacagcct     900
   tgggtctact aataatagga gtggtgaact gtgtcatcat gacccaggtg aaaaagaagc     960
   ccttgtgcct gcagagagaa gccaaggtgc ctcacttgcc tgccgataag gcccggggta    1020
   cacagggccc cgagcagcag cacctgctga tcacagcgcc gagctccagc agcagctccc    1080
   tggagagctc ggccagtgcg ttggacagaa gggcgcccac tcggaaccag ccacaggcac    1140
   caggcgtgga ggccagtggg gccggggagg cccgggccag caccgggagc tcagattctt    1200
   cccctggtgg ccatgggacc caggtcaatg tcacctgcat cgtgaacgtc tgtagcagct    1260
   ctgaccacag ctcacagtgc tcctcccaag ccagctccac aatgggagac acagattcca 1320
   gcccctcgga gtccccgaag gacgagcagg tccccttctc caaggaggaa tgtgcctttc 1380
   ggtcacagct ggagacgcca gagaccctgc tggggagcac cgaagagaag cccctgcccc 1440
   ttggagtgcc tgatgctggg atgaagccca gttaaccagg ccggtgtggg ctgtgtcgta 1500
   gccaaggtgg gctgagccct ggcaggatga ccctgcgaag gggccctggt ccttccaggc 1560
   ccccaccact aggactctga ggctctttct gggccaagtt cctctagtgc cctccacagc 1620
   cgcagcctcc ctctgacctg caggccaaga gcagaggcag cgagttgtgg aaagcctctg 1680
   ctgccatggc gtgtccctct cggaaggctg gctgggcatg gacgttcggg gcatgctggg 1740
   gcaagtccct gactctctgt gacctgcccc gcccagctgc acctgccagc ctggcttctg 1800
   gagcccttgg gttttttgtt tgtttgtttg tttgtttgtt tgtttctccc cctgggctct 1860
   gccccagctc tggcttccag aaaaccccag catccttttc tgcagagggg ctttctggag 1920
   aggagggatg ctgcctgagt cacccatgaa gacaggacag tgcttcagcc tgaggctgag 1980
   actgcgggat ggtcctgggg ctctgtgcag ggaggaggtg gcagccctgt agggaacggg 2040
   gtccttcaag ttagctcagg aggcttggaa agcatcacct caggccaggt gcagtggctc 2100
   acgcctatga tcccagcact ttgggaggct gaggcgggtg gatcacctga ggttaggagt 2160
   tcgagaccag cctggccaac atggtaaaac cccatctcta ctaaaaatac agaaattagc 2220
   cgggcgtggt ggcgggcacc tatagtccca gctactcaga agcctgaggc tgggaaatcg 2280
   tttgaacccg ggaagcggag gttgcaggga gccgagatca cgccactgca ctccagcctg 2340
   ggcgacagag cgagagtctg tctcaaaaga aaaaaaaaag caccgcctcc aaatgccaac 2400
   ttgtcctttt gtaccatggt gtgaaagtca gatgcccaga gggcccaggc aggccaccat 2460
   attcagtgct gtggcctggg caagataacg cacttctaac tagaaatctg ccaatttttt 2520
   aaaaaagtaa gtaccactca ggccaacaag ccaacgacaa agccaaactc tgccagccac 2580
   atccaacccc ccacctgcca tttgcaccct ccgccttcac tccggtgtgc ctgcagcccc 2640
   gcgcctcctt ccttgctgtc ctaggccaca ccatctcctt tcagggaatt tcaggaacta 2700
   gagatgactg agtcctcgta gccatctctc tactcctacc tcagcctaga ccctcctcct 2760
   cccccagagg ggtgggttcc tcttccccac tccccacctt caattcctgg gccccaaacg 2820
   ggctgccctg ccactttggt acatggccag tgtgatccca agtgccagtc ttgtgtctgc 2880
   gtctgtgttg cgtgtcgtgg gtgtgtgtag ccaaggtcgg taagttgaat ggcctgcctt 2940
   gaagccactg aagctgggat tcctccccat tagagtcagc cttccccctc ccagggccag 3000
   ggccctgcag aggggaaacc agtgtagcct tgcccggatt ctgggaggaa gcaggttgag 3060
   gggctcctgg aaaggctcag tctcaggagc atggggataa aggagaaggc atgaaattgt 3120
   ctagcagagc aggggcaggg tgataaattg ttgataaatt ccactggact tgagcttggc   3180
   agctgaacta ttggagggtg ggagagccca gccattacca tggagacaag aagggttttc   3240
   caccctggaa tcaagatgtc agactggctg gctgcagtga cgtgcacctg tactcaggag   3300
   gctgagggga ggatcactgg agcccaggag tttgaggctg cagcgagcta tgatcgcgcc   3360
   actacactcc agcctgagca acagagtgag accctgtctc ttaaagaaaa aaaaagtcag   3420
   actgctggga ctggccaggt ttctgcccac attggaccca catgaggaca tgatggagcg   3480
   cacctgcccc ctggtggaca gtcctgggag aacctcaggc ttccttggca tcacagggca   3540
   gagccgggaa gcgatgaatt tggagactct gtggggcctt ggttcccttg tgtgtgtgtg   3600
   ttgatcccaa gacaatgaaa gtttgcactg tatgctggac ggcattcctg cttatcaata   3660
   aacctgtttg ttttaaaaaa aa                                            3682
<210> 2
   <211> 461
   <212> PRT
   <213> Homo sapiens
<400> 2
   Met Ala Pro Val Ala Val Trp Ala Ala Leu Ala Val Gly Leu Glu Leu
   1               5                   10                  15
   Trp Ala Ala Ala His Ala Leu Pro Ala Gln Val Ala Phe Thr Pro Tyr
               20                  25                  30
   Ala Pro Glu Pro Gly Ser Thr Cys Arg Leu Arg Glu Tyr Tyr Asp Gln
           35                  40                  45
   Thr Ala Gln Met Cys Cys Ser Lys Cys Ser Pro Gly Gln His Ala Lys
       50                  55                  60
   Val Phe Cys Thr Lys Thr Ser Asp Thr Val Cys Asp Ser Cys Glu Asp
   65                  70                  75                  80
   Ser Thr Tyr Thr Gln Leu Trp Asn Trp Val Pro Glu Cys Leu Ser Cys
                   85                  90                  95
   Gly Ser Arg Cys Ser Ser Asp Gln Val Glu Thr Gln Ala Cys Thr Arg
               100                 105                 110
   Glu Gln Asn Arg Ile Cys Thr Cys Arg Pro Gly Trp Tyr Cys Ala Leu
           115                 120                 125
   Ser Lys Gln Glu Gly Cys Arg Leu Cys Ala Pro Leu Arg Lys Cys Arg
       130                 135                 140
   Pro Gly Phe Gly Val Ala Arg Pro Gly Thr Glu Thr Ser Asp Val Val
   145                 150                 155                 160
   Cys Lys Pro Cys Ala Pro Gly Thr Phe Ser Asn Thr Thr Ser Ser Thr
                   165                 170                 175
   Asp Ile Cys Arg Pro His Gln Ile Cys Asn Val Val Ala Ile Pro Gly
               180                 185                 190
   Asn Ala Ser Met Asp Ala Val Cys Thr Ser Thr Ser Pro Thr Arg Ser
           195                 200                 205
   Met Ala Pro Gly Ala Val His Leu Pro Gln Pro Val Ser Thr Arg Ser
       210                 215                 220
   Gln His Thr Gln Pro Thr Pro Glu Pro Ser Thr Ala Pro Ser Thr Ser
   225                 230                 235                 240
   Phe Leu Leu Pro Met Gly Pro Ser Pro Pro Ala Glu Gly Ser Thr Gly
                   245                 250                 255
   Asp Phe Ala Leu Pro Val Gly Leu Ile Val Gly Val Thr Ala Leu Gly
               260                 265                 270
   Leu Leu Ile Ile Gly Val Val Asn Cys Val Ile Met Thr Gln Val Lys
           275                 280                 285
   Lys Lys Pro Leu Cys Leu Gln Arg Glu Ala Lys Val Pro His Leu Pro
       290                 295                 300
   Ala Asp Lys Ala Arg Gly Thr Gln Gly Pro Glu Gln Gln His Leu Leu
   305                 310                 315                 320
   Ile Thr Ala Pro Ser Ser Ser Ser Ser Ser Leu Glu Ser Ser Ala Ser
                   325                 330                 335
   Ala Leu Asp Arg Arg Ala Pro Thr Arg Asn Gln Pro Gln Ala Pro Gly
               340                 345                 350
   Val Glu Ala Ser Gly Ala Gly Glu Ala Arg Ala Ser Thr Gly Ser Ser
           355                 360                 365
   Asp Ser Ser Pro Gly Gly His Gly Thr Gln Val Asn Val Thr Cys Ile
       370                 375                 380
   Val Asn Val Cys Ser Ser Ser Asp His Ser Ser Gln Cys Ser Ser Gln
   385                 390                 395                 400
   Ala Ser Ser Thr Met Gly Asp Thr Asp Ser Ser Pro Ser Glu Ser Pro
                   405                 410                 415
   Lys Asp Glu Gln Val Pro Phe Ser Lys Glu Glu Cys Ala Phe Arg Ser
               420                 425                 430
   Gln Leu Glu Thr Pro Glu Thr Leu Leu Gly Ser Thr Glu Glu Lys Pro
           435                 440                 445
   Leu Pro Leu Gly Val Pro Asp Ala Gly Met Lys Pro Ser
       450                 455                 460
<210> 3
   <211> 3417
   <212> DNA
   <213> Homo sapiens
<400> 3
   cggaggcagc gagaaagcgc agccaggcgg ctgctcggcg ttctctcagg tgactgctcg      60
   gagttctccc agtgtttggt gttgcaagca ggatccaaag gagacctata gtgactccca     120
   ggagctctta gtgaccaagt gaaggtacct gtggggctca ttgtgcccat tgctctttca     180
   ctgctttcaa ctggtagttg tgggttgaag cactggacaa tgccacatac tttgtggatg     240
   gtgtgggtct tgggggtcat catcagcctc tccaaggaag aatcctccaa tcaggcttct     300
   ctgtcttgtg accgcaatgg tatctgcaag ggcagctcag gatctttaaa ctccattccc     360
   tcagggctca cagaagctgt aaaaagcctt gacctgtcca acaacaggat cacctacatt     420
   agcaacagtg acctacagag gtgtgtgaac ctccaggctc tggtgctgac atccaatgga     480
   attaacacaa tagaggaaga ttctttttct tccctgggca gtcttgaaca tttagactta     540
   tcctataatt acttatctaa tttatcgtct tcctggttca agcccctttc ttctttaaca     600
   ttcttaaact tactgggaaa tccttacaaa accctagggg aaacatctct tttttctcat     660
   ctcacaaaat tgcaaatcct gagagtggga aatatggaca ccttcactaa gattcaaaga     720
   aaagattttg ctggacttac cttccttgag gaacttgaga ttgatgcttc agatctacag     780
   agctatgagc caaaaagttt gaagtcaatt cagaatgtaa gtcatctgat ccttcatatg     840
   aagcagcata ttttactgct ggagattttt gtagatgtta caagttccgt ggaatgtttg     900
   gaactgcgag atactgattt ggacactttc catttttcag aactatccac tggtgaaaca     960
   aattcattga ttaaaaagtt tacatttaga aatgtgaaaa tcaccgatga aagtttgttt    1020
   caggttatga aacttttgaa tcagatttct ggattgttag aattagagtt tgatgactgt    1080
   acccttaatg gagttggtaa ttttagagca tctgataatg acagagttat agatccaggt    1140
   aaagtggaaa cgttaacaat ccggaggctg catattccaa ggttttactt attttatgat    1200
   ctgagcactt tatattcact tacagaaaga gttaaaagaa tcacagtaga aaacagtaaa 1260
   gtttttctgg ttccttgttt actttcacaa catttaaaat cattagaata cttggatctc 1320
   agtgaaaatt tgatggttga agaatacttg aaaaattcag cctgtgagga tgcctggccc 1380
   tctctacaaa ctttaatttt aaggcaaaat catttggcat cattggaaaa aaccggagag 1440
   actttgctca ctctgaaaaa cttgactaac attgatatca gtaagaatag ttttcattct 1500
   atgcctgaaa cttgtcagtg gccagaaaag atgaaatatt tgaacttatc cagcacacga 1560
   atacacagtg taacaggctg cattcccaag acactggaaa ttttagatgt tagcaacaac 1620
   aatctcaatt tattttcttt gaatttgccg caactcaaag aactttatat ttccagaaat 1680
   aagttgatga ctctaccaga tgcctccctc ttacccatgt tactagtatt gaaaatcagt 1740
   aggaatgcaa taactacgtt ttctaaggag caacttgact catttcacac actgaagact 1800
   ttggaagctg gtggcaataa cttcatttgc tcctgtgaat tcctctcctt cactcaggag 1860
   cagcaagcac tggccaaagt cttgattgat tggccagcaa attacctgtg tgactctcca 1920
   tcccatgtgc gtggccagca ggttcaggat gtccgcctct cggtgtcgga atgtcacagg 1980
   acagcactgg tgtctggcat gtgctgtgct ctgttcctgc tgatcctgct cacgggggtc 2040
   ctgtgccacc gtttccatgg cctgtggtat atgaaaatga tgtgggcctg gctccaggcc 2100
   aaaaggaagc ccaggaaagc tcccagcagg aacatctgct atgatgcatt tgtttcttac 2160
   agtgagcggg atgcctactg ggtggagaac cttatggtcc aggagctgga gaacttcaat 2220
   ccccccttca agttgtgtct tcataagcgg gacttcattc ctggcaagtg gatcattgac 2280
   aatatcattg actccattga aaagagccac aaaactgtct ttgtgctttc tgaaaacttt 2340
   gtgaagagtg agtggtgcaa gtatgaactg gacttctccc atttccgtct ttttgatgag 2400
   aacaatgatg ctgccattct cattcttctg gagcccattg agaaaaaagc cattccccag 2460
   cgcttctgca agctgcggaa gataatgaac accaagacct acctggagtg gcccatggac 2520
   gaggctcagc gggaaggatt ttgggtaaat ctgagagctg cgataaagtc ctaggttccc 2580
   atatttaaga ccagtctttg tctagttggg atctttatgt cactagttat agttaagttc 2640
   attcagacat aattatataa aaactacgtg gatgtaccgt catttgagga cttgcttact 2700
   aaaactacaa aacttcaaat tttgtctggg gtgctgtttt ataaacatat gccagattta 2760
   aaaattggtt tttggttttt cttttttcta tgagataacc atgatcataa gtctattact 2820
   gatatctgaa tatagtccct tggtatccaa gggaattggt tgcaggatcc tcgtggatat 2880
   caaaattcat agatgatcaa gtcccttata agagtggcat agtatttgca tataacctgt 2940
   gtacattctc ctgtatactt taaatcatct ctagattact tatgataccc aatacaatgt 3000
   aaatactatg taaatagttg tactgtcttt ttatttatat tattattgtt attttttatt 3060
   ttcaaaattt ttaaaacata cttttgatcc acagttggtt gacttcatgg atgcagaacc 3120
   catggatata gagggccaac tgtaatctgt agcaactggc ttagttcatt aggaaacagc 3180
   acaaatgaac ttaagattct caatgactgt gtcattcttt cttcctgcta agagactcct 3240
   ctgtggccac aaaaggcatt ctctgtccta cctagctgtc acttctctgt gcagctgatc 3300
   tcaagagcaa caaggcaaag tatttggggc actccccaaa acttgttgct attcctagaa 3360
   aaaagtgctg tgtatttcct attaaacttt acaggatgag aaaaaaaaaa aaaaaaa 3417
<210> 4
   <211> 784
   <212> PRT
   <213> Homo sapiens
<400> 4
   Met Pro His Thr Leu Trp Met Val Trp Val Leu Gly Val Ile Ile Ser
   1               5                   10                  15
   Leu Ser Lys Glu Glu Ser Ser Asn Gln Ala Ser Leu Ser Cys Asp Arg
               20                  25                  30
   Asn Gly Ile Cys Lys Gly Ser Ser Gly Ser Leu Asn Ser Ile Pro Ser
           35                  40                  45
   Gly Leu Thr Glu Ala Val Lys Ser Leu Asp Leu Ser Asn Asn Arg Ile
       50                  55                  60
   Thr Tyr Ile Ser Asn Ser Asp Leu Gln Arg Cys Val Asn Leu Gln Ala
   65                  70                  75                  80
   Leu Val Leu Thr Ser Asn Gly Ile Asn Thr Ile Glu Glu Asp Ser Phe
                   85                  90                  95
   Ser Ser Leu Gly Ser Leu Glu His Leu Asp Leu Ser Tyr Asn Tyr Leu
               100                 105                 110
   Ser Asn Leu Ser Ser Ser Trp Phe Lys Pro Leu Ser Ser Leu Thr Phe
           115                 120                 125
   Leu Asn Leu Leu Gly Asn Pro Tyr Lys Thr Leu Gly Glu Thr Ser Leu
       130                 135                 140
   Phe Ser His Leu Thr Lys Leu Gln Ile Leu Arg Val Gly Asn Met Asp
   145                 150                 155                 160
   Thr Phe Thr Lys Ile Gln Arg Lys Asp Phe Ala Gly Leu Thr Phe Leu
                   165                 170                 175
   Glu Glu Leu Glu Ile Asp Ala Ser Asp Leu Gln Ser Tyr Glu Pro Lys
               180                 185                 190
   Ser Leu Lys Ser Ile Gln Asn Val Ser His Leu Ile Leu His Met Lys
           195                 200                 205
   Gln His Ile Leu Leu Leu Glu Ile Phe Val Asp Val Thr Ser Ser Val
       210                 215                 220
   Glu Cys Leu Glu Leu Arg Asp Thr Asp Leu Asp Thr Phe His Phe Ser
   225                 230                 235                 240
   Glu Leu Ser Thr Gly Glu Thr Asn Ser Leu Ile Lys Lys Phe Thr Phe
                   245                 250                 255
   Arg Asn Val Lys Ile Thr Asp Glu Ser Leu Phe Gln Val Met Lys Leu
               260                 265                 270
   Leu Asn Gln Ile Ser Gly Leu Leu Glu Leu Glu Phe Asp Asp Cys Thr
           275                 280                 285
   Leu Asn Gly Val Gly Asn Phe Arg Ala Ser Asp Asn Asp Arg Val Ile
       290                 295                 300
   Asp Pro Gly Lys Val Glu Thr Leu Thr Ile Arg Arg Leu His Ile Pro
   305                 310                 315                 320
   Arg Phe Tyr Leu Phe Tyr Asp Leu Ser Thr Leu Tyr Ser Leu Thr Glu
                   325                 330                 335
   Arg Val Lys Arg Ile Thr Val Glu Asn Ser Lys Val Phe Leu Val Pro
               340                 345                 350
   Cys Leu Leu Ser Gln His Leu Lys Ser Leu Glu Tyr Leu Asp Leu Ser
           355                 360                 365
   Glu Asn Leu Met Val Glu Glu Tyr Leu Lys Asn Ser Ala Cys Glu Asp
       370                 375                 380
   Ala Trp Pro Ser Leu Gln Thr Leu Ile Leu Arg Gln Asn His Leu Ala
   385                 390                 395                 400
   Ser Leu Glu Lys Thr Gly Glu Thr Leu Leu Thr Leu Lys Asn Leu Thr
                   405                 410                 415
   Asn Ile Asp Ile Ser Lys Asn Ser Phe His Ser Met Pro Glu Thr Cys
               420                 425                 430
   Gln Trp Pro Glu Lys Met Lys Tyr Leu Asn Leu Ser Ser Thr Arg Ile
           435                 440                 445
   His Ser Val Thr Gly Cys Ile Pro Lys Thr Leu Glu Ile Leu Asp Val
       450                 455                 460
   Ser Asn Asn Asn Leu Asn Leu Phe Ser Leu Asn Leu Pro Gln Leu Lys
   465                 470                 475                 480
   Glu Leu Tyr Ile Ser Arg Asn Lys Leu Met Thr Leu Pro Asp Ala Ser
                   485                 490                 495
   Leu Leu Pro Met Leu Leu Val Leu Lys Ile Ser Arg Asn Ala Ile Thr
               500                 505                 510
   Thr Phe Ser Lys Glu Gln Leu Asp Ser Phe His Thr Leu Lys Thr Leu
           515                 520                 525
   Glu Ala Gly Gly Asn Asn Phe Ile Cys Ser Cys Glu Phe Leu Ser Phe
       530                 535                 540
   Thr Gln Glu Gln Gln Ala Leu Ala Lys Val Leu Ile Asp Trp Pro Ala
   545                 550                 555                 560
   Asn Tyr Leu Cys Asp Ser Pro Ser His Val Arg Gly Gln Gln Val Gln
                   565                 570                 575
   Asp Val Arg Leu Ser Val Ser Glu Cys His Arg Thr Ala Leu Val Ser
               580                 585                 590
   Gly Met Cys Cys Ala Leu Phe Leu Leu Ile Leu Leu Thr Gly Val Leu
           595                 600                 605
   Cys His Arg Phe His Gly Leu Trp Tyr Met Lys Met Met Trp Ala Trp
       610                 615                 620
   Leu Gln Ala Lys Arg Lys Pro Arg Lys Ala Pro Ser Arg Asn Ile Cys
   625                 630                 635                 640
   Tyr Asp Ala Phe Val Ser Tyr Ser Glu Arg Asp Ala Tyr Trp Val Glu
                   645                 650                 655
   Asn Leu Met Val Gln Glu Leu Glu Asn Phe Asn Pro Pro Phe Lys Leu
               660                 665                 670
   Cys Leu His Lys Arg Asp Phe Ile Pro Gly Lys Trp Ile Ile Asp Asn
               675                 680                 685
   Ile Ile Asp Ser Ile Glu Lys Ser His Lys Thr Val Phe Val Leu Ser
       690                 695                 700
   Glu Asn Phe Val Lys Ser Glu Trp Cys Lys Tyr Glu Leu Asp Phe Ser
   705                 710                 715                 720
   His Phe Arg Leu Phe Asp Glu Asn Asn Asp Ala Ala Ile Leu Ile Leu
                   725                 730                 735
   Leu Glu Pro Ile Glu Lys Lys Ala Ile Pro Gln Arg Phe Cys Lys Leu
               740                 745                 750
   Arg Lys Ile Met Asn Thr Lys Thr Tyr Leu Glu Trp Pro Met Asp Glu
           755                 760                 765
   Ala Gln Arg Glu Gly Phe Trp Val Asn Leu Arg Ala Ala Ile Lys Ser
       770                 775                 780
<210> 5
   <211> 2672
   <212> DNA
   <213> Homo sapiens
<400> 5
   cttccagaga gcaatatggc tggttcccca acatgcctca ccctcatcta tatcctttgg      60
   cagctcacag ggtcagcagc ctctggaccc gtgaaagagc tggtcggttc cgttggtggg     120
   gccgtgactt tccccctgaa gtccaaagta aagcaagttg actctattgt ctggaccttc    180
   aacacaaccc ctcttgtcac catacagcca gaagggggca ctatcatagt gacccaaaat     240
   cgtaataggg agagagtaga cttcccagat ggaggctact ccctgaagct cagcaaactg     300
   aagaagaatg actcagggat ctactatgtg ggatatacaa gctcatcact ccagcagccc     360
   tccacccagg agtacgtgct gcatgtctac gagcacctgt caaagcctaa agtcaccatg     420
   ggtctgcaga gcaataagaa tggcacctgt gtgaccaatc tgacatgctg catggaacat     480
   ggggaagagg atgtgattta tacctggaag gccctggggc aagcagccaa tgagtcccat     540
   aatgggtcca tcctccccat ctcctggaga tggggagaaa gtgatatgac cttcatctgc     600
   gttgccagga accctgtcag cagaaacttc tcaagcccca tccttgccag gaagctctgt     660
   gaaggtgctg ctgatgaccc agattcctcc atggtcctcc tgtgtctcct gttggtgccc     720
   ctcctgctca gtctctttgt actggggcta tttctttggt ttctgaagag agagagacaa     780
   gaagagtaca ttgaagagaa gaagagagtg gacatttgtc gggaaactcc taacatatgc     840
   ccccattctg gagagaacac agagtacgac acaatccctc acactaatag aacaatccta     900
   aaggaagatc cagcaaatac ggtttactcc actgtggaaa taccgaaaaa gatggaaaat 960
   ccccactcac tgctcacgat gccagacaca ccaaggctat ttgcctatga gaatgttatc 1020
   tagacagcag tgcactcccc taagtctctg ctcaaaaaaa aaacaattct cggcccaaag 1080
   aaaacaatca gaagaattca ctgatttgac tagaaacatc aaggaagaat gaagaacgtt 1140
   gacttttttc caggataaat tatctctgat gcttctttag atttaagagt tcataattcc 1200
   atccactgct gagaaatctc ctcaaaccca gaaggtttaa tcacttcatc ccaaaaatgg 1260
   gattgtgaat gtcagcaaac cataaaaaaa gtgcttagaa gtattcctat agaaatgtaa 1320
   atgcaaggtc acacatatta atgacagcct gttgtattaa tgatggctcc aggtcagtgt 1380
   ctggagtttc attccatccc agggcttgga tgtaaggatt ataccaagag tcttgctacc 1440
   aggagggcaa gaagaccaaa acagacagac aagtccagca gaagcagatg cacctgacaa 1500
   aaatggatgt attaattggc tctataaact atgtgcccag cactatgctg agcttacact 1560
   aattggtcag acgtgctgtc tgccctcatg aaattggctc caaatgaatg aactactttc 1620
   atgagcagtt gtagcaggcc tgaccacaga ttcccagagg gccaggtgtg gatccacagg 1680
   acttgaaggt caaagttcac aaagatgaag aatcagggta gctgaccatg tttggcagat 1740
   actataatgg agacacagaa gtgtgcatgg cccaaggaca aggacctcca gccaggcttc 1800
   atttatgcac ttgtgctgca aaagaaaagt ctaggtttta aggctgtgcc agaacccatc 1860
   ccaataaaga gaccgagtct gaagtcacat tgtaaatcta gtgtaggaga cttggagtca 1920
   ggcagtgaga ctggtggggc acggggggca gtgggtactt gtaaaccttt aaagatggtt 1980
   aattcattca atagatattt attaagaacc tatgcggccc ggcatggtgg ctcacacctg 2040
   taatcccagc actttgggag gccaaggtgg gtgggtcatc tgaggtcagg agttcaagac 2100
   cagcctggcc aacatggtga aaccccatct ctactaaaga tacaaaaatt tgctgagcgt 2160
   ggtggtgtgc acctgtaatc ccagctactc gagaggccaa ggcatgagaa tcgcttgaac 2220
   ctgggaggtg gaggttgcag tgagctgaga tggcaccact gcactccggc ctaggcaacg 2280
   agagcaaaac tccaatacaa acaaacaaac aaacacctgt gctaggtcag tctggcacgt 2340
   aagatgaaca tccctaccaa cacagagctc accatctctt atacttaagt gaaaaacatg 2400
   gggaagggga aaggggaatg gctgcttttg atatgttccc tgacacatat cttgaatgga 2460
   gacctcccta ccaagtgatg aaagtgttga aaaacttaat aacaaatgct tgttgggcaa 2520
   gaatgggatt gaggattatc ttctctcaga aaggcattgt gaaggaattg agccagatct 2580
   ctctccctac tgcaaaaccc tattgtagta aaaaagtctt ctttactatc ttaataaaac 2640
   agatattgtg agattcaaaa aaaaaaaaaa aa 2672
<210> 6
   <211> 335
   <212> PRT
   <213> Homo sapiens
<400> 6
   Met Ala Gly Ser Pro Thr Cys Leu Thr Leu Ile Tyr Ile Leu Trp Gln
   1               5                   10                  15
   Leu Thr Gly Ser Ala Ala Ser Gly Pro Val Lys Glu Leu Val Gly Ser
               20                  25                  30
   Val Gly Gly Ala Val Thr Phe Pro Leu Lys Ser Lys Val Lys Gln Val
           35                  40                  45
   Asp Ser Ile Val Trp Thr Phe Asn Thr Thr Pro Leu Val Thr Ile Gln
       50                  55                  60
   Pro Glu Gly Gly Thr Ile Ile Val Thr Gln Asn Arg Asn Arg Glu Arg
   65                  70                  75                  80
   Val Asp Phe Pro Asp Gly Gly Tyr Ser Leu Lys Leu Ser Lys Leu Lys
                   85                  90                  95
   Lys Asn Asp Ser Gly Ile Tyr Tyr Val Gly Ile Tyr Ser Ser Ser Leu
               100                 105                 110
   Gln Gln Pro Ser Thr Gln Glu Tyr Val Leu His Val Tyr Glu His Leu
           115                 120                 125
   Ser Lys Pro Lys Val Thr Met Gly Leu Gln Ser Asn Lys Asn Gly Thr
       130                 135                 140
   Cys Val Thr Asn Leu Thr Cys Cys Met Glu His Gly Glu Glu Asp Val
   145                 150                 155                 160
   Ile Tyr Thr Trp Lys Ala Leu Gly Gln Ala Ala Asn Glu Ser His Asn
                   165                 170                 175
   Gly Ser Ile Leu Pro Ile Ser Trp Arg Trp Gly Glu Ser Asp Met Thr
               180                 185                 190
   Phe Ile Cys Val Ala Arg Asn Pro Val Ser Arg Asn Phe Ser Ser Pro
           195                 200                 205
   Ile Leu Ala Arg Lys Leu Cys Glu Gly Ala Ala Asp Asp Pro Asp Ser
       210                 215                 220
   Ser Met Val Leu Leu Cys Leu Leu Leu Val Pro Leu Leu Leu Ser Leu
   225                 230                 235                 240
   Phe Val Leu Gly Leu Phe Leu Trp Phe Leu Lys Arg Glu Arg Gln Glu
                   245                 250                 255
   Glu Tyr Ile Glu Glu Lys Lys Arg Val Asp Ile Cys Arg Glu Thr Pro
               260                 265                 270
   Asn Ile Cys Pro His Ser Gly Glu Asn Thr Glu Tyr Asp Thr Ile Pro
           275                 280                 285
   His Thr Asn Arg Thr Ile Leu Lys Glu Asp Pro Ala Asn Thr Val Tyr
       290                 295                 300
   Ser Thr Val Glu Ile Pro Lys Lys Met Glu Asn Pro His Ser Leu Leu
   305                 310                 315                 320
   Thr Met Pro Asp Thr Pro Arg Leu Phe Ala Tyr Glu Asn Val Ile
                   325                 330                 335
<210> 7
   <211> 2111
   <212> DNA
   <213> Homo sapiens
<400> 7
   gggcagtcgt aggagactct gaaaaagcaa ataaatcaat gttaaatcag aaatgtgaat      60
   gtagtaaggg gctgaagaga caggggaaga gaatacatgg gaaaatattg aaaaggacag     120
   agtgatcaaa aagagcaggg acatgggagc attgggcagc acactgggag ccatttcact     180
   ttatgctctt attgtatgat tgagaaaaaa atgtccttag tggttaagtg gcttttcaat     240
   gccacatcag acttgttcca tagcagttga attaggggaa ggtgaataag ttggaggttg     300
   gtgacaagga gagaagctgg aacagagagg agagtcagaa ccagagggaa atgagagact     360
   gagtaggcat ctcagggttt ttgaaggagt ggattttctt tgttgcagtc aggggaggtt     420
   tgtctgttgg ctgcagaaag aagtcagaat agagatatcg tggggtaggt ttgtttggaa     480
   cagaaatcaa agaccaattt ttctgagaga aggaaataac atctgcaaat gatatgctgt     540
   ttttgctact tccattgtta gctgttctcc caggtgatgg caatgcagac gggctcaagg     600
   agcctctctc cttccatgtc acctggatcg catcctttta caaccattcc tggaaacaaa     660
   atctggtctc aggttggctg agtgatttgc agactcatac ctgggacagc aattccagca     720
   ccatcgtttt cctgtgcccc tggtccaggg gaaacttcag caatgaggag tggaaggaac     780
   tggaaacatt attccgtata cgcaccattc ggtcatttga gggaattcgt agatacgccc     840
   atgaattgca gtttgaatat ccttttgaga tacaggtgac aggaggctgt gagctgcact     900
   ctggaaaggt ctcaggaagc ttcttgcagt tagcttatca aggatcagac tttgtgagct     960
   tccagaacaa ttcatggttg ccatatccag tggctgggaa tatggccaag catttctgca 1020
   aagtgctcaa tcagaatcag catgaaaatg acataacaca caatcttctc agtgacacct 1080
   gcccacgttt catcttgggt cttcttgatg caggaaaggc acatctccag cggcaagtga 1140
   agcccgaggc ctggctgtcc catggcccca gtcctggccc tggccatctg cagcttgtgt 1200
   gccatgtctc aggattctac ccaaagcccg tgtgggtgat gtggatgcgg ggtgagcagg 1260
   agcagcaggg cactcagcga ggggacatct tgcccagtgc tgatgggaca tggtatctcc 1320
   gcgcaaccct ggaggtggcc gctggggagg cagctgacct gtcctgtcgg gtgaagcaca 1380
   gcagtctaga gggccaggac atcgtcctct actgggagca tcacagttcc gtgggcttca 1440
   tcatcttggc ggtgatagtg cctttacttc ttctgatagg tcttgcgctt tggttcagga 1500
   aacgctgttt ctgttaagac acaccatgag cctcctcgtc acccttctcc ttttggggtg 1560
   agagaccagc agcccaaggg ctccagacac acctgaacac atcgtgatga tgacgtcctc 1620
   tcaactctct ttgtaaaaat tttgttattt ttgcttgttt ctgattaatg attgtttgtc 1680
   aatataagct caatttaatt ttgcaggatt tgttgttctg acctgggttc tgggactttt 1740
   aaattcaaat tttatctcca gatggaatgg ggtcctagca acctccacat gttcaactat 1800
   taatggatca tcaggcctgt tttagatatc ccttactcca gagggccttc cctgacttac 1860
   aagtgggaag cagtctcttc ctggtctgaa ctcccgccac attttagccg tactttgcta 1920
   actgtgctcc tcacttcctc ttcttcattg cagttattta gatcccccct ttccttctaa 1980
   tttttcagct ccttcaatgc aaagtacatg tatttttaat atatgcatcc ctggtgaagg 2040
   atcttgcctg catgaaacat gttctcaata aaactctgtg ttgaatttat gccaaaaaaa 2100
   aaaaaaaaaa a 2111
<210> 8
   <211> 327
   <212> PRT
   <213> Homo sapiens
<400> 8
   Met Leu Phe Leu Leu Leu Pro Leu Leu Ala Val Leu Pro Gly Asp Gly
   1               5                   10                  15
   Asn Ala Asp Gly Leu Lys Glu Pro Leu Ser Phe His Val Thr Trp Ile
               20                  25                  30
   Ala Ser Phe Tyr Asn His Ser Trp Lys Gln Asn Leu Val Ser Gly Trp
           35                  40                  45
   Leu Ser Asp Leu Gln Thr His Thr Trp Asp Ser Asn Ser Ser Thr Ile
       50                  55                  60
   Val Phe Leu Cys Pro Trp Ser Arg Gly Asn Phe Ser Asn Glu Glu Trp
   65                  70                  75                  80
   Lys Glu Leu Glu Thr Leu Phe Arg Ile Arg Thr Ile Arg Ser Phe Glu
                   85                  90                  95
   Gly Ile Arg Arg Tyr Ala His Glu Leu Gln Phe Glu Tyr Pro Phe Glu
               100                 105                 110
   Ile Gln Val Thr Gly Gly Cys Glu Leu His Ser Gly Lys Val Ser Gly
           115                 120                 125
   Ser Phe Leu Gln Leu Ala Tyr Gln Gly Ser Asp Phe Val Ser Phe Gln
       130                 135                 140
   Asn Asn Ser Trp Leu Pro Tyr Pro Val Ala Gly Asn Met Ala Lys His
   145                 150                 155                 160
   Phe Cys Lys Val Leu Asn Gln Asn Gln His Glu Asn Asp Ile Thr His
                   165                 170                 175
   Asn Leu Leu Ser Asp Thr Cys Pro Arg Phe Ile Leu Gly Leu Leu Asp
               180                 185                 190
   Ala Gly Lys Ala His Leu Gln Arg Gln Val Lys Pro Glu Ala Trp Leu
           195                 200                 205
   Ser His Gly Pro Ser Pro Gly Pro Gly His Leu Gln Leu Val Cys His
       210                 215                 220
   Val Ser Gly Phe Tyr Pro Lys Pro Val Trp Val Met Trp Met Arg Gly
   225                 230                 235                 240
   Glu Gln Glu Gln Gln Gly Thr Gln Arg Gly Asp Ile Leu Pro Ser Ala
                   245                 250                 255
   Asp Gly Thr Trp Tyr Leu Arg Ala Thr Leu Glu Val Ala Ala Gly Glu
               260                 265                 270
   Ala Ala Asp Leu Ser Cys Arg Val Lys His Ser Ser Leu Glu Gly Gln
           275                 280                 285
   Asp Ile Val Leu Tyr Trp Glu His His Ser Ser Val Gly Phe Ile Ile
       290                 295                 300
   Leu Ala Val Ile Val Pro Leu Leu Leu Leu Ile Gly Leu Ala Leu Trp
   305 310 315 320
   Phe Arg Lys Arg Cys Phe Cys
   325
<210> 9
   <211> 1396
   <212> DNA
   <213> Homo sapiens
<400> 9
   ggcagttgga agagagaaga agtcactaca gggtactgag gaaaagcttt gctgaaattg      60
   gagatcaaat accagctctg ccagtaagaa gttgcatctc ccagtgaaat gctgctgctg     120
   ccatttcaac tgttagctgt tctctttcct ggtggtaaca gtgaacatgc cttccagggg     180
   ccgacctcct ttcatgttat ccagacctcg tcctttacca atagtacctg ggcacaaact     240
   caaggctcag gctggttgga tgatttgcag attcatggct gggatagcga ctcaggcact     300
   gccatattcc tgaagccttg gtctaaaggt aactttagtg ataaggaggt tgctgagtta     360
   gaggagatat tccgagtcta catctttgga ttcgctcgag aagtacaaga ctttgccggt     420
   gatttccaga tgaaataccc ctttgagatc cagggcatag caggctgtga gctacattct     480
   ggaggtgcca tagtaagctt cctgagggga gctctaggag gattggattt cctgagtgtc     540
   aagaatgctt catgtgtgcc ttccccagaa ggtggcagca gggcacagaa attctgtgca     600
   ctaatcatac aatatcaagg tatcatggaa actgtgagaa ttctcctcta tgaaacctgc     660
   ccccgatatc tcttgggcgt cctcaatgca ggaaaagcag atctgcaaag acaagtgaag     720
   cctgaggcct ggctgtccag tggccccagt cctggacctg gccgtctgca gcttgtgtgc     780
   catgtctcag gattctaccc aaagcccgtg tgggtgatgt ggatgcgggg tgagcaggag     840
   cagcagggca ctcagctagg ggacatcctg cccaatgcta actggacatg gtatctccga     900
   gcaaccctgg atgtggcaga tggggaggcg gctggcctgt cctgtcgggt gaagcacagc     960
   agtttagagg gccaggacat catcctctac tggagaaacc ccacctccat tggctcaatt    1020
   gttttggcaa taatagtgcc ttccttgctc cttttgctat gccttgcatt atggtatatg    1080
   aggcgccggt catatcagaa tatcccatga gccatcatca tgtctcctct cccattcgca    1140
   ataagtacca agaagcccaa gatatcagcc caaaagtcaa tcttatcata tttcaaatga    1200
   ttttcaaatt tgatgaaatc agagttttca tgtattttaa aataaattat tatttaaaca    1260
   tcagcaaaaa agtacttaaa actgtaaatt tattatgaga ctgtactaac agtgtgattc    1320
   accctgattt tacacacatt aaaatgttag aaaaaatgtg tctcaaaata aatgaaatat    1380
   aatacatatg acttaa                                                    1396
<210> 10
   <211> 333
   <212> PRT
   <213> Homo sapiens
<400> 10
   Met Leu Leu Leu Pro Phe Gln Leu Leu Ala Val Leu Phe Pro Gly Gly
   1               5                   10                  15
   Asn Ser Glu His Ala Phe Gln Gly Pro Thr Ser Phe His Val Ile Gln
               20                  25                  30
   Thr Ser Ser Phe Thr Asn Ser Thr Trp Ala Gln Thr Gln Gly Ser Gly
           35                  40                  45
   Trp Leu Asp Asp Leu Gln Ile His Gly Trp Asp Ser Asp Ser Gly Thr
       50                  55                  60
   Ala Ile Phe Leu Lys Pro Trp Ser Lys Gly Asn Phe Ser Asp Lys Glu
   65                  70                  75                  80
   Val Ala Glu Leu Glu Glu Ile Phe Arg Val Tyr Ile Phe Gly Phe Ala
                   85                  90                  95
   Arg Glu Val Gln Asp Phe Ala Gly Asp Phe Gln Met Lys Tyr Pro Phe
               100                 105                 110
   Glu Ile Gln Gly Ile Ala Gly Cys Glu Leu His Ser Gly Gly Ala Ile
           115                 120                 125
   Val Ser Phe Leu Arg Gly Ala Leu Gly Gly Leu Asp Phe Leu Ser Val
       130                 135                 140
   Lys Asn Ala Ser Cys Val Pro Ser Pro Glu Gly Gly Ser Arg Ala Gln
   145                 150                 155                 160
   Lys Phe Cys Ala Leu Ile Ile Gln Tyr Gln Gly Ile Met Glu Thr Val
                   165                 170                 175
   Arg Ile Leu Leu Tyr Glu Thr Cys Pro Arg Tyr Leu Leu Gly Val Leu
               180                 185                 190
   Asn Ala Gly Lys Ala Asp Leu Gln Arg Gln Val Lys Pro Glu Ala Trp
           195                 200                 205
   Leu Ser Ser Gly Pro Ser Pro Gly Pro Gly Arg Leu Gln Leu Val Cys
       210                 215                 220
   His Val Ser Gly Phe Tyr Pro Lys Pro Val Trp Val Met Trp Met Arg
   225                 230                 235                 240
   Gly Glu Gln Glu Gln Gln Gly Thr Gln Leu Gly Asp Ile Leu Pro Asn
                   245                 250                 255
   Ala Asn Trp Thr Trp Tyr Leu Arg Ala Thr Leu Asp Val Ala Asp Gly
               260                 265                 270
   Glu Ala Ala Gly Leu Ser Cys Arg Val Lys His Ser Ser Leu Glu Gly
           275                 280                 285
   Gln Asp Ile Ile Leu Tyr Trp Arg Asn Pro Thr Ser Ile Gly Ser Ile
       290                 295                 300
   Val Leu Ala Ile Ile Val Pro Ser Leu Leu Leu Leu Leu Cys Leu Ala
   305                 310                 315                 320
   Leu Trp Tyr Met Arg Arg Arg Ser Tyr Gln Asn Ile Pro
                   325                 330
<210> 11
   <211> 6701
   <212> DNA
   <213> Homo sapiens
<400> 11
   aaagccctca gcctttgtgt ccttctctgc gccggagtgg ctgcagctca cccctcagct     60
   ccccttgggg cccagctggg agccgagata gaagctcctg tcgccgctgg gcttctcgcc    120
   tcccgcagag ggccacacag agaccgggat ggccacctcc atgggcctgc tgctgctgct    180
   gctgctgctc ctgacccagc ccggggcggg gacgggagct gacacggagg cggtggtctg    240
   cgtggggacc gcctgctaca cggcccactc gggcaagctg agcgctgccg aggcccagaa    300
   ccactgcaac cagaacgggg gcaacctggc cactgtgaag agcaaggagg aggcccagca    360
   cgtccagcga gtactggccc agctcctgag gcgggaggca gccctgacgg cgaggatgag    420
   caagttctgg attgggctcc agcgagagaa gggcaagtgc ctggacccta gtctgccgct    480
   gaagggcttc agctgggtgg gcggggggga ggacacgcct tactctaact ggcacaagga    540
   gctccggaac tcgtgcatct ccaagcgctg tgtgtctctg ctgctggacc tgtcccagcc    600
   gctccttccc agccgcctcc ccaagtggtc tgagggcccc tgtgggagcc caggctcccc    660
   cggaagtaac attgagggct tcgtgtgcaa gttcagcttc aaaggcatgt gccggcctct    720
   ggccctgggg ggcccaggtc aggtgaccta caccaccccc ttccagacca ccagttcctc    780
   cttggaggct gtgccctttg cctctgcggc caatgtagcc tgtggggaag gtgacaagga    840
   cgagactcag agtcattatt tcctgtgcaa ggagaaggcc cccgatgtgt tcgactgggg    900
   cagctcgggc cccctctgtg tcagccccaa gtatggctgc aacttcaaca atgggggctg 960
   ccaccaggac tgctttgaag ggggggatgg ctccttcctc tgcggctgcc gaccaggatt 1020
   ccggctgctg gatgacctgg tgacctgtgc ctctcgaaac ccttgcagct ccagcccatg 1080
   tcgtgggggg gccacgtgcg tcctgggacc ccatgggaaa aactacacgt gccgctgccc 1140
   ccaagggtac cagctggact cgagtcagct ggactgtgtg gacgtggatg aatgccagga 1200
   ctccccctgt gcccaggagt gtgtcaacac ccctgggggc ttccgctgcg aatgctgggt 1260
   tggctatgag ccgggcggtc ctggagaggg ggcctgtcag gatgtggatg agtgtgctct 1320
   gggtcgctcg ccttgcgccc agggctgcac caacacagat ggctcatttc actgctcctg 1380
   tgaggagggc tacgtcctgg ccggggagga cgggactcag tgccaggacg tggatgagtg 1440
   tgtgggcccg gggggccccc tctgcgacag cttgtgcttc aacacacaag ggtccttcca 1500
   ctgtggctgc ctgccaggct gggtgctggc cccaaatggg gtctcttgca ccatggggcc 1560
   tgtgtctctg ggaccaccat ctgggccccc cgatgaggag gacaaaggag agaaagaagg 1620
   gagcaccgtg ccccgtgctg caacagccag tcccacaagg ggccccgagg gcacccccaa 1680
   ggctacaccc accacaagta gaccttcgct gtcatctgac gcccccatca catctgcccc 1740
   actcaagatg ctggccccca gtgggtcccc aggcgtctgg agggagccca gcatccatca 1800
   cgccacagct gcctctggcc cccaggagcc tgcaggtggg gactcctccg tggccacaca 1860
   aaacaacgat ggcactgacg ggcaaaagct gcttttattc tacatcctag gcaccgtggt 1920
   ggccatccta ctcctgctgg ccctggctct ggggctactg gtctatcgca agcggagagc 1980
   gaagagggag gagaagaagg agaagaagcc ccagaatgcg gcagacagtt actcctgggt 2040
   tccagagcga gctgagagca gggccatgga gaaccagtac agtccgacac ctgggacaga 2100
   ctgctgaaag tgaggtggcc ctagagacac tagagtcacc agccaccatc ctcagagctt 2160
   tgaactcccc attccaaagg ggcacccaca tttttttgaa agactggact ggaatcttag 2220
   caaacaattg taagtctcct ccttaaaggc cccttggaac atgcaggtat tttctacggg 2280
   tgtttgatgt tcctgaagtg gaagctgtgt gttggcgtgc cacggtgggg atttcgtgac 2340
   tctataatga ttgttactcc ccctcccttt tcaaattcca atgtgaccaa ttccggatca 2400
   gggtgtgagg aggccggggc taaggggctc ccctgaatat cttctctgct cacttccacc 2460
   atctaagagg aaaaggtgag ttgctcatgc tgattaggat tgaaatgatt tgtttctctt 2520
   cctaggatga aaactaaatc aattaattat tcaattaggt aagaagatct ggttttttgg 2580
   tcaaagggaa catgttcgga ctggaaacat ttctttacat ttgcattcct ccatttcgcc 2640
   agcacaagtc ttgctaaatg tgatactgtt gacatcctcc agaatggcca gaagtgcaat 2700
   taacctctta ggtggcaagg aggcaggaag tgcctcttta gttcttacat ttctaatagc 2760
   cttgggttta tttgcaaagg aagcttgaaa aatatgagaa aagttgcttg aagtgcatta 2820
   caggtgtttg tgaagtcaca taatctacgg ggctagggcg agagaggcca gggatttgtt 2880
   cacagatact tgaattaatt catccaaatg tactgaggtt accacacact tgactacgga 2940
   tgtgatcaac actaacaagg aaacaaattc aaggacaacc tgtctttgag ccagggcagg 3000
   cctcagacac cctgcctgtg gccccgcctc cacttcatcc tgcccggaat gccagtgctc 3060
   cgagctcaga cagaggaagc cctgcagaaa gttccatcag gctgtttcct aaaggatgtg 3120
   tgaacgggag atgatgcact gtgttttgaa agttgtcatt ttaaagcatt ttagcacagt 3180
   tcatagtcca cagttgatgc agcatcctga gattttaaat cctgaagtgt gggtggcgca 3240
   cacaccaagt agggagctag tcaggcagtt tgcttaagga acttttgttc tctgtctctt 3300
   ttccttaaaa ttgggggtaa ggagggaagg aagagggaaa gagatgacta actaaaatca 3360
   tttttacagc aaaaactgct caaagccatt taaattatat cctcatttta aaagttacat 3420
   ttgcaaatat ttctccctat gataatgtag tcgatagtgt gcactctttc tctctctctc 3480
   tctctctcac acacacacac acacacacac acacacacac agagacacgg caccattctg 3540
   cctggggcac tggaacacat tcctgggggt caccgatggt cagagtcact agaagttacc 3600
   tgagtatctc tgggaggcct catgtctcct gtgggctttt taccaccact gtgcaggaga 3660
   acagacagag gaaatgtgtc tccctccaag gccccaaagc ctcagagaaa gggtgtttct 3720
   ggttttgcct tagcaatgca tcggtctctg aggtgacact ctggagtggt tgaagggcca 3780
   caaggtgcag ggttaatact cttgccagtt ttgaaatata gatgctatgg ttcagattgt 3840
   ttttaataga aaactaaagg ggcaggggaa gtgaaaggaa agatggaggt tttgtgcggc 3900
   tcgatggggc atttggaact tctttttaaa gtcatctcat ggtctccagt tttcagttgg 3960
   aactctggtg tttaacactt aagggagaca aaggctgtgt ccatttggca aaacttcctt 4020
   ggccacgaga ctctaggtga tgtgtgaagc tgggcagtct gtggtgtgga gagcagccat 4080
   ctgtctggcc attcagagga ttctaaagac atggctggat gcgctgctga ccaacatcag 4140
   cacttaaata aatgcaaatg caacatttct ccctctgggc cttgaaaatc cttgccctta 4200
   tcatttgggg tgaaggagac atttctgtcc ttggcttccc acagccccaa cgcagtctgt 4260
   gtatgattcc tgggatccaa cgagccctcc tattttcaca gtgttctgat tgctctcaca 4320
   gcccaggccc atcgtctgtt ctctgaatgc agccctgttc tcaacaacag ggaggtcatg 4380
   gaacccctct gtggaaccca caaggggaga aatgggtgat aaagaatcca gttcctcaaa 4440
   accttccctg gcaggctggg tccctctcct gctgggtggt gctttctctt gcacaccact 4500
   cccaccacgg ggggagagcc agcaacccaa ccagacagct caggttgtgc atctgatgga 4560
   aaccactggg ctcaaacacg tgctttattc tcctgtttat ttttgctgtt actttgaagc 4620
   atggaaattc ttgtttgggg gatcttgggg ctacagtagt gggtaaacaa atgcccaccg 4680
   gccaagaggc cattaacaaa tcgtccttgt cctgaggggc cccagcttgc tcgggcgtgg 4740
   cacagtgggg aatccaaggg tcacagtatg gggagaggtg caccctgcca cctgctaact 4800
   tctcgctaga cacagtgttt ctgcccaggt gacctgttca gcagcagaac aagccagggc 4860
   catggggacg gggaagtttt tcacttggag atggacacca agacaatgaa gatttgttgt 4920
   ccaaataggt caataattct gggagactct tggaaaaaac tgaatatatt caggaccaac 4980
   tctctccctc ccctcatccc acatctcaaa gcagacaatg taaagagaga acatctcaca 5040
   cacccagctc gccatgccta ctcattcctg aatttcaggt gccatcactg ctctttcttt 5100
   cttctttgtc atttgagaaa ggatgcagga ggacaattcc cacagataat ctgaggaatg 5160
   cagaaaaacc agggcaggac agttatcgac aatgcattag aacttggtga gcatcctctg 5220
   tagagggact ccacccctgc tcaacagctt ggcttccagg caagaccaac cacatctggt 5280
   ctctgccttc ggtggcccac acacctaagc gtcatcgtca ttgccatagc atcatgatgc 5340
   aacacatcta cgtgtagcac tacgacgtta tgtttgggta atgtggggat gaactgcatg 5400
   aggctctgat taaggatgtg gggaagtggg ctgcggtcac tgtcggcctt gcaaggccac 5460
   ctggaggcct gtctgttagc cagtggtgga ggagcaaggc ttcaggaagg gccagccaca 5520
   tgccatcttc cctgcgatca ggcaaaaaag tggaattaaa aagtcaaacc tttatatgca 5580
   tgtgttatgt ccattttgca ggatgaactg agtttaaaag aatttttttt tctcttcaag 5640
   ttgctttgtc ttttccatcc tcatcacaag cccttgtttg agtgtcttat ccctgagcaa 5700
   tctttcgatg gatggagatg atcattaggt acttttgttt caacctttat tcctgtaaat 5760
   atttctgtga aaactaggag aacagagatg agatttgaca aaaaaaaatt gaattaaaaa 5820
   taacacagtc tttttaaaac taacatagga aagcctttcc tattatttct cttcttagct 5880
   tctccattgt ctaaatcagg aaaacaggaa aacacagctt tctagcagct gcaaaatggt 5940
   ttaatgcccc ctacatattt ccatcacctt gaacaatagc tttagcttgg gaatctgaga 6000
   tatgatccca gaaaacatct gtctctactt cggctgcaaa acccatggtt taaatctata 6060
   tggtttgtgc attttctcaa ctaaaaatag agatgataat ccgaattctc catatattca 6120
   ctaatcaaag acactatttt catactagat tcctgagaca aatactcact gaagggcttg 6180
   tttaaaaata aattgtgttt tggtctgttc ttgtagataa tgcccttcta ttttaggtag 6240
   aagctctgga atccctttat tgtgctgttg ctcttatctg caaggtggca agcagttctt 6300
   ttcagcagat tttgcccact attcctctga gctgaagttc tttgcataga tttggcttaa 6360
   gcttgaatta gatccctgca aaggcttgct ctgtgatgtc agatgtaatt gtaaatgtca 6420
   gtaatcactt catgaatgct aaatgagaat gtaagtattt ttaaatgtgt gtatttcaaa 6480
   tttgtttgac taattctgga attacaagat ttctatgcag gatttacctt catcctgtgc 6540
   atgtttccca aactgtgagg agggaaggct cagagatcga gcttctcctc tgagttctaa 6600
   caaaatggtg ctttgagggt cagcctttag gaaggtgcag ctttgttgtc ctttgagctt 6660
   tctgttatgt gcctatccta ataaactctt aaacacattg a 6701
<210> 12
   <211> 652
   <212> PRT
   <213> Homo sapiens
<400> 12
   Met Ala Thr Ser Met Gly Leu Leu Leu Leu Leu Leu Leu Leu Leu Thr
   1               5                   10                  15
   Gln Pro Gly Ala Gly Thr Gly Ala Asp Thr Glu Ala Val Val Cys Val
               20                  25                  30
   Gly Thr Ala Cys Tyr Thr Ala His Ser Gly Lys Leu Ser Ala Ala Glu
           35                  40                  45
   Ala Gln Asn His Cys Asn Gln Asn Gly Gly Asn Leu Ala Thr Val Lys
       50                  55                  60
   Ser Lys Glu Glu Ala Gln His Val Gln Arg Val Leu Ala Gln Leu Leu
   65                  70                  75                  80
   Arg Arg Glu Ala Ala Leu Thr Ala Arg Met Ser Lys Phe Trp Ile Gly
                   85                  90                  95
   Leu Gln Arg Glu Lys Gly Lys Cys Leu Asp Pro Ser Leu Pro Leu Lys
               100                 105                 110
   Gly Phe Ser Trp Val Gly Gly Gly Glu Asp Thr Pro Tyr Ser Asn Trp
           115                 120                 125
   His Lys Glu Leu Arg Asn Ser Cys Ile Ser Lys Arg Cys Val Ser Leu
       130                 135                 140
   Leu Leu Asp Leu Ser Gln Pro Leu Leu Pro Ser Arg Leu Pro Lys Trp
   145                 150                 155                 160
   Ser Glu Gly Pro Cys Gly Ser Pro Gly Ser Pro Gly Ser Asn Ile Glu
                   165                 170                 175
   Gly Phe Val Cys Lys Phe Ser Phe Lys Gly Met Cys Arg Pro Leu Ala
               180                 185                 190
   Leu Gly Gly Pro Gly Gln Val Thr Tyr Thr Thr Pro Phe Gln Thr Thr
           195                 200                 205
   Ser Ser Ser Leu Glu Ala Val Pro Phe Ala Ser Ala Ala Asn Val Ala
       210                 215                 220
   Cys Gly Glu Gly Asp Lys Asp Glu Thr Gln Ser His Tyr Phe Leu Cys
   225                 230                 235                 240
   Lys Glu Lys Ala Pro Asp Val Phe Asp Trp Gly Ser Ser Gly Pro Leu
                   245                 250                 255
   Cys Val Ser Pro Lys Tyr Gly Cys Asn Phe Asn Asn Gly Gly Cys His
               260                 265                 270
   Gln Asp Cys Phe Glu Gly Gly Asp Gly Ser Phe Leu Cys Gly Cys Arg
           275                 280                 285
   Pro Gly Phe Arg Leu Leu Asp Asp Leu Val Thr Cys Ala Ser Arg Asn
       290                 295                 300
   Pro Cys Ser Ser Ser Pro Cys Arg Gly Gly Ala Thr Cys Val Leu Gly
   305                 310                 315                 320
   Pro His Gly Lys Asn Tyr Thr Cys Arg Cys Pro Gln Gly Tyr Gln Leu
                   325                 330                 335
   Asp Ser Ser Gln Leu Asp Cys Val Asp Val Asp Glu Cys Gln Asp Ser
               340                 345                 350
   Pro Cys Ala Gln Glu Cys Val Asn Thr Pro Gly Gly Phe Arg Cys Glu
           355                 360                 365
   Cys Trp Val Gly Tyr Glu Pro Gly Gly Pro Gly Glu Gly Ala Cys Gln
       370                 375                 380
   Asp Val Asp Glu Cys Ala Leu Gly Arg Ser Pro Cys Ala Gln Gly Cys
   385                 390                 395                 400
   Thr Asn Thr Asp Gly Ser Phe His Cys Ser Cys Glu Glu Gly Tyr Val
                   405                 410                 415
   Leu Ala Gly Glu Asp Gly Thr Gln Cys Gln Asp Val Asp Glu Cys Val
               420                 425                 430
   Gly Pro Gly Gly Pro Leu Cys Asp Ser Leu Cys Phe Asn Thr Gln Gly
           435                 440                 445
   Ser Phe His Cys Gly Cys Leu Pro Gly Trp Val Leu Ala Pro Asn Gly
       450                 455                 460
   Val Ser Cys Thr Met Gly Pro Val Ser Leu Gly Pro Pro Ser Gly Pro
   465                 470                 475                 480
   Pro Asp Glu Glu Asp Lys Gly Glu Lys Glu Gly Ser Thr Val Pro Arg
                   485                 490                 495
   Ala Ala Thr Ala Ser Pro Thr Arg Gly Pro Glu Gly Thr Pro Lys Ala
               500                 505                 510
   Thr Pro Thr Thr Ser Arg Pro Ser Leu Ser Ser Asp Ala Pro Ile Thr
           515                 520                 525
   Ser Ala Pro Leu Lys Met Leu Ala Pro Ser Gly Ser Pro Gly Val Trp
       530                 535                 540
   Arg Glu Pro Ser Ile His His Ala Thr Ala Ala Ser Gly Pro Gln Glu
   545                 550                 555                 560
   Pro Ala Gly Gly Asp Ser Ser Val Ala Thr Gln Asn Asn Asp Gly Thr
                   565                 570                 575
   Asp Gly Gln Lys Leu Leu Leu Phe Tyr Ile Leu Gly Thr Val Val Ala
               580                 585                 590
   Ile Leu Leu Leu Leu Ala Leu Ala Leu Gly Leu Leu Val Tyr Arg Lys
           595                 600                 605
   Arg Arg Ala Lys Arg Glu Glu Lys Lys Glu Lys Lys Pro Gln Asn Ala
       610                 615                 620
   Ala Asp Ser Tyr Ser Trp Val Pro Glu Arg Ala Glu Ser Arg Ala Met
   625                 630                 635                 640
   Glu Asn Gln Tyr Ser Pro Thr Pro Gly Thr Asp Cys
                   645                 650
<210> 13
   <211> 2664
   <212> DNA
   <213> Homo sapiens
<400> 13
   gctatctgag gaagttctgg tagagagaag agctcaagag catgggcaga gtcagctcct     60
   gagtgggctg aacgctcccc tcagctcctg cagtgctaat taagggaggg agcagcgggg    120
   agcttgcagt gaccaagagg gtgttgaggc taggaggcca cgataaacag gatacgataa    180
   aagtccttaa ccaagacgca gatgggaaga agcgttagag cgagcagcac tcacatctca    240
   agaaccagcc tttcaaacag tttccagaga tggattatcc tactttactt ttggctcttc    300
   ttcatgtata cagagctcta tgtgaagagg tgctttggca tacatcagtt ccctttgccg    360
   agaacatgtc tctagaatgt gtgtatccat caatgggcat cttaacacag gtggagtggt    420
   tcaagatcgg gacccagcag gattccatag ccattttcag ccctactcat ggcatggtca    480
   taaggaagcc ctatgctgag agggtttact ttttgaattc aacgatggct tccaataaca     540
   tgactctttt ctttcggaat gcctctgaag atgatgttgg ctactattcc tgctctcttt    600
   acacttaccc acagggaact tggcagaagg tgatacaggt ggttcagtca gatagttttg    660
   aggcagctgt gccatcaaat agccacattg tttcggaacc tggaaagaat gtcacactca    720
   cttgtcagcc tcagatgacg tggcctgtgc aggcagtgag gtgggaaaag atccagcccc    780
   gtcagatcga cctcttaact tactgcaact tggtccatgg cagaaatttc acctccaagt    840
   tcccaagaca aatagtgagc aactgcagcc acggaaggtg gagcgtcatc gtcatccccg    900
   atgtcacagt ctcagactcg gggctttacc gctgctactt gcaggccagc gcaggagaaa    960
   acgaaacctt cgtgatgaga ttgactgtag ccgagggtaa aaccgataac caatataccc   1020
   tctttgtggc tggagggaca gttttattgt tgttgtttgt tatctcaatt accaccatca   1080
   ttgtcatttt ccttaacaga aggagaagga gagagagaag agatctattt acagagtcct   1140
   gggatacaca gaaggcaccc aataactata gaagtcccat ctctaccagt caacctacca   1200
   atcaatccat ggatgataca agagaggata tttatgtcaa ctatccaacc ttctctcgca   1260
   gaccaaagac tagagtttaa gcttattctt gacatgagtg cattagtaat gactcttatg   1320
   tactcatgca tggatcttta tgcaattttt ttccactacc caaggtctac cttagatact   1380
   agttgtctga attgagttac tttgatagga aaaatacttc attacctaaa atcatttttc   1440
   atagaactgt ttcagaaaac ctgactctaa ctggtttata tacaaaagaa aacttactgt   1500
   atcatataac agaatgatcc aggggagatt aagctttggg caagggctat ttaccagggc   1560
   ttaaatgttg tgtctagaat taagtatggg cataaactgg cttctgaatc cctttccaga   1620
   gtgttggatc catttccctg gtcttggcct cactctcatg caggctttcc tcttgtgttg   1680
   gcaagatggc tgccaactct tggcaattca tacatccttg tttctgtctg gtagagagtt   1740
   tgcttctcaa atggagcaaa caaatttgat tattttttca ttgttaaata ggcaacatga   1800
   ccagaaagga tggaatggct taagtaaact aagggttcac ttctagagct gagaagcagg   1860
   gtcaaagcac aatactgggc aattcagagc atggttagaa gaggaaaggg gagtctcaaa   1920
   gctggagagt ttaccaacaa atattgactg cagtgattaa ccaagacatt tttgttaact   1980
   aaaaagtgaa atatgggatg gattctagaa atggggtatc tctgtccata cttctagaat   2040
   ccactctatc agcatagtcc agaagaatac ctggcagtag aagaaatgaa tattcaagag   2100
   gaagataaat gcgagagggc aatcctttac tattctcata tttatttatc tctcattctg 2160
   tatagaattc ttgccgccat cccaggtcta gccttaggag caaatgtagt agatagtcga 2220
   ataataaata acttaatgtt ttggacatat tttgtctact tttgagaatt atttttaata 2280
   tgtaaattct ctcaaaaggg tcaggcacct agttattatt ttttaatgat tatgtgaaag 2340
   ttgaatataa tataccacta aaagtgacag ttgaaagtgg tggcatagga tggtagggta 2400
   gaaatttggg agggaaaaaa gaaattggga gggtacaggc aacaggagaa aggaatcaaa 2460
   ccacagaaaa atacaaaggg aaacttctgc ttcactattc agacaaagac agccctaatg 2520
   acatcaccaa cagtcaaagc aattagagac catacctaat attgtttaaa ttctagatgt 2580
   aggctaacaa tgaaaagtat ttgccaaact gaataaaact gtcatggtta ccttgaaaaa 2640
   aaaaaaaaaa aaaaaaaaaa aaaa 2664
<210> 14
   <211> 336
   <212> PRT
   <213> Homo sapiens
<400> 14
   Met Asp Tyr Pro Thr Leu Leu Leu Ala Leu Leu His Val Tyr Arg Ala
   1 5 10 15
   Leu Cys Glu Glu Val Leu Trp His Thr Ser Val Pro Phe Ala Glu Asn
   20 25 30
   Met Ser Leu Glu Cys Val Tyr Pro Ser Met Gly Ile Leu Thr Gln Val
   35 40 45
   Glu Trp Phe Lys Ile Gly Thr Gln Gln Asp Ser Ile Ala Ile Phe Ser
   50 55 60
   Pro Thr His Gly Met Val Ile Arg Lys Pro Tyr Ala Glu Arg Val Tyr
   65 70 75 80
   Phe Leu Asn Ser Thr Met Ala Ser Asn Asn Met Thr Leu Phe Phe Arg
   85 90 95
   Asn Ala Ser Glu Asp Asp Val Gly Tyr Tyr Ser Cys Ser Leu Tyr Thr
   100 105 110
   Tyr Pro Gln Gly Thr Trp Gln Lys Val Ile Gln Val Val Gln Ser Asp
   115 120 125
   Ser Phe Glu Ala Ala Val Pro Ser Asn Ser His Ile Val Ser Glu Pro
   130 135 140
   Gly Lys Asn Val Thr Leu Thr Cys Gln Pro Gln Met Thr Trp Pro Val
   145 150 155 160
   Gln Ala Val Arg Trp Glu Lys Ile Gln Pro Arg Gln Ile Asp Leu Leu
   165 170 175
   Thr Tyr Cys Asn Leu Val His Gly Arg Asn Phe Thr Ser Lys Phe Pro
   180 185 190
   Arg Gln Ile Val Ser Asn Cys Ser His Gly Arg Trp Ser Val Ile Val
   195 200 205
   Ile Pro Asp Val Thr Val Ser Asp Ser Gly Leu Tyr Arg Cys Tyr Leu
   210 215 220
   Gln Ala Ser Ala Gly Glu Asn Glu Thr Phe Val Met Arg Leu Thr Val
   225 230 235 240
   Ala Glu Gly Lys Thr Asp Asn Gln Tyr Thr Leu Phe Val Ala Gly Gly
   245 250 255
   Thr Val Leu Leu Leu Leu Phe Val Ile Ser Ile Thr Thr Ile Ile Val
   260 265 270
   Ile Phe Leu Asn Arg Arg Arg Arg Arg Glu Arg Arg Asp Leu Phe Thr
   275 280 285
   Glu Ser Trp Asp Thr Gln Lys Ala Pro Asn Asn Tyr Arg Ser Pro Ile
   290 295 300
   Ser Thr Ser Gln Pro Thr Asn Gln Ser Met Asp Asp Thr Arg Glu Asp
   305 310 315 320
   Ile Tyr Val Asn Tyr Pro Thr Phe Ser Arg Arg Pro Lys Thr Arg Val
   325 330 335
<210> 15
   <211> 19
   <212> DNA
   <213> Artificial
<220>
   <223> primer
<400> 15
   gtccacctcg acctgctct 19
<210> 16
   <211> 19
   <212> DNA
   <213> Artificial
<220>
   <223> primer
<400> 16
   cactggggca ggagaaact 19
<210> 17
   <211> 19
   <212> DNA
   <213> Artificial
<220>
   <223> primer
<400> 17
   tgggaaagca tcgctacac 19
<210> 18
   <211> 20
   <212> DNA
   <213> Artificial
<220>
   <223> primer
<400> 18
   gcactggagc tggaaatagc 20
<210> 19
   <211> 19
   <212> DNA
   <213> Artificial
<220>
   <223> primer
<400> 19
   gggagaatcc aagcaggac 19
<210> 20
   <211> 20
   <212> DNA
   <213> Artificial
<220>
   <223> primer
<400> 20
   ggaagctcct cgatctctga 20
<210> 21
   <211> 20
   <212> DNA
   <213> Artificial
<220>
   <223> primer
<400> 21
   tcgctgtttc tacggtagcc 20
<210> 22
   <211> 19
   <212> DNA
   <213> Artificial
<220>
   <223> primer
<400> 22
   gggtgggttt gatgtaggc 19
<210> 23
   <211> 21
   <212> DNA
   <213> Artificial
<220>
   <223> primer
<400> 23
   cagccacaat cactcatcag a 21
<210> 24
   <211> 19
   <212> DNA
   <213> Artificial
<220>
   <223> primer
<400> 24
   agggtgggtt tgctgtagg 19
<210> 25
   <211> 18
   <212> DNA
   <213> Artificial
<220>
   <223> primer
<400> 25
   ggagctcgtg gtctcagg 18
<210> 26
   <211> 21
   <212> DNA
   <213> Artificial
<220>
   <223> primer
<400> 26
   cttggagtcg gacttgtttt g 21
<210> 27
   <211> 19
   <212> DNA
   <213> Artificial
<220>
   <223> primer
<400> 27
   gcaccatggt ctcatccat 19
<210> 28
   <211> 19
   <212> DNA
   <213> Artificial
<220>
   <223> primer
<400> 28
   gtggctttgg agaggttcc 19
<210> 29
   <211> 18
   <212> DNA
   <213> Artificial
<220>
   <223> primer
<400> 29
   ggagctcgtg gtctcagg 18
<210> 30
   <211> 20
   <212> DNA
   <213> Artificial
<220>
   <223> primer
<400> 30
   gggtttgatg taggctcctg 20
<210> 31
   <211> 20
   <212> DNA
   <213> Artificial
<220>
   <223> primer
<400> 31
   ggagataccg ctgccactat 20
<210> 32
   <211> 18
   <212> DNA
   <213> Artificial
<220>
   <223> primer
<400> 32
   ggtgggtttg ctgtaggc 18
<210> 33
   <211> 23
   <212> DNA
   <213> Artificial
<220>
   <223> primer
<400> 33
   ttcgaattcc tcggtatatc ttg 23
<210> 34
   <211> 20
   <212> DNA
   <213> Artificial
<220>
   <223> primer
<400> 34
   cacctccagt tatgcgtctg 20
<210> 35
   <211> 20
   <212> DNA
   <213> Artificial
<220>
   <223> primer
<400> 35
   atgatgaagc ctggaatgga 20
<210> 36
   <211> 21
   <212> DNA
   <213> Artificial
<220>
   <223> primer
<400> 36
   tcagagcttt acggagctca t 21
<210> 37
   <211> 20
   <212> DNA
   <213> Artificial
<220>
   <223> primer
<400> 37
   gcctggaaga gattcgtgac 20
<210> 38
   <211> 22
   <212> DNA
   <213> Artificial
<220>
   <223> primer
<400> 38
   cttcagagct tcgtagagag ca 22
<210> 39
   <211> 20
   <212> DNA
   <213> Artificial
<220>
   <223> primer
<400> 39
   aagtgaggct ggtgttggtt 20
<210> 40
   <211> 21
   <212> DNA
   <213> Artificial
<220>
   <223> primer
<400> 40
   cgtcttgtag ctccacgtct t 21
<210> 41
   <211> 19
   <212> DNA
   <213> Artificial
<220>
   <223> primer
<400> 41
   agccacatcg ctcagacac 19
<210> 42
   <211> 19
   <212> DNA
   <213> Artificial
<220>
   <223> primer
<400> 42
   gcccaatacg accaaatcc 19
<210> 43
   <211> 18
   <212> DNA
   <213> Artificial
<220>
   <223> primer
<400> 43
   ccgcagcaga atggacag 18
<210> 44
   <211> 18
   <212> DNA
   <213> Artificial
<220>
   <223> primer
<400> 44
   gctcctgggg tgtagatg 18
<210> 45
   <211> 18
   <212> DNA
   <213> Artificial
<220>
   <223> primer
<400> 45
   accgcagaag gtgcagac 18
<210> 46
   <211> 21
   <212> DNA
   <213> Artificial
<220>
   <223> primer
<400> 46
   ctggcttgat ttcttcaggt g 21
<210> 47
   <211> 18
   <212> DNA
   <213> Artificial
<220>
   <223> primer
<400> 47
   ccgcagcaga atggacag 18
<210> 48
   <211> 19
   <212> DNA
   <213> Artificial
<220>
   <223> primer
<400> 48
   gcaggaggaa cagaaagag 19
<210> 49
   <211> 19
   <212> DNA
   <213> Artificial
<220>
   <223> primer
<400> 49
   agccacatcg ctcagacac 19
<210> 50
   <211> 19
   <212> DNA
   <213> Artificial
<220>
   <223> primer
<400> 50
   gcccaatacg accaaatcc 19